(19) Europäisches Patentamt
European Patent Office
Office européen des brevets

(11) EP 4 682 156 A1

(12) **EUROPEAN PATENT APPLICATION**
published in accordance with Art. 153(4) EPC

(43) Date of publication:
**21.01.2026 Bulletin 2026/04**

(21) Application number: **24770004.0**

(22) Date of filing: **14.03.2024**

(51) International Patent Classification (IPC):
***C07K 5/103*** *(2006.01)* ***A61K 47/64*** *(2017.01)*
***A61K 47/68*** *(2017.01)* ***A61P 35/00*** *(2006.01)*
***C07K 7/02*** *(2006.01)* ***C07K 7/06*** *(2006.01)*
***C07K 7/64*** *(2006.01)* ***C07K 16/32*** *(2006.01)*

(52) Cooperative Patent Classification (CPC):
**A61K 47/64; A61K 47/68; A61P 35/00; C07K 5/1005; C07K 7/02; C07K 7/06; C07K 7/64; C07K 16/32**

(86) International application number:
**PCT/CN2024/081715**

(87) International publication number:
**WO 2024/188314 (19.09.2024 Gazette 2024/38)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **15.03.2023 CN 202310249755**

(71) Applicant: **Shanghai Affinity Biopharmaceutical Co., Ltd.**
**Shanghai 201203 (CN)**

(72) Inventors:
- **LIU, Yuan**
  **Shanghai 201203 (CN)**
- **CHEN, Tao**
  **Shanghai 201203 (CN)**
- **LI, Yuling**
  **Shanghai 201203 (CN)**
- **LIU, Chen**
  **Shanghai 201203 (CN)**

(74) Representative: **Mewburn Ellis LLP**
**Aurora Building**
**Counterslip**
**Bristol BS1 6BX (GB)**

# (54) LINKER AND DRUG CONJUGATE USING SAME, AND ANTIBODY-DRUG CONJUGATE AND USE THEREOF

(57) Provided are a linker and a drug conjugate using same, and an antibody-drug conjugate and a use thereof. Specifically, provided is a conjugate as shown in formula (II) or a stereoisomer thereof or a pharmaceutically acceptable salt thereof, wherein L is a linker (-GGFL-) as shown in formula (I). The conjugate comprising the linker as shown in formula (I) has high stability at a non-target site and can be quickly activated at a target site

R1-L-R2 Formula (II)

EP 4 682 156 A1

## Description

### TECHNICAL FIELD

The present invention belongs to the field of medicine, specifically, relates to a linker and drug conjugate using the same, and antibody-drug conjugate and use thereof.

### BACKGROUND

Conjugation technology has a wide range of applications in tumor treatment, disease diagnosis, and efficient screening. Among them, small molecule drug conjugates (SMDCs) and antibody drug conjugates (ADCs) are composed of targeting molecules or antibodies, linkers, and effector molecules (such as cytotoxic agents). The mechanisms of action of SMDC and ADC are similar. They usually enter the blood circulation through intravenous injection. After entering the tumor tissue through the blood circulation, they bind to the metastatic or associated receptors on the surface of the tumor cells. Then, under the mediation of the receptors, they undergo endocytosis to form endosomes. The endosomes then fuse with lysosomes, releasing cytotoxic molecules into the cytoplasm. After the cytotoxic molecules binding to DNA, microtubules, etc., their replication or mitosis are affected, ultimately leading to cell apoptosis.

In summary, there is an urgent need in the art to develop a new conjugate drug with improved stability and improved therapeutic effect.

### SUMMARY OF THE INVENTION

The purpose of the present invention is to provide a linker for drug conjugation, a tumor-targeting drug conjugate, an antibody drug conjugate, a pharmaceutical composition and uses thereof.

In the first aspect of the present invention, provided is a conjugate or a stereoisomer thereof, or a pharmaceutically acceptable salt thereof, the conjugate is of formula (II);

R1-L-R2 formula (II);

wherein

L is a linker (-GGFL-) as shown in formula (I);

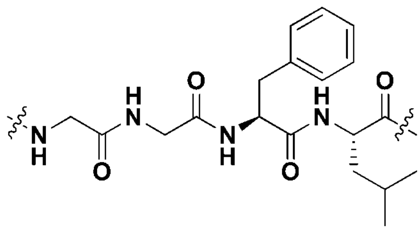

formula (I);

R1 is a group having one or more functions selected from the group consisting of:
activation of the tumor microenvironment, improvment of the water solubility of tumor-targeting drugs, and providing a coupling site to an antibody element;
R2 is a group derived from a drug having cytotoxicity or a drug carrying cytotoxicity.

In another preferred embodiment, the antibody element is an antibody or the antigen-binding domain thereof.

In another preferred embodiment, the conjugate is a tumor-targeting conjugate.

In another preferred embodiment, in the molecule of the drug having cytotoxicity or the drug carrying cytotoxicity, there exists at least one reactive group (such as an amino group, or an imino group (such as), or a hydroxyl group).

In another preferred embodiment, the group derived from a drug having cytotoxicity or a drug carrying cytotoxicity refers to a monovalent group formed by the reaction of the reactive group (such as an amino group, or an imino group, or a hydroxyl group) with a group such as -OC(O)$C_{1-6}$ alkyl, -COOH, or an amino group.

In another preferred embodiment, the group derived from a drug having cytotoxicity or a drug carrying cytotoxicity refers to a monovalent group formed by an amino group or an imino group losing a hydrogen, or losing the hydroxyl group or the hydrogen on the hydroxyl group.

In another preferred embodiment, the drug having cytotoxicity or the drug carrying cytotoxicity is selected from

the group consisting of: Doxorubicin, Daunorubicin, Epirubicin, Taxanes, Taxol, Docetaxel, topoisomerase inhibitors, Camptothecins, Irinotecan, Exatecan and its derivative DXd, TLR7/8 agonist, Resiquimod, Imiquimod, Loxoribine, 1-(4-aminobutyl)-2-butyl-1H-imidazo[4,5-C]quinolin-4-amine (T785) or its analogs, Cisplatin, Carboplatin, Rapamycin, Everolimus, Methotrexate, Fludarabine, Gemcitabine, Cytarabine, Melphalan, Nimustine, Mitomycin, Mitoxantrone, MMAE, MMAF, and MMAD.

In another preferred embodiment, R2 is -D1 or -Am-D2; wherein D1 is a monovalent group derived from a drug having $-NH_2$ or -NH- in its molecule, D2 is a monovalent group derived from a drug having -OH in its molecule, Am is -NH-$C_{1-6}$ alkylene-O-, and the NH terminal of Am is connected to L.

In another preferred embodiment, D1 is selected from groups shown in Table E1.

In another preferred embodiment, when R2 is D1, the conjugate is selected from the following Table B1:

Table B1

| | |
|---|---|
| 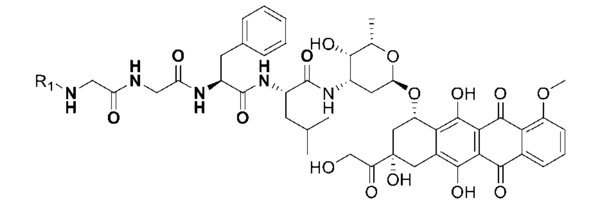 | 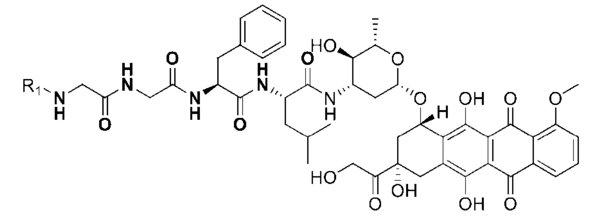 |
| 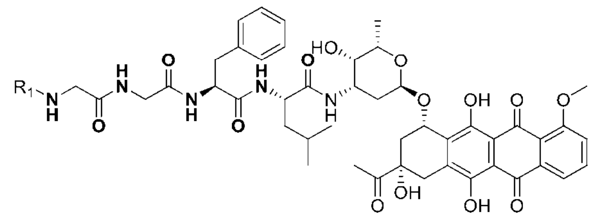 | 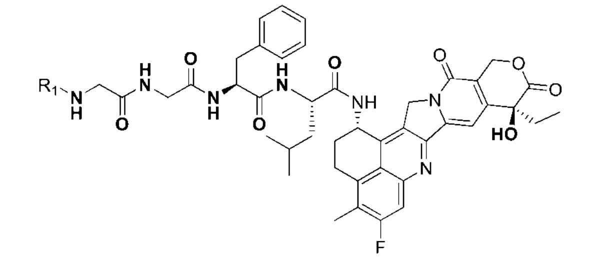 |

| | |
|---|---|
| 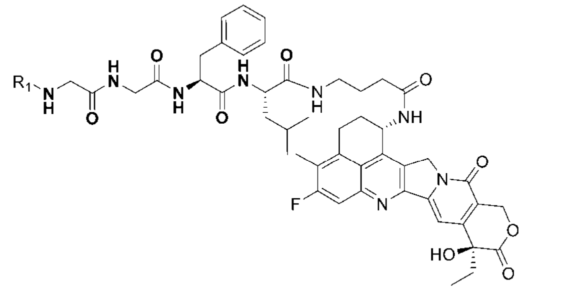 | 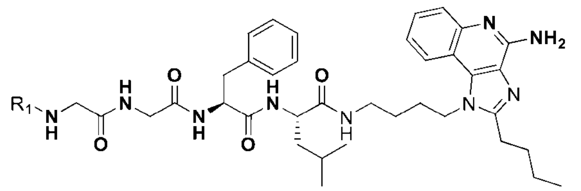 |
| 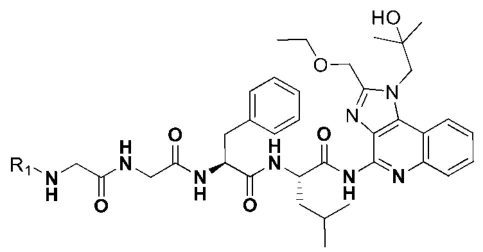 | 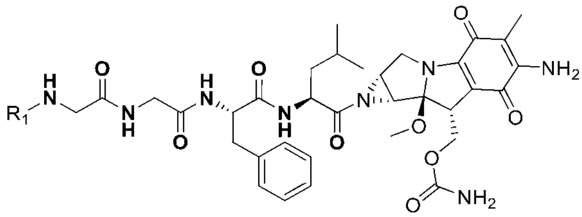 |
| 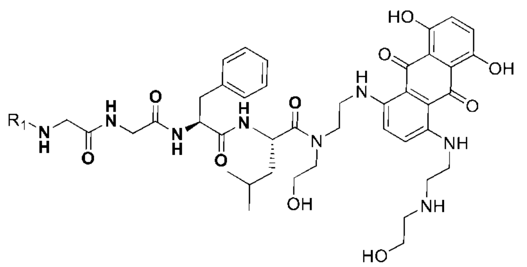 | 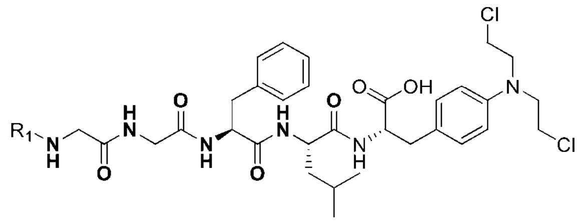 |
| 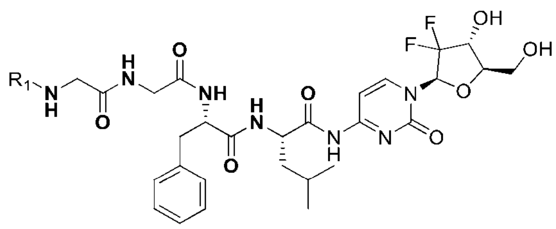 | |
| 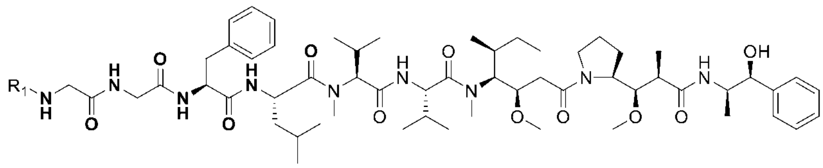 | |
| 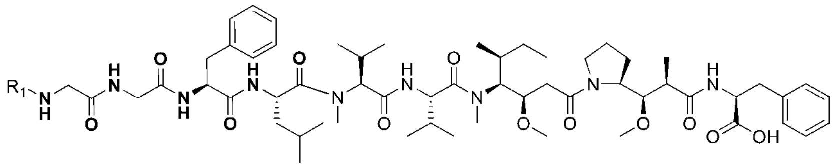 | |
| 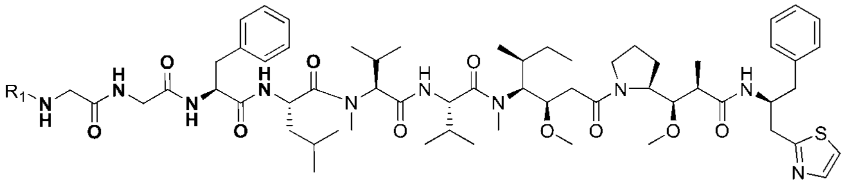 | |

In another preferred embodiment, -Am- is

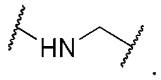

In another preferred embodiment, D2 is selected from groups shown in Table E2.

In another preferred embodiment, when R2 is -Am-D2, the conjugate is selected from the following Table B2:

Table B2

| | |
|---|---|
| 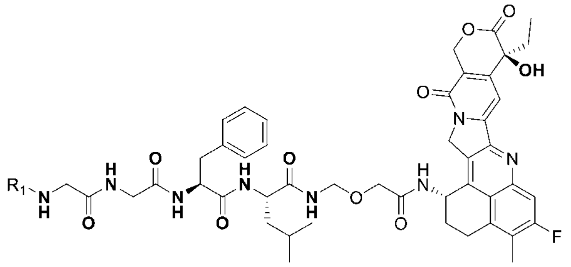 | 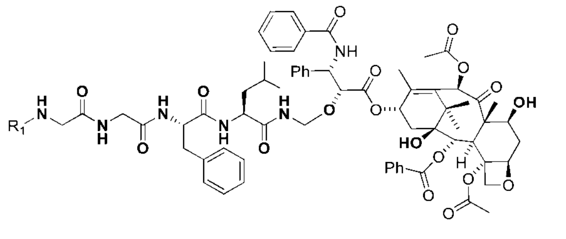 |
| 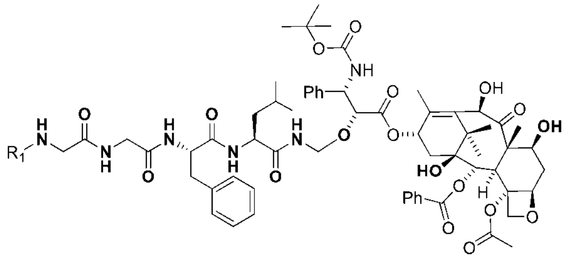 | 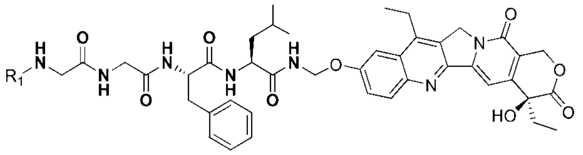 |
| 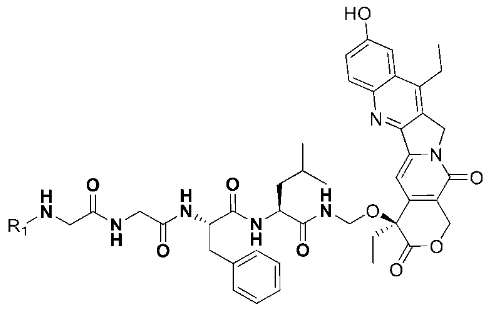 | 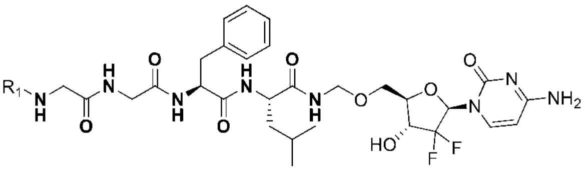 |
| 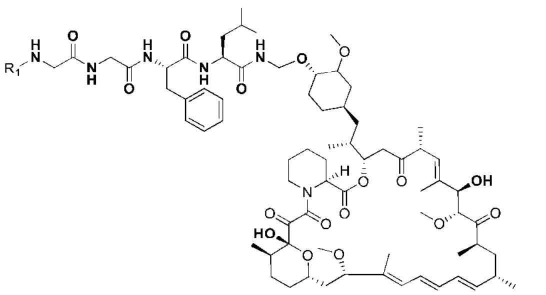 | 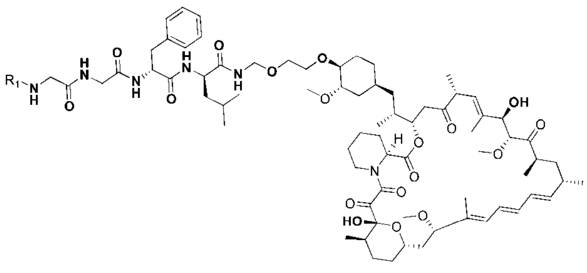 |

In another preferred embodiment, R1 contains one or more groups selected from the group consisting of: an activating group in the tumor microenvironment, a modifying group that improves the water solubility of tumor-targeting drugs, and a reactive group that can be coupled with antibodies.

In another preferred embodiment, R1 contains a reactive group that can be coupled with antibodies.

In another preferred embodiment, R1 contains a modifying group that improves the water solubility of tumor-targeting drugs.

In another preferred embodiment, the modifying group that improves the water solubility of tumor-targeting drugs is a PEG chain.

In another preferred embodiment, the improvement of the water solubility of the tumor-targeting drug refers to including a PEG chain structural unit in R1.

In another preferred embodiment, the PEG chain is a linear or branched PEG chain.

In another preferred embodiment, the PEG chain has 1 to 40 (preferably 1 to 20, more preferably 1 to 10) $-CH_2CH_2O-$ structural units.

In another preferred embodiment, providing a coupling site to an antibody means including a reactive group that can be coupled to an antibody in R1.

In another preferred embodiment, the reactive group that can be coupled to antibodies is selected from the group consisting of:

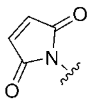, 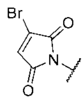, 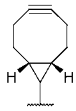, 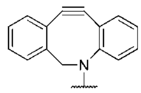, and 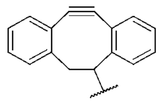.

In another preferred embodiment, the activating group in the tumor microenvironment is selected from the group consisting of:

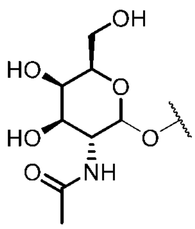

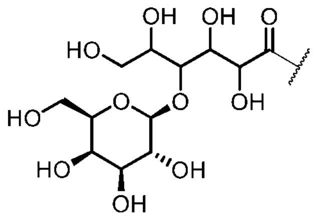

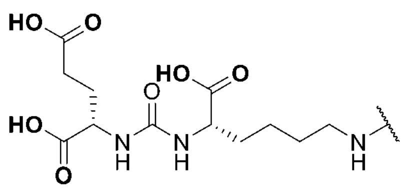

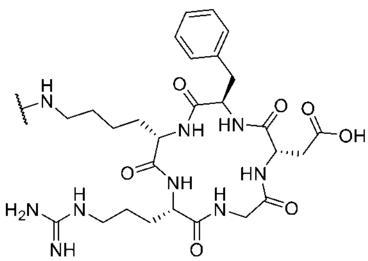

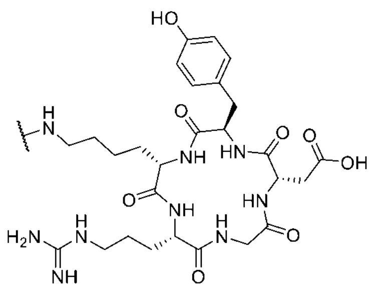.

In another preferred embodiment, R1 is selected from the following Table C1-A and C1-B

Table C1-A

| $R_{1-1}$ | $R_{1-7}$ | $R_{1-8}$ |
|---|---|---|
| 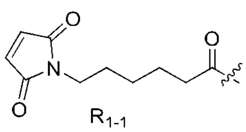 $R_{1-1}$ | 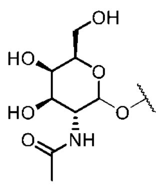 $R_{1-7}$ | 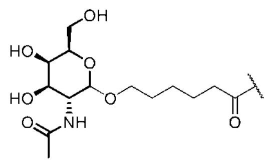 $R_{1-8}$ |
| $R_{1-9}$ | $R_{1-10}$ | $R_{1-11}$ |
| 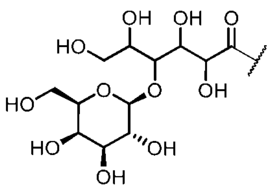 $R_{1-9}$ | 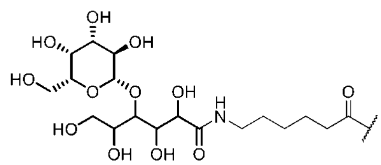 $R_{1-10}$ | 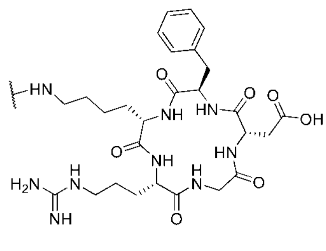 $R_{1-11}$ |
| $R_{1-12}$ | $R_{1-13}$ | |

(continued)

| | | |
|---|---|---|
| 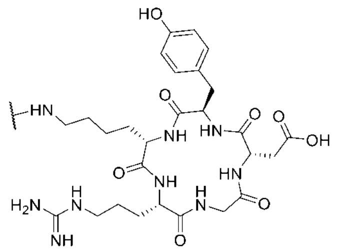 R1-12 | 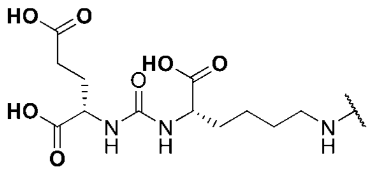 R1-13 | |
| $R_{1-25}$ | $R_{1-29}$ | $R_{1-37}$ |
| 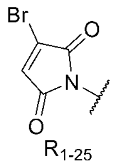 $R_{1-25}$ | 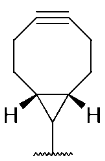 $R_{1-29}$ | 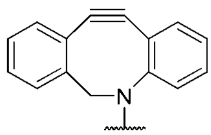 $R_{1-37}$ |
| $R_{1-42}$ | $R_{1-43}$ | $R_{1-45}$ |
| 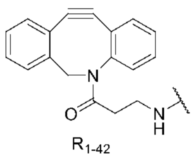 $R_{1-42}$ | 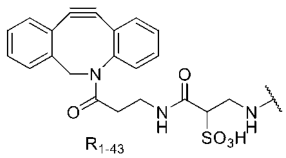 $R_{1-43}$ | 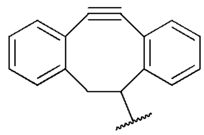 $R_{1-45}$ |
| $R_{1-44}$ | | |
| 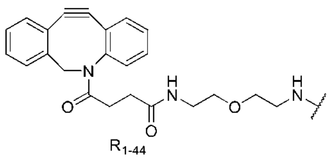 $R_{1-44}$ | | |

Table C1-B

| $R_{1-2}$ | $R_{1-3}$ | $R_{1-4}$ |
|---|---|---|
| 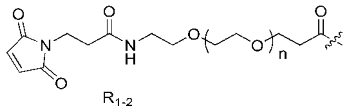 $R_{1-2}$ | 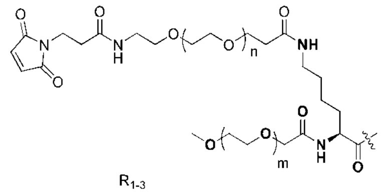 $R_{1-3}$ | 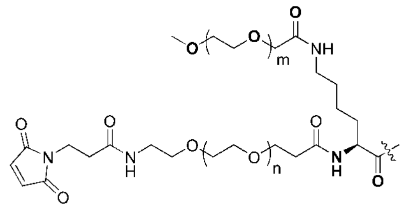 $R_{1-4}$ |
| $R_{1-5}$ | $R_{1-6}$ | |
| 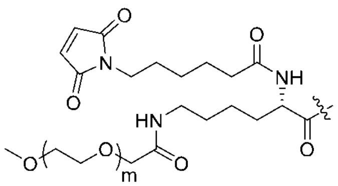 $R_{1-5}$ | 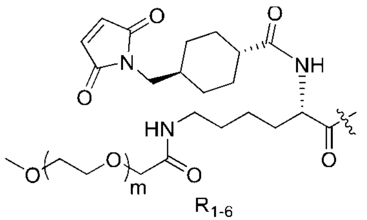 $R_{1-6}$ , | |
| $R_{1-26}$ | $R_{1-27}$ | $R_{1-28}$ |
| 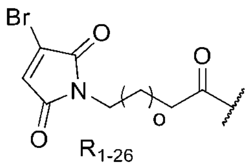 $R_{1-26}$ | 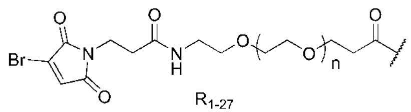 $R_{1-27}$ | 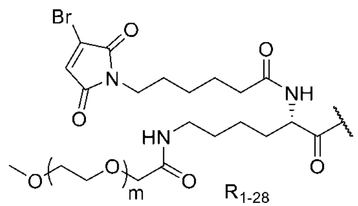 $R_{1-28}$ |
| $R_{1-30}$ | $R_{1-31}$ | $R_{1-32}$ |
| 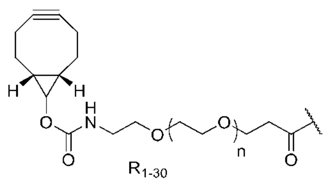 $R_{1-30}$ | 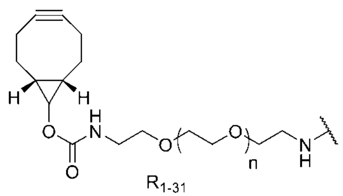 $R_{1-31}$ | 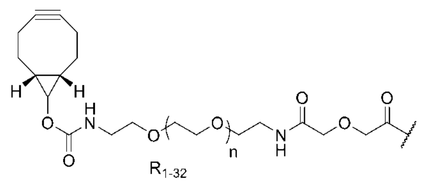 $R_{1-32}$ |
| $R_{1-33}$ | $R_{1-34}$ | $R_{1-35}$ |

(continued)

| | | |
|---|---|---|
| 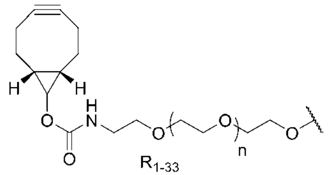 R1-33 | 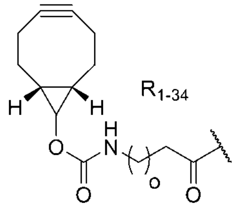 R1-34 | 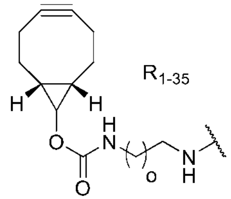 R1-35 |
| $R_{1-36}$ | | |
| 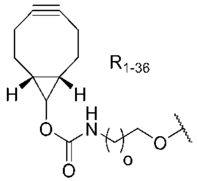 R1-36 | | |
| $R_{1-38}$ | $R_{1-39}$ | $R_{1-40}$ |
| 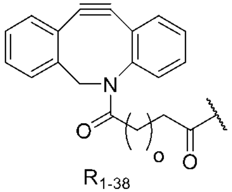 R1-38 | 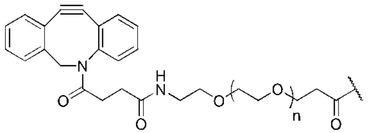 R1-39 | 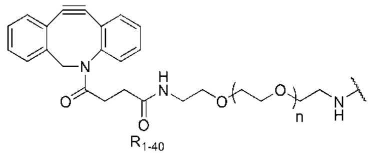 R1-40 |
| $R_{1-41}$ | | |
| 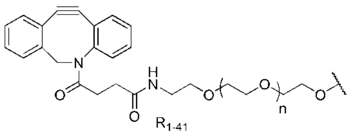 R1-41 | | |

in Table C1-B,

o is 0, or an integer from 1 to 20; preferably, is 0, or an integer from 1 to 10, more preferably, is an integer from 1 to 10 (such as 1, 2, 3, 4, 5, 6, 7, 8, 9, or 10);

m and n are each independently 0, or an integer from 1 to 20; preferably, m and n are each independently 0, or an integer from 1 to 10, more preferably, m and n are each independently an integer from 1 to 10 (such as 1, 2, 3, 4, 5, 6, 7, 8, 9, or 10).

In another preferred embodiment, R1 is a group formed by a click reaction between Ra and Rb.

In another preferred embodiment, Ra is

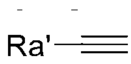,

and Rb is Rb'-$N_3$.

In another preferred embodiment, Ra is Ra'-$N_3$, and Rb is

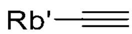.

In another preferred embodiment, Rb' is connected to L.

In another preferred embodiment, the group formed by a click reaction between Ra and Rb is

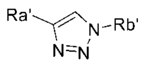.

In another preferred embodiment, Ra is selected from the following Table D1-A and D1-B:

Table D1-A

<table>
<tr><th>$R_{a-2}$</th><th>$R_{a-5}$</th><th>$R_{a-6}$</th></tr>
<tr><td>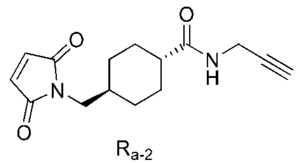<br>$R_{a-2}$</td><td>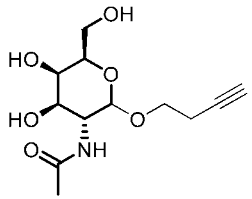<br>$R_{a-5}$</td><td>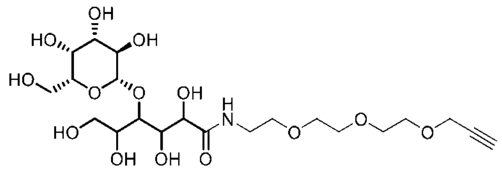<br>$R_{a-6}$</td></tr>
<tr><th colspan="3">$R_{a-7}$</th></tr>
<tr><td colspan="3">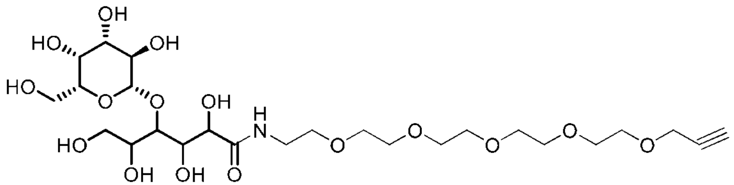<br>$R_{a-7}$</td></tr>
</table>

Table D1-B

| $R_{a-1}$ | $R_{a-3}$ |
|---|---|
| 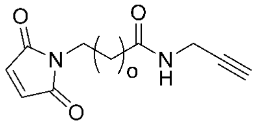 $R_{a-1}$ | 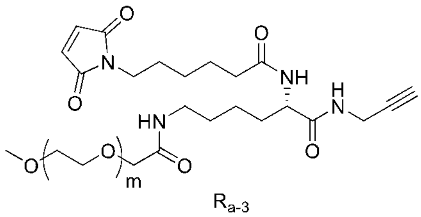 $R_{a-3}$ |
| $R_{a-4}$ | $R_{a-8}$ |
| 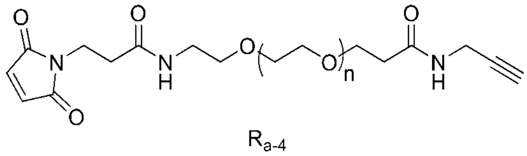 $R_{a-4}$ | 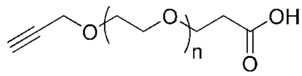 Ra-8 |
| $R_{a-9}$ | |
| 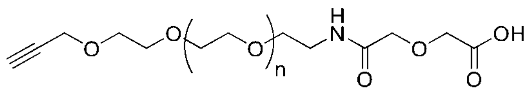 Ra-9 | |

in Table D1-B,

o is 0, or an integer from 1 to 20; preferably, is 0, or an integer from 1 to 10, more preferably, is an integer from 1 to 10 (such as 1, 2, 3, 4, 5, 6, 7, 8, 9, or 10);
m and n are each independently 0, or an integer from 1 to 20; preferably, are 0, or an integer from 1 to 10, more preferably, are an integer from 1 to 10 (such as 1, 2, 3, 4, 5, 6, 7, 8, 9, or 10).

In another preferred embodiment, Rb is selected from the following Table D2-A and D2-B:

Table D2-A

| $R_{b-1}$ | $R_{b-4}$ |
|---|---|
| 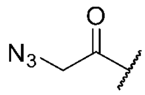 $R_{b-1}$ | 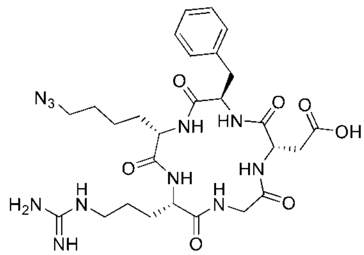 Rb-4 |
| $R_{b-5}$ | $R_{b-7}$ |

(continued)

| | |
|---|---|
| 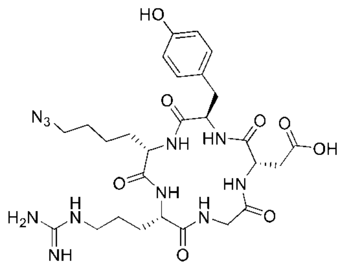 Rb-5 | 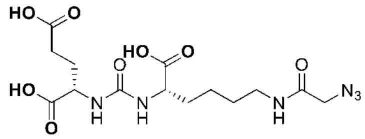 Rb-7 |

Table D2-B

| $R_{b-2}$ | $R_{b-3}$ |
|---|---|
| 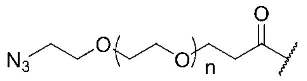 $R_{b-2}$ | 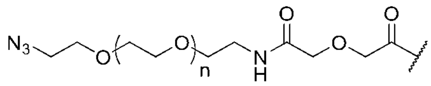 $R_{b-3}$ |

wherein n is each independently 0, or an integer from 1 to 20; preferably, is 0, or an integer from 1 to 10, more preferably, is an integer from 1 to 10 (such as 1, 2, 3, 4, 5, 6, 7, 8, 9, or 10).

In another preferred embodiment, R1 is selected from the following Table C2-A and C2-B:

Table C2-A

| | |
|---|---|
| $R_{1-17}$ | 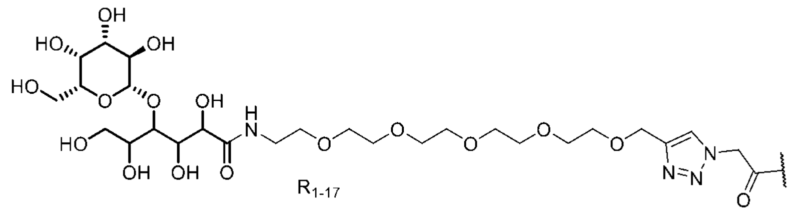 $R_{1-17}$ |
| $R_{1-18}$ | 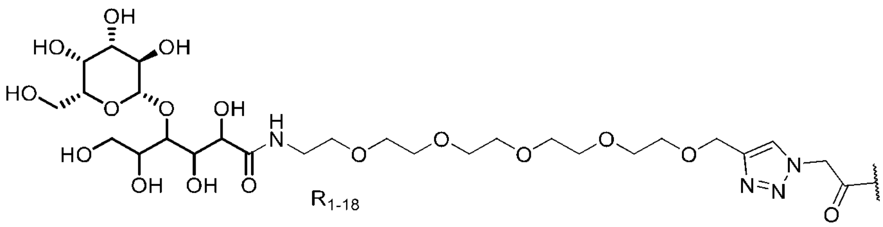 $R_{1-18}$ |

Table C2-B

| | |
|---|---|
| $R_{1-14}$ | 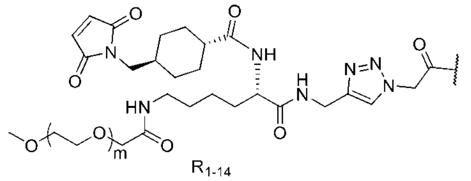 $R_{1-14}$ |
| $R_{1-15}$ | 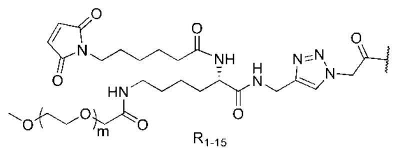 $R_{1-15}$ |

(continued)

| | |
|---|---|
| $R_{1-16}$ | 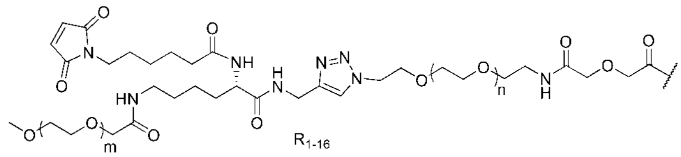 $R_{1-16}$ |
| $R_{1-19}$ | 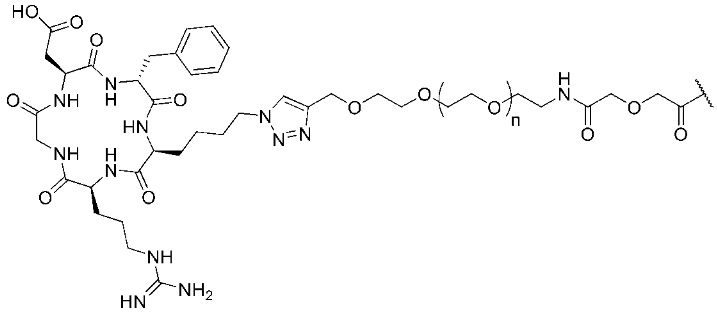 $R_{1-19}$ |
| $R_{1-20}$ | 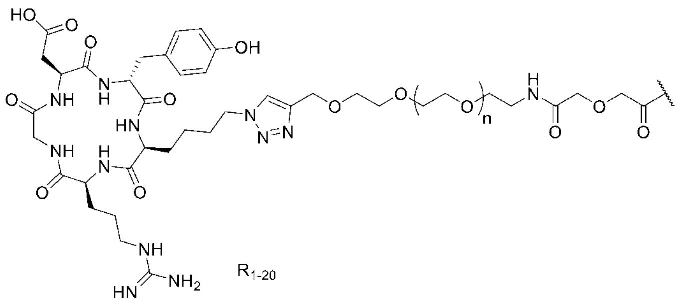 $R_{1-20}$ |
| $R_{1-21}$ | 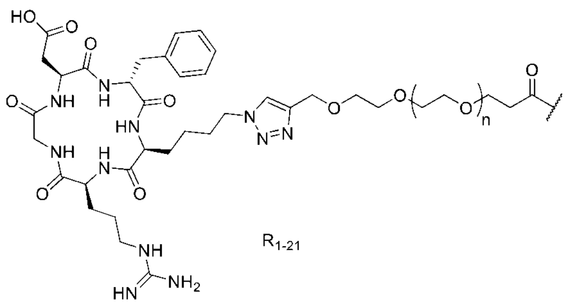 $R_{1-21}$ |
| $R_{1-22}$ | 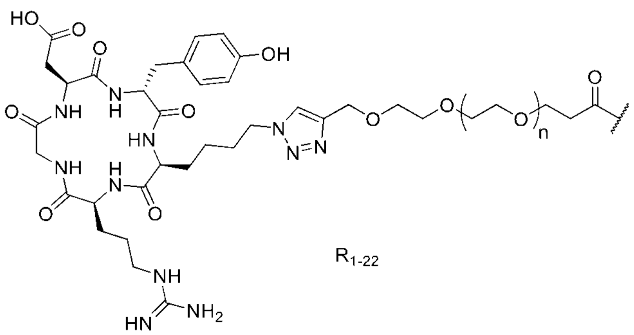 $R_{1-22}$ |
| $R_{1-23}$ | 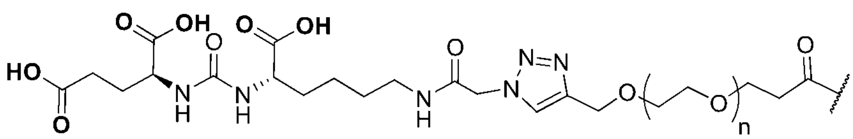 $R_{1-23}$ |

(continued)

| | |
|---|---|
| $R_{1-24}$ | 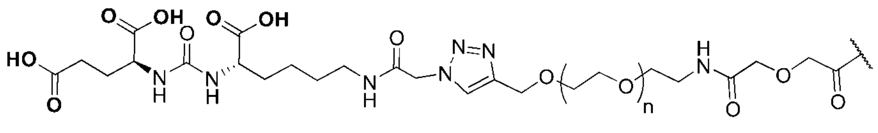 $R_{1-24}$ |

wherein m and n are each independently 0, or an integer from 1 to 20; preferably, are 0, or an integer from 1 to 10, more preferably, are an integer from 1 to 10(such as 1, 2, 3, 4, 5, 6, 7, 8, 9, or 10).

In another preferred embodiment, the conjugate is selected from Table A1, Table A2 and Table A3.

In another preferred embodiment, the conjugate is selected from Table A1.

In another preferred embodiment, the conjugate can release substances having cytotoxicity or carrying cytotoxicity in the tumor environment.

In another preferred embodiment, Cathepsin L is present in the tumor environment.

In another preferred embodiment, the substance having cytotoxicity or carrying cytotoxicity is a drug having cytotoxicity or carrying cytotoxicity or a derivative thereof (the derivative refers to a derivative being modified in order to be connected to L).

In another preferred embodiment, in the tumor environment, the release rate of the conjugate releasing substances having cytotoxicity or carrying cytotoxicity within 60 hours (preferably within 40 hours, more preferably within 20 hours) is >80%, preferably >85%, and more preferably >90%, based on the total molar amount of the conjugate.

In another preferred embodiment, the conjugate substantially does not release substances having cytotoxicity or carrying cytotoxicity in a non-tumor environment (such as the environment of blood, plasma, heart, and liver).

In another preferred embodiment, in the non-tumor environment, the release rate of the conjugate releasing substances having cytotoxicity or carrying cytotoxicity over 2 hours (preferably over 10 hours, more preferably over 20 hours) is < 10%, based on the total molar amount of the conjugate.

In another preferred embodiment, the ratio of the release rate of the conjugate in a tumor environment to that in a non-tumor environment over 20 hours (i.e., the release rate in the presence of cathepsin L or in the tumor environment / the release rate in the absence of cathepsin L or in the non-tumor environment) is > 10, preferably > 15.

In the second aspect of the present invention, provided is an antibody conjugate or a stereoisomer thereof, or a pharmaceutically acceptable salt thereof, the antibody conjugate is as shown in formula (III),

$$R3\text{-}(R1'\text{-}L\text{-}R2)_x \qquad \text{Formula (III);}$$

wherein

R3 refers to an antibody moiety,
R1'-L-R2 refers to a group formed by the conjugation of the conjugate of formula (II) with the antibody element through the R1 group; wherein, R1, L, and R2 are as defined in claim 1; R1' is a group formed by the conjugation of R1 with the antibody element; and
x is 1 to 8.

In another preferred embodiment, R1 is a group having

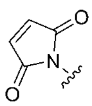,

and R1' is a group obtained from

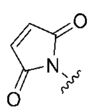

in R1 after being coupled to form

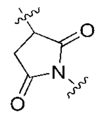
.

In another preferred embodiment, R1 is a group having

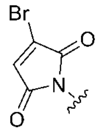
,

and R1' is a group obtained from

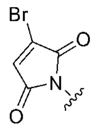

in R1 after being coupled to form

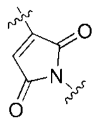
.

In another preferred embodiment, R1 is a group having

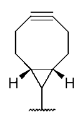
,

and R1' is a group obtained from

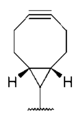

in R1 after being coupled to form

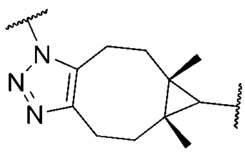
or
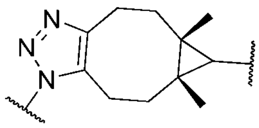
.

In another preferred embodiment, R1 is a group having

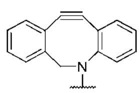
or
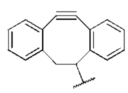
,

and R1' is a group obtained from

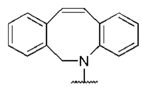 or 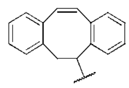

in R1 after being coupled to form:

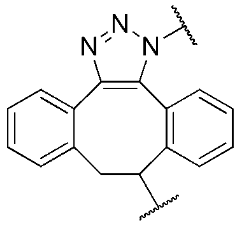 or 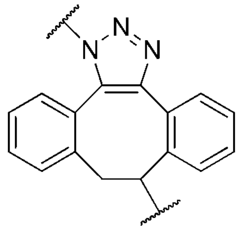 ,

or,

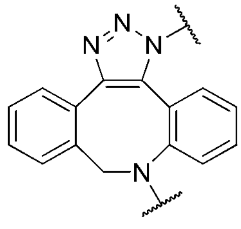 or 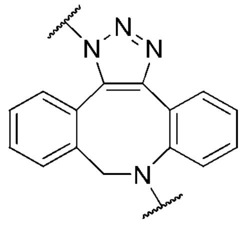 .

In another preferred embodiment, the antibody in the antibody moiety may be unmodified and can form a connecttion with the R1 group of the conjugate via a group on an amino acid side chain in the antibody (such as -SH in a cysteine residue in the antibody).

In another preferred embodiment, the antibody in the antibody moiety may be modified with an active linking group (such as an alkynyl group), thereby forming a connection with the R1 group of the conjugate via the modified active group.

In another preferred embodiment, the antibody is selected from the group consisting of: HER2, CD19, CD20, EGFR, CD22, CD3, TROP2, Glycoprotein NMB, Guanylyl cyclase C, CEA, AXL, GCC, CD79b, PSMA, ENPP3, Mesothelin, CD138, NaPi2b, CD56, CD74, FOLR1, DLL3, CEACAM5, CD142, SLAMF7, CD25, SLTRK6, CD37, CD70, AGS-22, C4.4A, FGFR2, Ly6E, MUC16, BCMA, pCadherin, Ephrin-A, LAMP1, MUC1, PDL1, HER2, NY-ESO-1, BCMA, WT1, MUC1, CD20, CD23, ROR1, CD123, CD33, CD44v6, CD174, CD30, CD133, cMet, FAP, EphA2, GD2, GPC3, IL-13Ra2, LewisY, SS1, CD171, EGFR, EGFRvIII, VEGFR2, NY-ESO-1, MUC-1 , and MAGE-A3, or combinations thereof.

In the third aspect of the present invention, provided is a pharmaceutical composition, comprising: (i) the conjugate or the stereoisomer thereof, or the pharmaceutically acceptable salt thereof according to the first aspect, or the antibody conjugate or the stereoisomer thereof, or the pharmaceutically acceptable salt thereof according to the second aspect; and (ii) pharmaceutically acceptable carriers.

In the fourth aspect of the present invention, provided is use of the conjugate according to the first aspect, or the antibody conjugate according to the second aspect in the preparation of a medicament for treating or preventing tumor or inflammation.

In another preferred embodiment, the tumor is selected from the group consisting of: bladder cancer, brain cancer, breast cancer, cervical cancer, colorectal cancer, esophageal cancer, kidney cancer, liver cancer, lung cancer, nasopharyngeal cancer, pancreatic cancer, prostate cancer, skin cancer, stomach cancer, uterine cancer, ovarian cancer, testicular cancer, and blood cancer, or a combination thereof.

In the fifth aspect of the present invention, provided is a linker, the linker is as shown in formula (I);

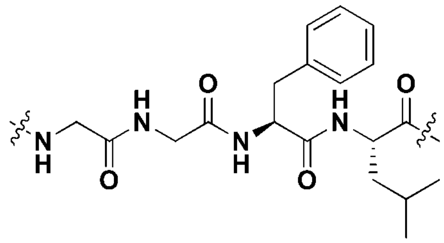

Formula (I).

In another preferred embodiment, the linker is used for coupling a drug.

In another preferred embodiment, the linker is selective.

In another preferred embodiment, the linker can be cleaved by cathepsin L.

In another preferred embodiment, the linker is essentially incapable of being cleaved by enzymes other than cathepsin L (such as cathepsin B).

In another preferred embodiment, in the tumor environment, >80%, preferably >85%, and more preferably >90% of the conjugate having the linker can be cleaved by enzymes within 60 hours (preferably within 40 hours, more preferably within 20 hours), based on the total molar amount of the conjugate.

In another preferred embodiment, in a non-tumor environment (such as the environment of blood, plasma, heart, and liver), the conjugate having the linker is essentially incapable of being cleaved by enzymes.

In another preferred embodiment, in the non-tumor environment, <10% of the conjugate having the linker can be cleaved by enzymes over 2 hours (preferably over 10 hours, more preferably over 20 hours), based on the total molar amount of the conjugate.

It should be understood that within the scope of the present invention, the above technical features of the present invention and the technical features specifically described in the following (e.g/, embodiments) can be combined with each other, thereby forming a new or preferred technical solution. Due to space limitations, it will not be repeated herein.

## DESCRIPTION OF THE DRAWINGS

Fig. 1 shows a graph showing that L-AM-DXD cannot be cleaved by cathepsin L in Example 5;
Fig. 2 shows a graph showing that EMC-GGFG-AM-DXD was cleaved by cathepsin L in Example 6;
Fig. 3 shows a graph showing that MI-PEG6-GGFL-AM-DXD was cleaved by cathepsin L in Example 6;
Fig. 4 shows a graph showing that MI-PEG6-GGFL-AM-DXD cannot be cleaved by cathepsin B in Example 7;
Fig. 5 shows a graph showing that EMC-GGFG-AM-DXD was cleaved by cathepsin B in Example 7;
Fig. 6 shows a stability test graph of EMC-GGFG-AM-DXD in human plasma in Example 8;
Fig. 7 shows a stability test graph of MI-PEG6-GGFL-AM-DXD in human plasma in Example 8;
Fig. 8 shows a stability test graph of GGFG-AM-DXD in monkey heart homogenate in Example 9;
Fig. 9 shows a stability test graph of MI-PEG6-GGFL-AM-DXD in monkey heart homogenate in Example 9;
Fig. 10 shows a stability graph of MI-PEG6-GGFL-AM-DXD in monkey liver homogenate in Example 10;
Fig. 11 shows a stability graph of EMC-GGFG-AM-DXD in monkey liver homogenate in Example 10;
Fig. 12 shows a stability graph of GGFG-AM-DXD in human gastric cancer tumor homogenate in Example 11;
Fig. 13 shows a stability graph of MI-PEG6-GGFL-AM-DXD human gastric cancer tumor homogenate in Example 11;
Fig. 14 shows a stability graph of GGFG-AM-DXD in CT26 tumor homogenate in Example 12;
Fig. 15 shows a stability graph of GGFL-AM-DXD in CT26 tumor homogenate in Example 12;
Fig. 16 shows the *in vivo* therapeutic effect of the antibody-drug conjugates based on GGFL linker and GGFG linker in Example 13 in mice.

## EMBODIMENTS FOR CARRYING OUT THE INVENTION

After extensive and in-depth research, the present inventors unexpectedly found that the -GGFL- linker (i.e., the linker represented by formula (I)) has excellent selectivity and can only be cleaved by cathepsin L to release cytotoxic substances, while being stable in other environments where cathepsin L is not present (such as liver, heart, plasma, etc.), thereby providing a drug that has extremely high safety outside the tumor and effectively releases cytotoxic substances in the tumor, thereby effectively treating the tumor. Based on this, the inventors completed the present invention.

**TERMS**

As used herein, the terms "conjugate of the present invention" and "drug conjugate of the present invention" can be used interchangeably and refer to a kind of conjugate of formula (II) having a linker of formula (I) (i.e., -GGFL-).
As used herein, the terms "antibody conjugate of the present invention" and "antibody-drug conjugate of the present invention" can be used interchangeably and refer to an antibody-conjugate formed by an antibody (Ab) or its active fragment or its antigen-binding domain and the conjugate of formula (II) or the pharmaceutically acceptable salt or solvent compound thereof.
Unless otherwise defined, all technical and scientific terms used herein have the same meaning as would normally be understood by those of ordinary skill in the art to which the invention belongs. As used herein, term "about" means that the value may change by no more than 1% from the enumerated value when used in reference to a specific enumerated value. For example, the expression "about 100" includes 99 and 101 and all values between them (e. g., 99.1, 99.2, 99.3, 99.4, etc.).
As used herein, the term "amino acid residue" refers to a group formed by the removal of an H from $-NH_2$ at the N-terminal and the removal of -OH from -COOH at the C-terminal of an amino acid. Generally, the chain segment of an amino acid (residue) containing the N-terminal and the C-terminal is called the main chain, and the part that determines the specific type of an amino acid is called the side chain. Unless otherwise defined, in the present invention, amino acids include natural or non-natural amino acids, including D and/or L-type amino acids. Examples of amino acids include, but are not limited to, Ala (A), Arg (R), Asn (N), Asp (D), Cys (C), Gln (Q) Glu (E), Gly (G), His (H), Ile (I), Leu (L), Lys (K), Met (M), Phe (F), Pro (P), Ser (S), Thr (T), Trp (W), Tyr (Y), Val (V). Preferably, the amino acid used herein is an amino acid selected from the group consisting of: L-glycine(L-Gly), L-alanine (L-Ala), β-alanine (β-Ala), L-glutamic acid(L-Glu), L-aspartic acid (L-Asp), L-histidine (L-His), L-Arginine (L-Arg), L-Lysine (L-Lys), L-Valine (L-Val), L-Serine (L-Ser), and L-Threonine(L-Thr); In addition, when an amino acid has 2 or more amino groups and/or 2 or more carboxyl groups, the term also includes groups formed by the removal of a H from - $NH_2$ and the removal of an -OH from -COOH on different carbon atoms, such as the divalent group $-C(O)-(CH_2)_2-C(COOH)-NH-$ formd by the removal of H from $-NH_2$ and the removal of H from -COOH on non-α-position in a glutamic acid, respectively.
In the present invention, the term "pharmaceutically acceptable" component(s) refer to a substance suitable for use in human and/or animals without excessive adverse side reactions (such as toxicity, stimulation and allergic reaction), i.e. a substance with reasonable benefit/risk ratio.
In the present invention, the term "effective amount" refers to an amount of a therapeutic agent that treats, alleviates or prevents target disease or condition, or an amount that exhibits a detectable therapeutic or preventive effect. The precise effective amount for a subject will depend on the subject's size and health, the nature and extent of the condition, and the therapeutic agent and/or combination of therapeutic agents selected for administration. Therefore, it is not useful to specify an exact effective amount in advance. However, for a given situation, the effective amount can be determined using routine experimentation, which is within the judgment of the clinician.
Unless otherwise specified, all compounds present in the present invention are intended to include all possible optical isomers, such as single chiral compounds, or mixtures of various chiral compounds (i.e. racemes). Among all compounds of the present invention, each chiral carbon atom may optionally be of the R or S configuration, or a mixture of the R and S configurations.
As used herein, the term "pharmaceutically acceptable salt" refers to a salt formed by a compound of the present invention with an acid or base that is suitable for use as a medicine. Pharmaceutically acceptable salts include inorganic salts and organic salts. A group of preferred salts are salts formed of the compound of the present invention and the acids. Acids suitable for forming salts include, but are not limited to, inorganic acids such as hydrochloric acid, hydrobromic acid, hydrofluoric acid, sulfuric acid, nitric acid, phosphoric acid and the like; organic acids such as methanoic acid, acetic acid, propionic acid, oxalic acid, malonic acid, succinic acid, fumanic acid, maleic acid, lactic acid, malic acid, tartaric acid, citric acid, bitter acid, methanesulfonic acid, benzenemethanesulfonic acid, benzenesulfonic acid, and the like; and acidic amino acids such as aspartic acid, glutamic acid and the like.
Unless otherwise specified, "amino acid" as used herein is intended to include any conventional amino acid, such as aspartic acid, glutamic acid, cysteine, asparagine, phenylalanine, glutamine, tyrosine, serine, methionine (methionine), tryptophan, glycine, valine, leucine, alanine, isoleucine, proline, threonine, histidine, lysine, and arginine.
When a trade name is used herein, the trade name is intended to include the trade name product formulation, its corresponding generic drug, and the active pharmaceutical ingredient of the trade name product.

**Antibody**

As used herein, the term "antibody element" includes antibody or the antigen-binding domain thereof. Preferred antibody elements include antibodies (such as intact antibodies, single-chain antibodies, nanobodies, antibody fragments), especially those antibodies against tumor cell markers (such as tumor markers located on the surface of tumor

cells).

As used herein, the term "antibody" or "immunoglobulin" is a heterotetrameric glycoprotein of about 150,000 daltons with the same structural characteristics, which consists of two identical light chains (L) and two identical heavy chains (H). Each light chain is connected to the heavy chain via a covalent disulfide bond, and the number of disulfide bonds between the heavy chains of different immunoglobulin isotypes are different. Each heavy and light chain also has regularly spaced intrachain disulfide bonds. Each heavy chain has a variable region (VH) at one end, followed by multiple constant regions. There is a variable region (VL) at one end of each light chain and a constant region at the other end. The constant region of the light chain is relative to the first constant region of the heavy chain, and the variable region of the light chain is relative to the variable region of the heavy chain. Special amino acid residues form an interface between the variable regions of the light chain and the heavy chain.

As used herein, the terms "single-domain antibody" and "nanobody" have the same meaning, and refer to cloning the variable region of the heavy chain of an antibody, and constructing a single-domain antibody consisting of only one heavy chain variable region, which is the smallest antigen-binding fragment that has complete functions. Usually, after obtaining an antibody naturally missing the light chain and the constant region 1 (CH1) of the heavy chain, the variable region of the antibody heavy chain is cloned to construct a single domain antibody consisting of only one heavy chain variable region.

As used herein, the term "variable" means that certain parts of the variable region of the antibody are different in sequence, which forms the binding and specificity to specific antigens of various specific antibodies. However, variabilities are not evenly distributed throughout the variable regions of antibodies. It is concentrated in three fragments that are called complementarity determining regions (CDR) or hypervariable regions in the variable regions of light chain and heavy chain. More conservative parts of the variable region are called the framework region (FR). The variable regions of the natural heavy and light chains each contain four FR regions, which are in a roughly β-folded conformation and are linked by three CDRs that form a linking loop, which in some cases can form a partially folded structure. The CDRs in each chain are closely placed together through the FR regions and form the antigen binding site of the antibody together with the CDRs in the other chain. Constant regions do not directly participate in the binding of antibodies to antigens, but they exhibit different effector functions, such as participating in antibody-dependent cytotoxicity of antibodies.

The "light chains" of vertebrate antibodies (immunoglobulins) can be classified in one of two distinct categories (called κ and λ) based on the amino acid sequence of constant regions thereof. According to the amino acid sequence of the constant region in heavy chain thereof, immunoglobulins can be classified into different types. There are five main classes of immunoglobulins: IgA, IgD, IgE, IgG and IgM, some of which can be further classified into subclasses (isotypes), such as IgG1, IgG2, IgG3, IgG4, IgA and IgA2. The constant regions in heavy chains corresponding to different classes of immunoglobulins are called α, δ, ε, γ, and μ, respectively. The subunit structures and three-dimensional configurations of different classes of immunoglobulins are well known to those skilled in the art.

Generally, the antigen-binding properties of antibodies can be described by 3 specific regions located in the variable regions of the heavy and light chains, called variable regions (CDR), which are divided into 4 framework regions (FRs). The amino acid sequence of 4 FRs is relatively conservative and does not directly participate in the binding reaction. These CDRs form a circular structure, and are made closer to each other in space structure through the β-pleated sheet formed by the FRs in between, and the CDRs on the heavy chain and the corresponding CDRs on the light chain constitute the antigen binding site of the antibody. It can be determined by comparing the amino acid sequences of antibodies of the same type which amino acids constitute the FR or CDR regions.

In the present invention, the polypeptide elements can include not only intact antibodies, but also fragments of antibodies with immunological activity (such as Fab or $(Fab')_2$ fragment; heavy chain of antibodies; or light chain of antibodies) or fusion proteins formed by antibodies and other sequences. Therefore, the present invention also includes fragments, derivatives and analogs of the antibodies.

**Cathepsin**

Papain-like cysteine proteases in mammals belong to Cathepsin, which are intracellular proteases mainly present in lysosomes. They are easily activated in weakly acidic environments and are a type of glycoprotein that is unstable in alkaline and neutral solutions (except cathepsins D, E, and S). Cathepsin is formed by the hydrolysis of inactive preprocathepsin, the *in vivo* synthesis pathway thereof is as follows: first, it is synthesized in the form of preprocathepsin on the ribosome-bound membrane, enters the endoplasmic reticulum through transferrin, and then enters the Golgi apparatus, at the same time, it is glycosylated and phosphorylated to form mannose-6-phosphate protein; finally, it is recognized by the mannose-6-phosphate-specific receptor on the lysosome and indirectly transported to the lysosome. In recent years, studies have shown that cathepsins are increasingly believed to play a role in lesions, tumor invasion, immune system -related diseases, and various parasitic infections. The main cause of death in cancer patients is the degradation of the extracellular matrix and basement membrane, which allows tumor cells to leave the tumor tissue and metastasize. The proteases required for the degradation process include a variety of cathepsins, which promote the

spatial expansion of tumors, the formation of tumor blood vessels, and the metastasis of tumor cells inside and outside the blood vessels through proteolysis. Upregulated expression of cathepsins, especially cathepsin B, has been found in many human tumors.

Most cathepsins are intracellular enzymes located in lysosomes. They are the main participants in proteolysis in the human body and are closely related to many major diseases such as human tumors, osteoporosis, and arthritis. They are also a type of target protease that has attracted much attention in recent years. According to their substrate specificity, they can be divided into endopeptidases, exopeptidases, aminopeptidases and carboxypeptidases; according to their proteolytic mechanism, they can be classified into serine proteases, cysteine proteases, aspartic acid proteases, threonine proteases and metalloproteases. Currently, the most studied is cathepsin B, since this enzyme is highly expressed in many tumor cancer cells and is a specific intracellular enzyme. Therefore, it is also the main linker cleavage enzyme considered in the development of ADC drugs, thereby achieving specific and efficient intracellular enzymatic cleavage. Cathepsin L is a major member of the lysosomal cysteine protease family and has a very unique synthesis and transport mechanism. The precursor peptide of precursor zymogen of Cathepsin L contains the ERF/WNIN motif and the GNFD motif. The spatial structure of cathepsin L is mainly composed of the L domain composed of $\alpha$-helices and the R domain composed of $\beta$-folds. Extensive research has shown that cathepsin L plays extremely important functions in physiological and pathological processes *in vivo,* as well as in parasitic animals. Its physiological effects are widely involved in protein hydrolysis, antigen presentation, T cell sorting, cell apoptosis, and embryonic development. Cathepsin L is also closely related to the occurrence of various types of tumors, cardiovascular diseases, and kidney diseases.

Shyam S et al. studied the expression of cathepsin L in human tumor cells, where cancers overall expressed higher levels of cathepsin L than that of normal tissues. Kidney and testicular tumors expressed the highest levels of cathepsin L. Non-small cell lung cancer had the next highest expression, and other cancers such as breast, ovarian, colon, adrenal, bladder, prostate, and thyroid cancers also had higher expression. Cathepsin L may be proven to be a useful diagnostic marker for human malignancies.

Dhhivya R et al. studied the targeted therapy of cathepsin L in cancer and believed that cathepsin L is a common phenomenon in human cancer and is closely related to metastasis, invasiveness and poor patient prognosis; cathepsin L is also believed to contribute to cancer-related osteolysis; in addition, the mechanism by which cathepsin L promotes progressive tumors and metastasis is emphasized, and the therapeutic effect of cathepsin L intervention strategies aimed at preventing metastatic progression and bone resorption is discussed.

## Drugs

As used herein, "drug" refers to any drug having the desired biological activity and a reactive functional group (such as amino ($-NH_2$), hydroxyl (-OH), etc.) for preparing the conjugate of the present invention or for forming a covalent bond with the linker of the present invention. Desirable biological activities include the diagnosis, cure, alleviation, treatment, and prevention of disease in humans or other animals. Thus, the term "drug" refers to compounds including those identified in official national pharmacopeias, as well as, for example, the official Homeopathic Pharmacopoeia of the United States, the official National Formulary, or any supplements thereto, so long as the requisite reactive functional groups are present. Typical medications are listed in the Physician's Desk Drug Reference (PDR) and the U.S. Food and Drug Administration's (FDA) Orange Book. It should be understood that as new drugs are continuously discovered and developed, these drugs should also be included in the "drugs" in the drug conjugate of the present invention.

Preferably, the drugs that can be used to constitute the present invention include but are not limited to: a drug having cytotoxicity or a drug carrying cytotoxicity.

The term "a drug having cytotoxicity or a drug carrying cytotoxicity" refers to a substance that inhibits or prevents cell expression activity, cell function and/or causes cell destruction. The term includes radioisotopes, chemotherapeutic agents, and toxins, such as small molecule toxins or enzymatically active toxins of bacterial, fungal, plant, or animal origin, including fragments and/or variants thereof. Preferably, the drug having cytotoxicity or the drug carrying cytotoxicity include but not limited to: Doxorubicin, Daunorubicin, Epirubicin, Taxanes, Taxol, Docetaxel, topoisomerase inhibitors, Camptothecins, Irinotecan, Exatecan and its derivative DXd, TLR7/8 agonist, Resiquimod, Imiquimod, Loxoribine, 1-(4-aminobutyl)-2-butyl-1H-imidazo[4,5-C]quinolin-4-amine (T785) or analogs, Cisplatin, Carboplatin, Rapamycin, Everolimus, Methotrexate, Fludarabine, Gemcitabine, Cytarabine, Melphalan, Nimustine, Mitomycin, Mitoxantrone, MMAE, MMAF, and MMAD.

## Linker

As used herein, the term "linker" refers to a divalent linking group used to connect two different functional moieties. In one embodiment of the present application, provided is a linker as shown in formula (1) (i.e., -GGFL-)

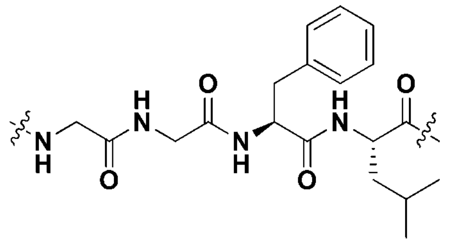

Formula (I).

This linker can be efficiently cleaved by cathepsin L in a tumor environment, but is stable in a non-tumor environment. As a result, the conjugate (or drug conjugate) and antibody conjugate (or antibody drug conjugate) using the linker of the present invention remain stable before reaching the target site (such as the tumor site), and are rapidly activated after reaching the target site (such as the tumor site), thereby effectively treating the tumor while avoiding excessive side effects (such as off-target toxicity).

**Drug Conjugate (or referred as Conjugate)**

In one embodiment of the present invention, provided is a conjugate (or drug conjugate) using the linker of formula (I).
Preferably, provided in the present invention is a tumor-targeting drug conjugate of formula (II)

R1-L-R2 formula (II);

wherein the structure of L is as shown in formula (I); R1 and R2 are as defined in the first aspect.
In another embodiment, R1 is an activating group in the tumor microenvironment, or a modifying group that improves the water solubility of tumor-targeting drugs. and/or, R2 is a drug having cytotoxicity or a drug carrying cytotoxicity, which is selected from: Doxorubicin, Daunorubicin, Epirubicin, Taxanes, Taxol, Docetaxel, topoisomerase inhibitors, Camptothecins, Irinotecan, Exatecan and its derivative DXd, TLR7/8 agonist, Resiquimod, Imiquimod, Loxoribine, 1-(4-aminobutyl)-2-butyl-1H-imidazo[4,5-C]quinolin-4-amine (T785) or analogs thereof, Cisplatin, Carboplatin, Rapamycin, Everolimus, Methotrexate, Fludarabine, Gemcitabine, Cytarabine, Melphalan, Nimustine, Mitomycin, Mitoxantrone, dolastatin 10 derivatives, MMAE, MMAF, and MMAD.
Preferably, R2 contains D1 or -Am-D2.
Preferably, when Drug (D) of R2 is a drug with $-NH_2$ or -NH- at the free end, and R2 is D1; the structure of the tumor-targeting drug conjugate is as shown in Table B1.
Preferably, when Drug (D) in R2 is a drug with -OH at the free end, $R_2$ is -AM-D2; wherein when the structure of -AM- is

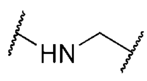,

the structure of the tumor-targeting drug conjugate is as shown in Table B2.
Preferably, wherein R1 is selected from the structures shown in Table C1.
Preferably, R1 is selected from a structure of

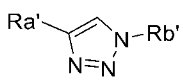

formed by a click reaction between

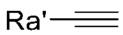 (Ra)

and Rb'-$N_3$ (Rb).
Preferably, when Ra is selected from the structures shown in Table D1, Rb is selected from compounds containing the groups described in Table D2.
Preferably, the drug conjugate is selected from the structures shown in Table A2 or Table A3. Preferably, the drug conjugate is selected from the structures shown in Table A1.

**Antibody Drug conjugate**

In one embodiment of present invention, further provided is an antibody conjugate (or anotibody drug conjugate) using the linker of formula (I) and the stereoisomer thereof or the pharmaceutically acceptable salt thereof.

Preferably, the antibody drug conjugate is as shown in formula (III)

R3-(R1'-L-R2)x Formula (III)

R3 refers to an antibody moiety, R1'-L-R2 refers to a group formed by the conjugation of the conjugate of formula (II) with the antibody element through the R1 group; wherein, R1, L, and R2 are as defined in claim 1; R1' is a group formed by the conjugation of R1 with the antibody element; and x is 1 to 8.

Preferably, the antibody drug conjugate is as shown in formula (IV)

R4-cys-S-(R1'-L-R2)x formula (IV);

In another embodiment, (R1'-L-R2) is any structure described in the first aspect or the above-mentioned drug conjugate;

R3 is an antibody having one or more cysteine residue mutations; cys is a cysteine residue contained in R3; S is an S atom in cysteine;

x is the number of (R1-L-R2) connected to the antibody, x=1-8.

Preferably, the antibody is selected from (but not limited to): HER2, CD19, CD20, EGFR, CD22, CD3, TROP2, Glycoprotein NMB, Guanylyl cyclase C, CEA, AXL, GCC, CD79b, PSMA, ENPP3, Mesothelin, CD138, NaPi2b, CD56, CD74, FOLR1, DLL3, CEACAM5, CD142, SLAMF7, CD25, SLTRK6, CD37, CD70, AGS-22, C4.4A, FGFR2, Ly6E, MUC16, BCMA, pCadherin, Ephrin-A, LAMP1, MUC1, PDL1, HER2, NY-ESO-1, BCMA, WT1, MUC1, CD20, CD23, ROR1, CD123, CD33, CD44v6, CD174, CD30, CD133, cMet, FAP, EphA2, GD2, GPC3, IL-13Ra2, LewisY, SS1, CD171, EGFR, EGFRvIII, VEGFR2, NY-ESO-1, MUC-1, or MAGE-A3, alternatively, can be selected from the antigen-binding domain of any antibody mentioned in the present invention.

**PHARMACEUTICAL COMPOSITIONS AND ADMINISTRATION METHOD**

Since the linker used in the drug conjugate (conjugate) or antibody drug conjugate (antibody conjugate) provided by the present invention can remain stable outside the tumor environment and be efficiently cleaved by cathepsin L after entering the tumor environment, thereby releasing a specific drug (such as a cytotoxic drug) in the tumor site or other target sites. Therefore, the drug conjugate or the antibody drug conjugate of the present invention can be used for treating tumors or inflammation (such as bladder cancer, brain cancer, breast cancer, cervical cancer, colorectal cancer, esophageal cancer, kidney cancer, liver cancer, lung cancer, nasopharyngeal cancer, pancreatic cancer, prostate cancer, skin cancer, stomach cancer, uterine cancer, ovarian cancer, testicular cancer, blood cancer and the like).

The above drug conjugate or antibody drug conjugate can be administered to a subject (e.g., human) in a therapeutically effective amount via an appropriate route. A subject in need of treatment can be a patient at risk of, or suspected of having a disorder associated with the activity or expression of a particular antigen. Such patients can be identified through routine physical examination.

Conventional methods, known to those of ordinary skill in the medical art, can be used to administer the pharmaceutical composition to a subject, depending on the type of the disease to be treated or the site of the disease. The compositions may also be administered through other conventional routes, for example, orally, parenterally, by inhalation spray, topically, rectally, nasally, buccally, vaginally or by implant. The term "parenteral" as used herein includes subcutaneous, intracutaneous, intravenous, intramuscular, intraarticular, intraarterial, intrasynovial, intrasternal, intrathecal, intralesional and intracranial injection or infusion techniques. Additionally, it can be administered to a subject via an injectable depot route, such as using 1-, 3-, or 6-month depot injectable or biodegradable materials and methods.

Injectable compositions may contain various carriers such as vegetable oils, dimethylactamide, dimethylformamide, ethyl lactate, ethyl carbonate, isopropyl myristate, ethanol, polyols (glycerol, propylene glycol, liquid polyethylene glycol, etc.). For intravenous administration, water-soluble antibodies can be administered by intravenous drip, whereby a pharmaceutical formulation containing the antibody and a physiologically acceptable excipient is infused. Physiologically acceptable excipients may include, for example, 5% dextrose, 0.9% saline, Ringer's solution, or other suitable excipients. Intramuscular formulations, for example, a sterile formulation of a suitable soluble salt of the antibody, can be dissolved and administered in a pharmaceutically acceptable excipient such as water-for-injection solution, 0.9% saline, or 5% dextrose solution.

When using the drug conjugate or antibody-drug conjugate of the present invention for treatment, delivery can be

performed by methods conventional in the art. For example, it can be introduced into cells using liposomes, hydrogels, cyclodextrins, biodegradable nanocapsules, or bioadhesive microspheres. Alternatively, the nucleic acid or vector can be delivered locally by direct injection or by using an infusion pump. Other methods include various delivery and carrier systems through the use of conjugates and biodegradable polymers.

The pharmaceutical composition of the present invention contains drug conjugates or antibody drug conjugates of the present invention in a safe and effective dosage range and pharmaceutically acceptable carriers. Such carriers include, but are not limited to, saline, buffer, dextrose, water, glycerol, ethanol, and combinations thereof. Generally, pharmaceutical preparations should be compatible with the mode of administration. The pharmaceutical composition of the present invention can be prepared in the form of a solution, for example, prepared by using physiological saline or an aqueous solution containing glucose and other adjuvants by conventional methods. The pharmaceutical composition is preferably manufactured under sterile conditions. The amount of active ingredient administered is a therapeutically effective amount.

The effective amount of the drug conjugate or antibody-drug conjugate described in the present invention may vary depending on the mode of administration and the severity of the disease to be treated. The selection of the preferred effective amount can be determined by those skilled in the art based on various factors (e.g., through clinical trials). The factors include, but are not limited to: pharmacokinetic parameters of the antibody conjugate such as bioavailability, metabolism, half-life, etc.; severity of the disease to be treated in the patient, weight of the patient, immune status of the patient, route of administration, etc. Generally, satisfactory effects can be obtained when the antibody-drug conjugate of the present invention is administered at a dose of about 0.0001 mg-50 mg/kg animal body weight (preferably 0.001 mg-10 mg/kg animal body weight) per day. For example, several divided doses may be administered daily or the dose may be proportionally reduced as indicated by the exigencies of the therapeutic situation.

The dosage forms of the compounds of the present invention used for local administration include ointments, powders, patches, sprays, and inhalants. The active ingredients are mixed under sterile conditions with a physiologically acceptable carriers and any preservatives, buffers, or propellants that may be necessary.

The compound of the present invention can be administered separately or in combination with other pharmaceutically acceptable therapeutic agents.

When the pharmaceutical composition is used, a safe and effective amount of the antibody -drug conjugate of the present invention is administered to a mammal (such as a human) in need of treatment, wherein the dosage at the time of administration is the pharmaceutically effective dosage, for people having a body weight of 60 kg, the daily dosage is usually 1~2000mg, preferably 5~ 500mg. Of course, the specific dosage should also consider factors such as the route of administration and the patient's health status, which are within the skill range of a skilled physician.

**The main advantages of the present invention include:**

(1) The linker for drug conjugation, the tumor-targeting drug conjugate, the antibody drug conjugate, the pharmaceutical composition and uses thereof of the present invention use a selectively targeted cathepsin-cleavable linker, GGFL can be effectively recognized and cleaved by cathepsin L. After the drug targets and enters the tumor cells, it can be cleaved by the cathepsin in the tumor cells to release drug molecules having therapeutic effects.
(2) Conjugates or antibody conjugates having the linker (-GGFL-) of the present invention exhibit excellent stability in non-tumor environments such as plasma, heart homogenate, and liver homogenate, which is significantly higher than conjugates using other linkers (-GGFG-) (see Examples 8 to 10).
(3) The conjugate or antibody conjugate having the linker (-GGFL-) of the present invention can be efficiently activated and release specific drugs in the tumor environment (see Examples 11 and 12).
(4) The conjugate or antibody conjugate having the linker (-GGFL-) of the present invention has a better cure rate than the linker (-GGFG-) in the prior art.
(5) The conjugate or antibody conjugate of the present invention exhibits excellent therapeutic effects *in vivo* (see Examples 13 and 14).

The present invention was further described hereafter in combination with specific embodiments. It should be understood that these examples are only used to illustrate the invention and not to limit the scope of the invention. Experimental methods in which the specific conditions are not specified in the following examples are usually in accordance with conventional conditions such as the conditions described in Sambrook et al., Molecular Cloning: Laboratory Manual (New York: Cold Spring Harbor Laboratory Press, 1989), or in accordance with the conditions recommended by the manufacturer. Unless otherwise stated, percentages and parts are percentages by weight and parts by weight.

**Example 1 A linker for drug conjugation**

The linker provided by the present invention has the following structure:

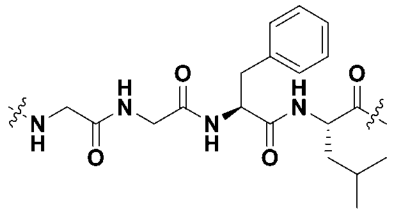

Formula (I).

The linker of formula (I) can be used in drug-drug conjugates, or used in antibody-drug conjugates, or used in pharmaceutical compositions comprising the drug-drug conjugates or antibody-drug conjugates.

Example 2 tumor-targeting drug conjugate

The drug has the following structure of formula (II):

$R_1$-L-$R_2$ Formula (II);

wherein the structure of L is

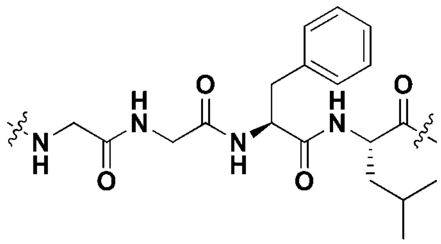

formula (I);

wherein $R_1$ is an activating group in the tumor microenvironment, or a modifying group that improves the water solubility of tumor-targeting drugs.

$R_2$ is a drug having cytotoxicity or a drug carrying cytotoxicity.

As a preferred embodiment of the present invention, $R_2$ contains D1 or -Am-D2.

Preferably, when Drug (D) is a drug with $-NH_2$ or -NHR' at the free end, wherein R' is C1-6 alkyl (such as methyl), and $R_2$ is D1; and D1 is a structure selected from the following Table E1:

Table E1

| | | | |
|---|---|---|---|
| D1.1 | 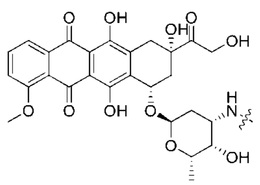 doxorubicin | D1.2 | 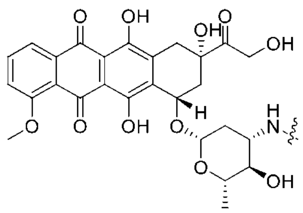 Epirubicin |
| D1.3 | 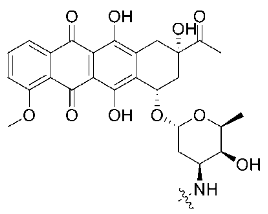 Daunorubicin | D1.4 | 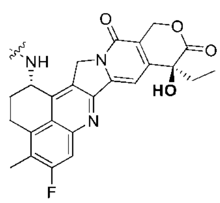 Exatecan |

(continued)

| | | | |
|---|---|---|---|
| D1.5 | 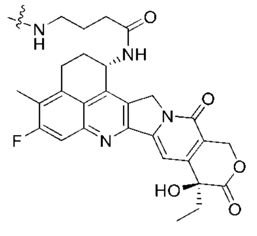<br>Exatecan derivative DXd-$NH_2$ | D1.6 | 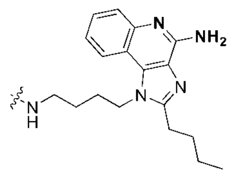<br>T785 |
| D1.7 | 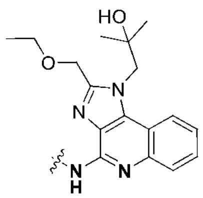<br>Resiquimod | D1.8 | 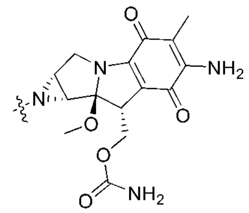<br>Mitomycin (NH) |
| D1.9 | 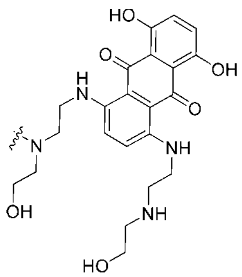<br>Mitoxantrone | D1.10 | 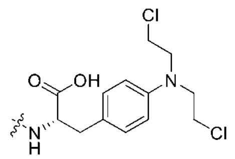<br>Melphalan |
| D1.11 | 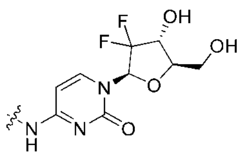<br>Gemcitabine | D1.12 | 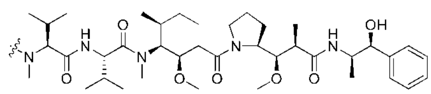<br>MMAE |
| D1.13 | 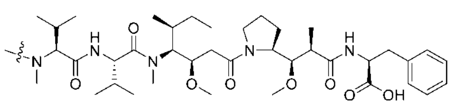<br>MMAF | D1.14 | 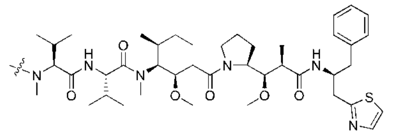<br>MMAD |

Preferably, when D is a drug with -OH at the free end, $R_2$ is -Am-D2; wherein, the structure of -Am- is:

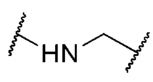;

and D2 is a structure selected from the following Table E2:

Table E2

| | | | |
|---|---|---|---|
| D2.1 | 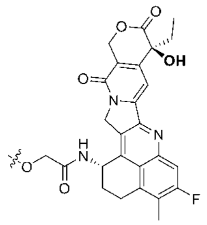<br>Exatecan derivative DXd-OH | D2.2 | 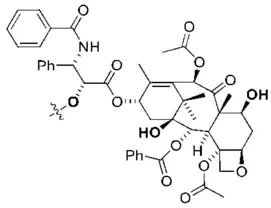<br>taxol |
| D2.3 | 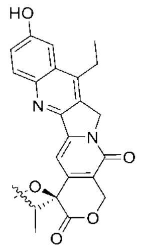<br>SN-38 (20) | D2.4 | 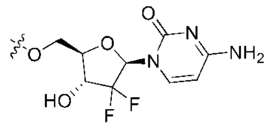<br>Gemcitabine |
| D2.5 | 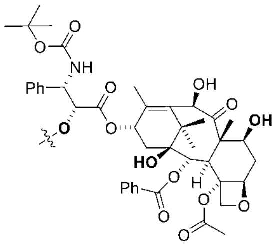<br>Docetaxel | D2.6 | 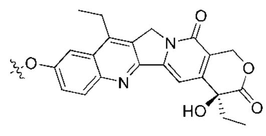<br>SN-38 (10) |
| D2.7 | 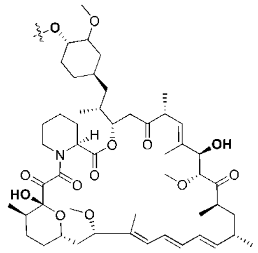<br>Rapamycin | D2.8 | 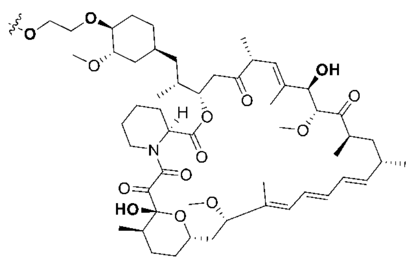<br>Everolimus, |

As a preferred embodiment of the present invention, wherein R1 is selected from the structures shown in Table C1;

or, R1 is selected from a structure of

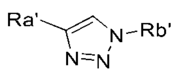

formed by a click reaction between

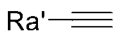 (Ra)

and Rb'-$N_3$ (Rb);
wherein, when Ra is selected from the structures shown in Table D1 (see above), Rb is selected from the compounds containing a group shown in Table D2 (see above).

In a specific embodiment, R1 is selected from Table B2 (see above).

In a preferred embodiment, the drug is selected from the following Table A1:

Table A1

| | |
|---|---|
| S1 | 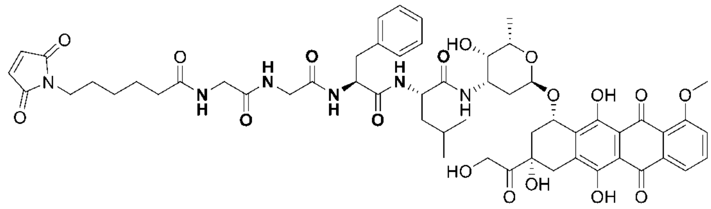 |
| S2 | 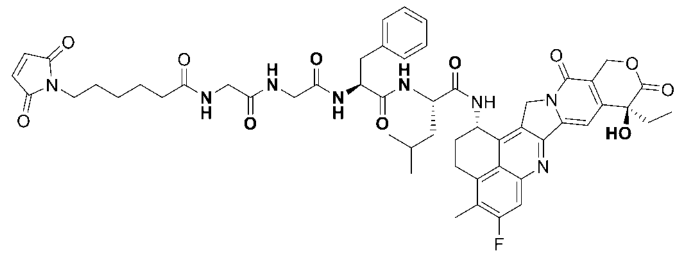 |
| S3 | 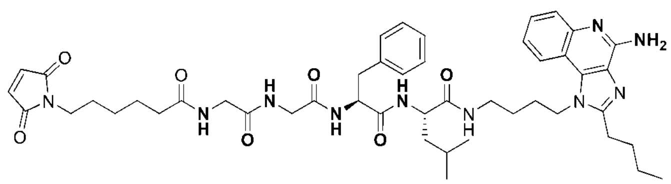 |
| S4 | 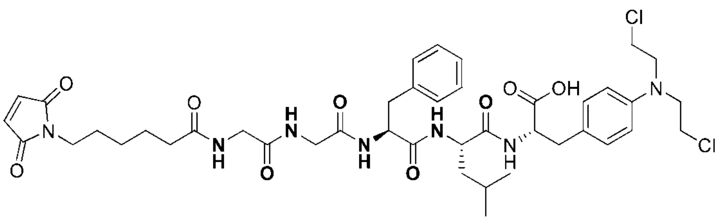 |
| S5 | 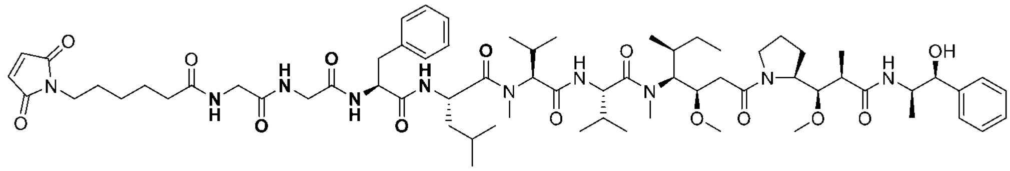 |
| S6 | 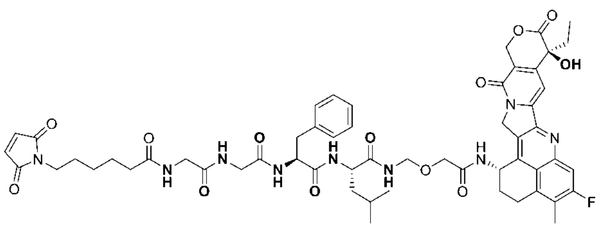 |
| S7 | 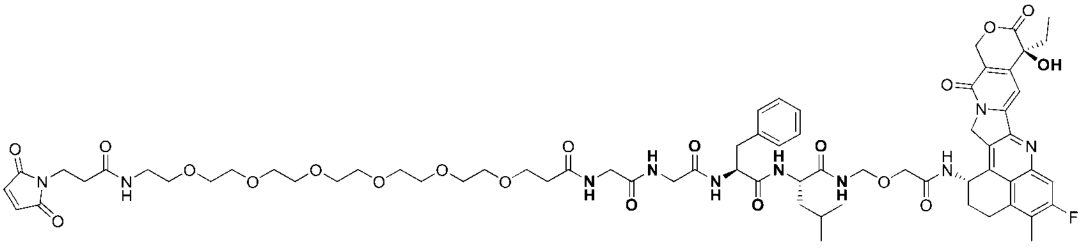 |

(continued)

| | |
|---|---|
| S8 | 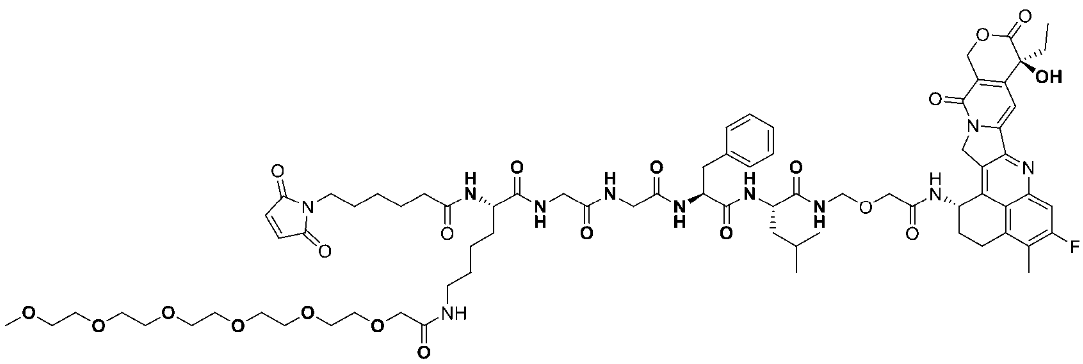 |
| S9 | 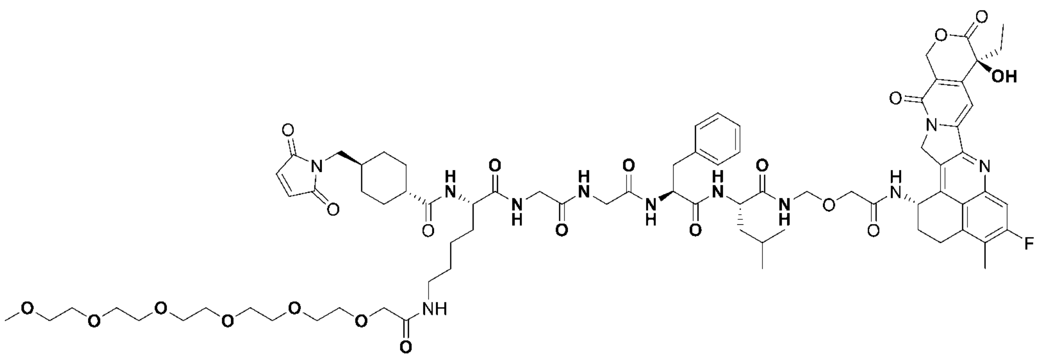 |
| S10 | 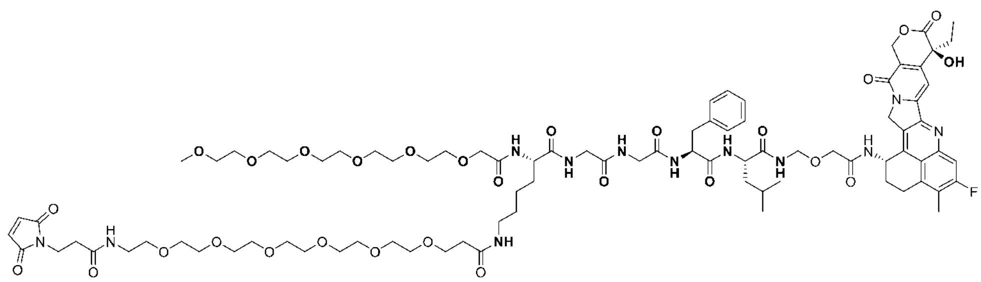 |
| S11 | 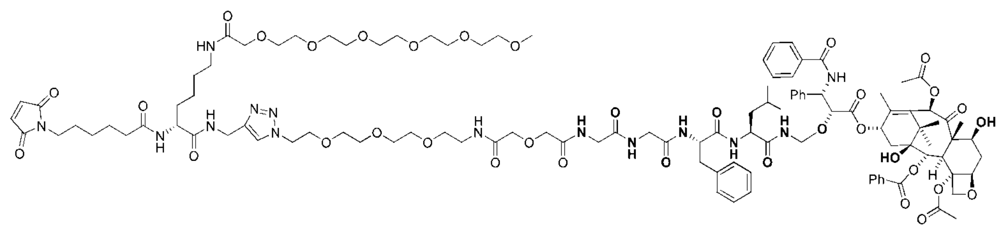 |
| S12 | 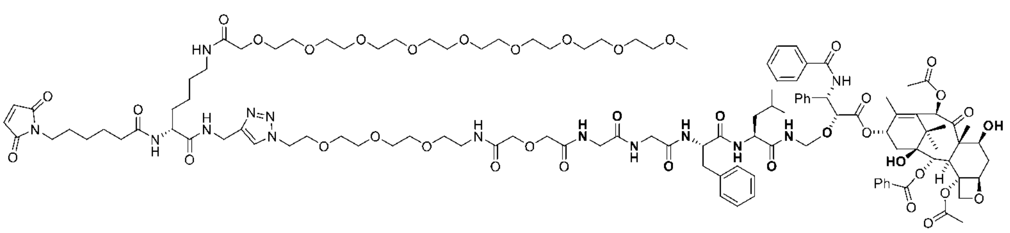 |

(continued)

| | |
|---|---|
| S13 | 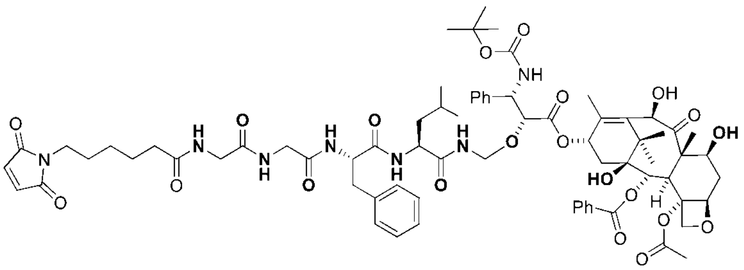 |
| S14 | 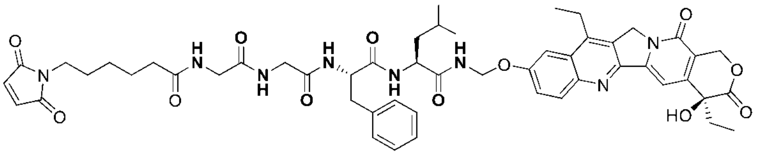 |
| S15 | 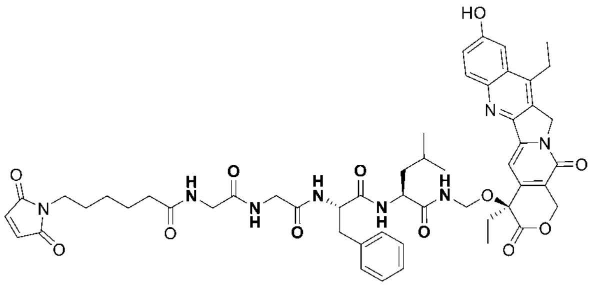 |
| S16 | 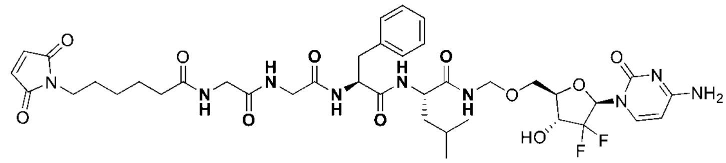 |
| S17 | 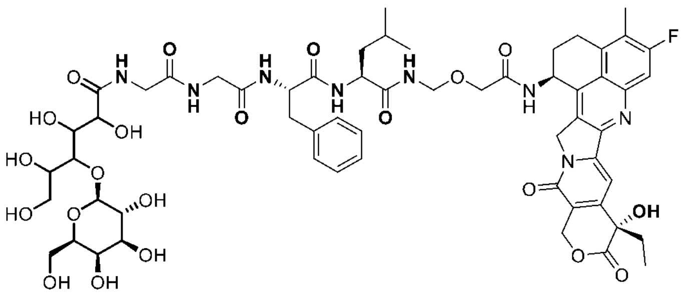 |
| S18 | 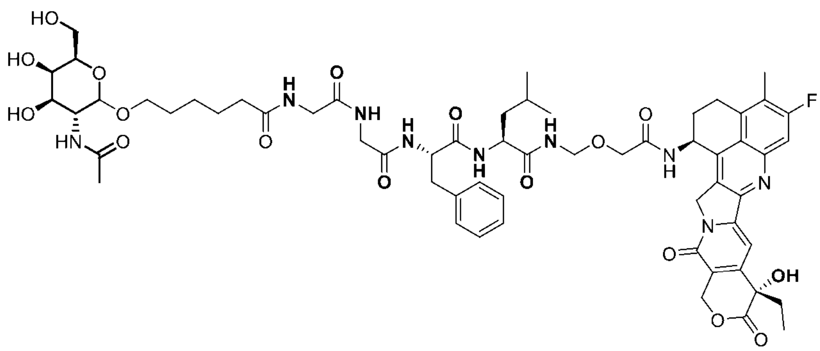 |

(continued)

| | |
|---|---|
| S19 | 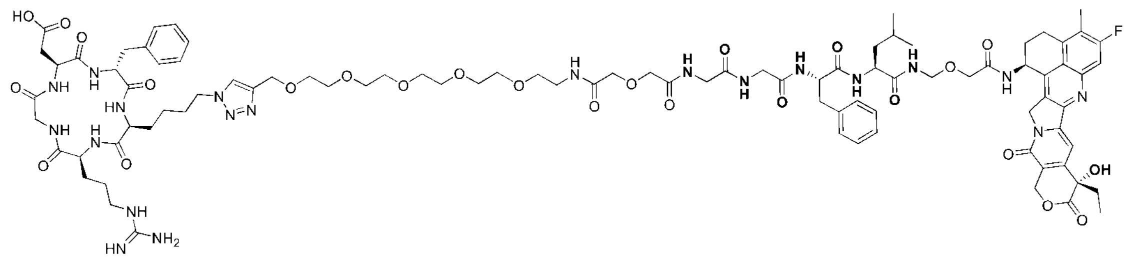 |
| S20 | 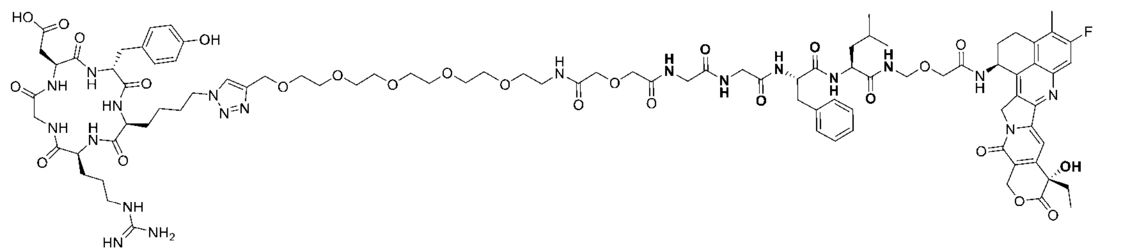 |
| S21 | 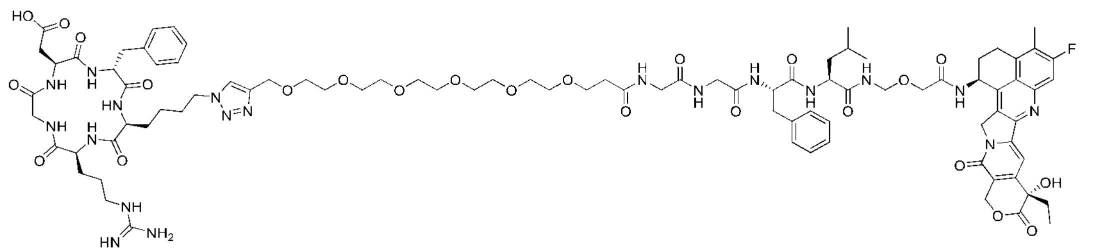 |
| S22 | 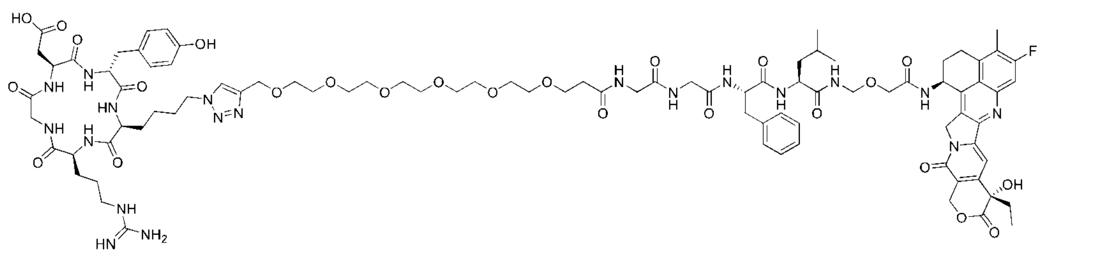 |
| S23 | 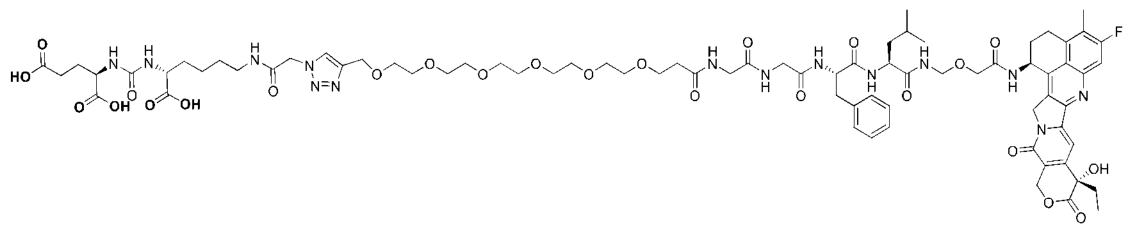 |
| S24 | 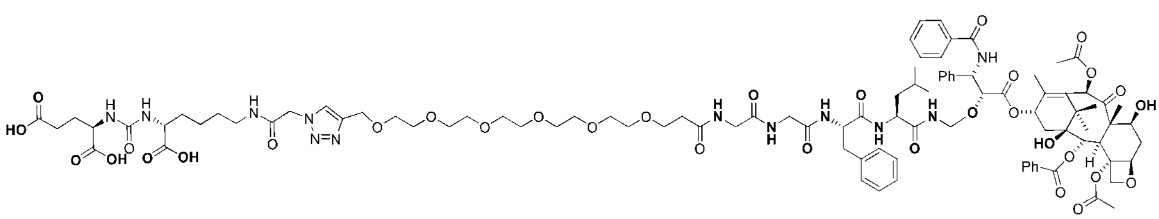 |

(continued)

| | |
|---|---|
| S25 | 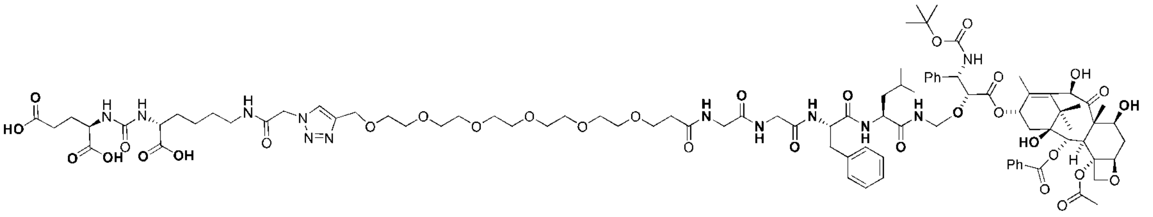 |
| S26 | 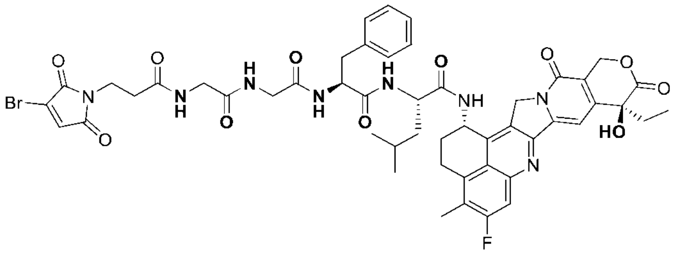 |
| S27 | 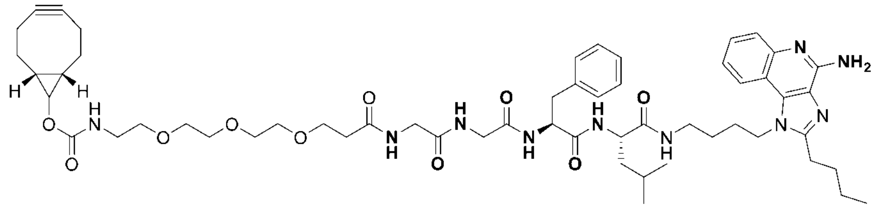 |
| S28 | 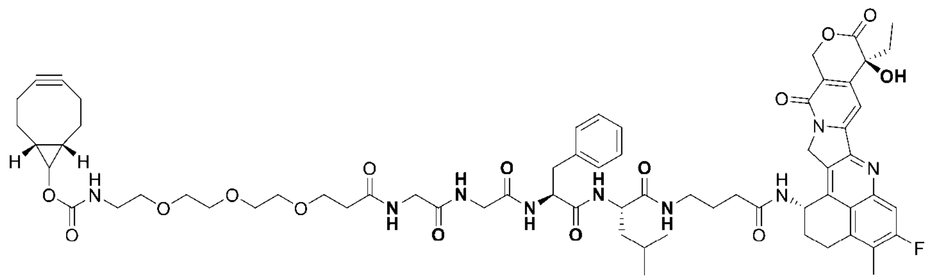 |
| S29 | 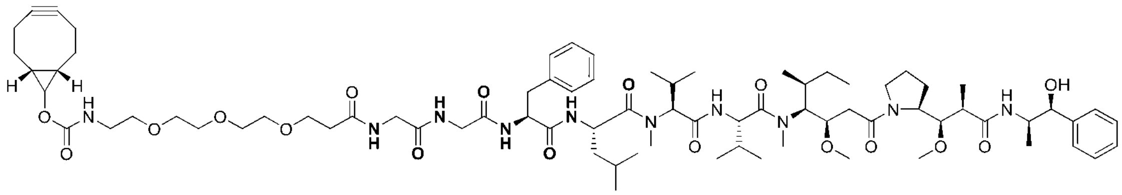 |
| S30 | 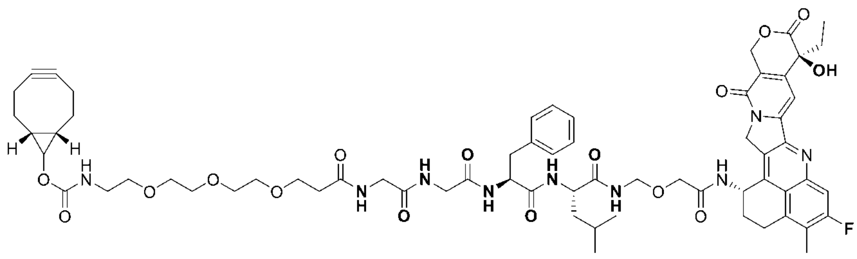 |
| S31 | 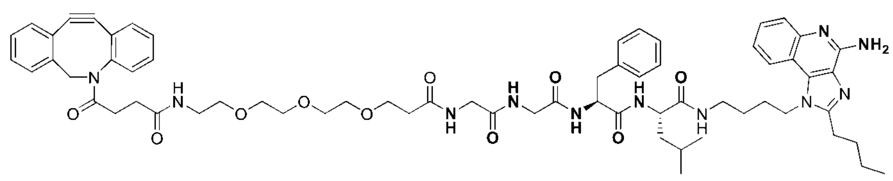 |

(continued)

| | |
|---|---|
| S32 | 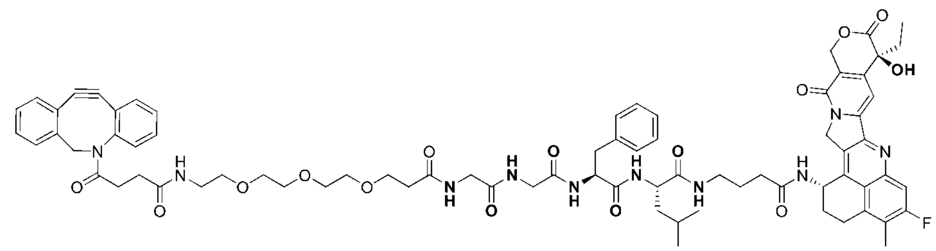 |
| S33 | 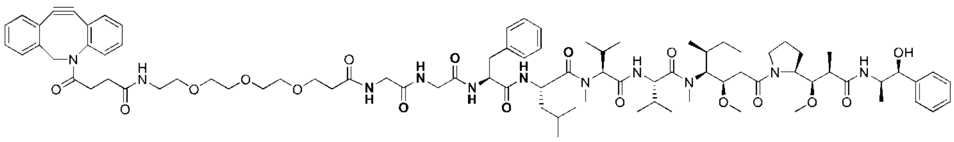 |
| S34 | 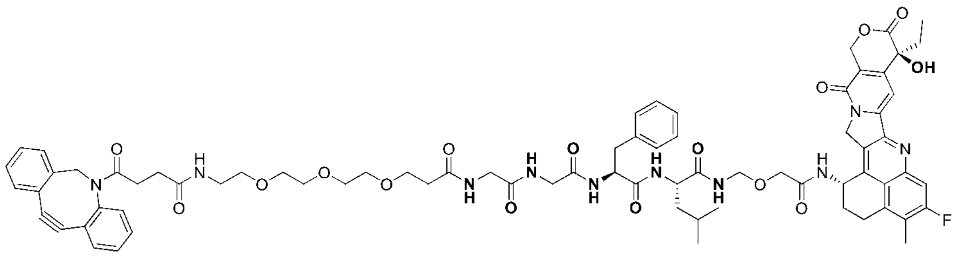 |

**Example 3 Preparation method of tumor-targeting drugs**

**1. Synthesis of Compound R1-Gly-Gly-Phe-Leu-N(H)-D**

The typical preparation method is shown in Synthesis Scheme 1: Synthesis Scheme 1:

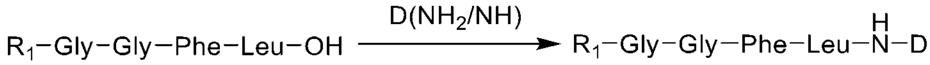


Synthesis Method:

Compound R1-Gly-Gly-Phe-Leu-OH (1.0 eq) was dissolved in an appropriate amount of DMF solvent, and then D ($NH_2$/NH) (i.e., a drug with a -$NH_2$ or -NH- group, 1.0 eq) and condensing agent HBTU (1.2 eq) were added in sequence. DIPEA (2.0 eq) was added to the reaction solution under ice bath, and then the reaction solution was slowly warmed to room temperature and continued to be stirred for 3-5 h. The obtained reaction solution was subjected to high vacuum to remove most of the solvent, then diluted with an appropriate amount of methanol, purified by reverse phase high pressure preparative chromatography, and finally lyophilized to obtain the target product. The compounds shown in the following Table A2 can be obtained according to the above method:

Table A2

| R1-Gly-Gly-Phe-Leu-D1 | | | | | | | |
|---|---|---|---|---|---|---|---|
| Compoun d No. | R1 | D ($NH_2$/NH) | D1 | Characters | Yield | Molecular weight | Mass spectrometr y detection M+H |
| 1.1 | $R_{1-1}$ | doxorubicin | D1.1 | white powder | 20 mg | 1111.17 | 1111.34 |
| 1.2 | $R_{1-1}$ | Epirubicin | D1.2 | white powder | 15 mg | 1111.17 | 1111.44 |
| 1.3 | $R_{1-1}$ | Daunorubicin | D1.3 | white powder | 18 mg | 1095.17 | 1095.35 |
| 1.4 | $R_{1-1}$ | Exatecan | D1.4 | Light yellow powder | 22 mg | 1003.10 | 1003.25 |

(continued)

| R1-Gly-Gly-Phe-Leu-D1 | | | | | | | |
|---|---|---|---|---|---|---|---|
| Compoun d No. | R1 | D ($NH_2$/NH) | D1 | Characters | Yield | Molecular weight | Mass spectrometr y detection M+H |
| 1.5 | $R_{1-1}$ | Exatecan derivative (DXd-$NH_2$) | D1.5 | Light yellow powder | 20 mg | 1088.20 | 1088.50 |
| 1.6 | $R_{1-1}$ | T785 | D1.6 | white powder | 10 mg | 879.08 | 879.28 |
| 1.7 | $R_{1-1}$ | Resiquimod | D1.7 | white powder | 8 mg | 882.03 | 882.34 |
| 1.8. | $R_{1-1}$ | Mitomycin | D1.8 | purple powder | 12 mg | 901.98 | 902.00 |
| 1.9 | $R_{1-1}$ | Mitoxantrone | D1.9 | Light yellow powder | 15 mg | 1012.13 | 1012.27 |
| 1.10 | $R_{1-1}$ | Melphalan | D1.10 | white powder | 20 mg | 872.84 | 872.54 |
| 1.11 | $R_{1-1}$ | Gemcitabine | D1.11 | white powder | 13 mg | 830.84 | 830.54 |
| 1.12 | $R_{1-1}$ | MMAE | D1.12 | white powder | 24 mg | 1285.64 | 1285.74 |
| 1.13 | $R_{1-1}$ | MMAF | D1.13 | white powder | 11 mg | 1299.62 | 1299.65 |
| 1.14 | $R_{1-1}$ | MMAD | D1.14 | white powder | 10 mg | 1352.75 | 1352.78 |
| 1.15 | $R_{1-30}$ (n=2) | MMAE | D1.12 | white powder | 12 mg | 1457.86 | 1457.88 |
| 1.16 | $R_{1-39}$ (n=2) | MMAE | D1.12 | white powder | 10 mg | 1582.99 | 1583.10 |
| 1.17 | $R_{1-2}$ (n=5) | Exatecan | D1.4 | Light yellow solid | 10 mg | 1296.41 | 1296.48 |
| 1.18 | $R_{1-3}$ (m=5, n=5) | Exatecan | D1.4 | Light yellow solid | 8 mg | 1716.92 | 1716.88 |
| 1.19 | $R_{1-4}$ (m=5, n=5) | Exatecan | D1.4 | Light yellow solid | 10 mg | 1716.92 | 1716.89 |
| 1.20 | $R_{1-5}$ (m=5) | Exatecan | D1.4 | Light yellow solid | 15 mg | 1423.60 | 1423.58 |
| 1.21 | $R_{1-6}$ (m=5) | Exatecan | D1.4 | Light yellow solid | 10 mg | 1449.64 | 1449.69 |
| 1.22 | $R_{1-14}$ (m=5) | Exatecan | D1.4 | Light yellow solid | 13 mg | 1587.77 | 1587.76 |
| 1.23 | $R_{1-15}$ (m=5) | Exatecan | D1.4 | Light yellow solid | 10 mg | 1561.73 | 1561.73 |
| 1.24 | $R_{1-16}$ (m=5, n=2) | Exatecan | D1.4 | Light yellow solid | 8 mg | 1795.00 | 1794.86 |
| 1.25 | $R_{1-26}$ (n=0) | Exatecan | D1.4 | Light yellow solid | 13 mg | 1039.91 | 1039.88 |
| 1.26 | $R_{1-30}$ (n=2) | Exatecan derivative DXd-NH2 | D1.5 | Light yellow solid | 7 mg | 1260.43 | 1260.59 |
| 1.27 | $R_{1-39}$ (n=2) | Exatecan derivative DXd-NH2 | D1.5 | Light yellow solid | 11 mg | 1385.56 | 1385.62 |

(continued)

| R1-Gly-Gly-Phe-Leu-D1 | | | | | | | |
|---|---|---|---|---|---|---|---|
| Compoun d No. | R1 | D ($NH_2$/NH) | D1 | Characters | Yield | Molecular weight | Mass spectrometr y detection M+H |
| 1.28 | $R_{1-2}$ | T785 | D1.6 | white powder | 10 mg | 1172.39 | 1172.63 |
| 1.29 | $R_{1-3}$ (m=5, n=5) | T785 | D1.6 | white powder | 12 mg | 1592.90 | 1592.88 |
| 1.30 | $R_{1-4}$ (m=5, n=5) | T785 | D1.6 | white powder | 25 mg | 1592.90 | 1592.78 |
| 1.31 | $R_{1-5}$ (m=5) | T785 | D1.6 | white powder | 20 mg | 1299.58 | 1299.74 |
| 1.32 | $R_{1-6}$ (m=5) | T785 | D1.6 | white powder | 30 mg | 1325.62 | 1325.77 |
| 1.33 | $R_{1-14}$ (m=5) | T785 | D1.6 | white powder | 21 mg | 1463.75 | 1463.80 |
| 1.34 | $R_{1-15}$ (m=5) | T785 | D1.6 | white powder | 15 mg | 1437.71 | 1437.79 |
| 1.35 | $R_{1-16}$ (m=5, n=3) | T785 | D1.6 | white powder | 20 mg | 1670.97 | 1671.93 |
| 1.36 | $R_{1-30}$ (n=2) | T785 | D1.6 | white powder | 10 mg | 1051.30 | 1051.59 |
| 1.37 | $R_{1-39}$ (n=2) | T785 | D1.6 | white powder | 8 mg | 1176.43 | 1176.62 |

**2. Synthesis of Compound R1-Gly-Gly-Phe-Leu-AM-D**

The synthetic route is shown in Synthesis Scheme 2

Synthesis Scheme 2:

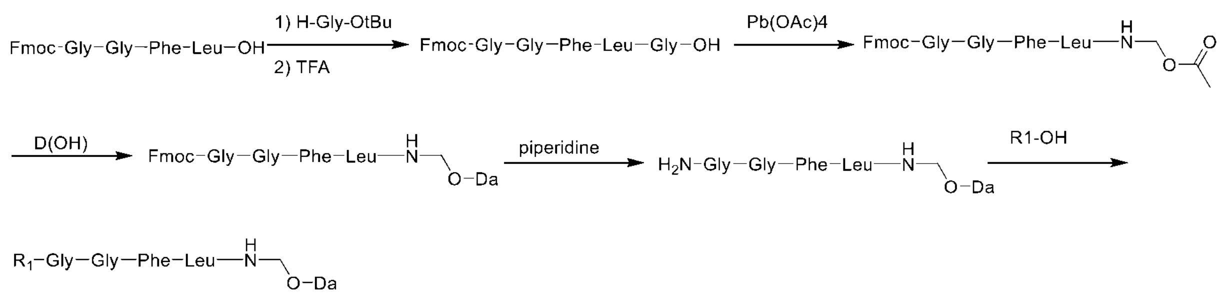


Synthesis of Compound Fmoc-Gly-Gly-Phe-Leu-Gly-OH

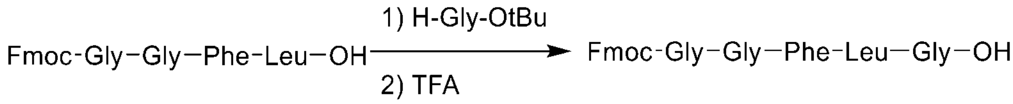


Fmoc-Gly-Gly-Phe-Leu-Gly-OH (1.0 eq) was dissolved in DMF, and cooled to 0 °C. H-Gly-OtBu (1.0 eq), HBTU (1.0 eq) and DIPEA (2.0 eq) were added under nitrogen protection, and the mixture was stirred for 2 to 3 hours at room temperature. TLC detection showed that the reaction of raw materials was completed. The reaction solution was diluted with dichloromethane, washed with water, and subjected to liquid seperation successively, the aqueous phase was extracted with dichloromethane, the organic phases were combined and dried over anhydrous sodium sulfate, spin-dried, and subjected to column chromatography to obtain Fmoc-Gly-Gly-Phe-Leu-Gly-OtBu.

The above crude product was dissolved in DCM, TFA/DCM (20%) was added, and the mixture was stirred at room temperature for 2-3 hours. TLC detection showed that the reaction of raw materials was completed. The reaction solution was concentrated and separated by high pressure preparative chromatography to obtain Fmoc-Gly-Gly-Phe-Leu-Gly-OH.

1) Synthesis of Compound Fmoc-Gly-Gly-Phe-Leu-AM-OAc (wherein -AM- is $-NH-CH_2-$)

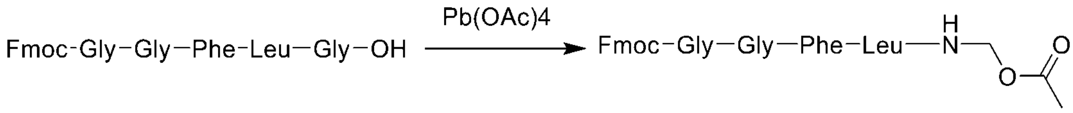


Fmoc-Gly-Gly-Phe-Leu-Gly-OH(1.0 eq) was dissolved in dichloromethane/tetrahydrofuran (15/5 mL), and cooled to 0 °C. Lead acetate (1.2 eq) and copper acetate (0.2 eq) were added under nitrogen protection, and the resulting mixture was stirred at 40-50 °C under nitrogen protection for 2-3 hours. TLC detection showed that the reaction of raw materials was completed. The reaction solution was diluted with dichloromethane, washed with water, and subjected to liquid seperation successively, the aqueous phase was extracted with dichloromethane, the organic phases were combined and dried over anhydrous sodium sulfate, spin-dried, and subjected to column chromatography to obtain Fmoc-Gly-Gly-Phe-Leu-AM-OAc.

2) Synthesis of Compound Fmoc-Gly-Gly-Phe-Leu-AM-O-Da (wherein -AM-O-Da is -AM-D2.)

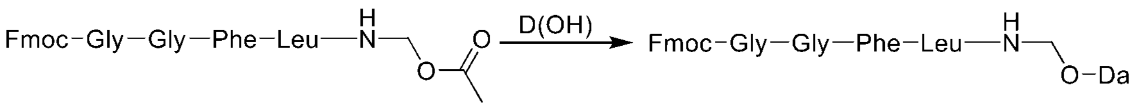


To a three-necked flask was added Compound Fmoc-Gly-Gly-Phe-Leu-AM-OAc (1.0 eq), and compound D(OH) (i.e., a drug with -OH group, 2.0 eq). TFA (0.3 eq) was added under nitrogen protection and ice bath, and the resulting mixture was stirred at room temperature for 2-3 h. After completion of the reaction, the reaction solution was concentrated and separated by high pressure preparative chromatography to obtain Fmoc-Gly-Gly-Phe-Leu-AM-O-Da as a white solid.

3) Synthesis of Compound H-Gly-Gly-Phe-Leu-AM-O-Da

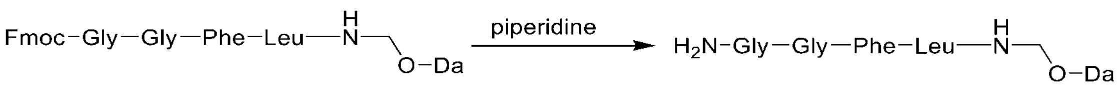


Compound Fmoc-Gly-Gly-Phe-Leu-AM-O-Da(1.0 eq) was dissolved in DMF. Piperidine (2.0 eq) was added, and the mixture was stirred at room temperature for 2-3 h under nitrogen protection. TLC detection showed that the reaction of raw materials was completed. The reaction solution was redissolved, and the solution was filtered, and then subjected to high pressure liquid chromatography to obtain the compound H-Gly-Gly-Phe-Leu-AM-O-Da as a solid product.

4) Synthesis of Compound R1-Gly-Gly-Phe-Leu-AM-O-Da

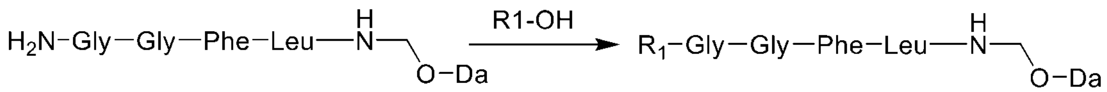


Compound R1-OH (1.0 eq) was dissolved in DMF (9/1 10 mL), and added with H-Gly-Gly-Phe-Leu-AM-D2(1.0 eq). Under nitrogen protection, HBTU (1.2 eq) and DIPEA (2.0 eq) were added and the mixture was stirred at room temperature for 1-2 h. TLC detection showed that the reaction of raw materials was completed. The crude reaction solution was filtered, and then subjected to high pressure liquid chromatography to obtain the compound R1-Gly-Gly-Phe-Leu-AM-D2 (as shown in Table A3) as a solid product.

Table A3

| R1-Gly-Gly-Phe-Leu-NH-$CH_2$-D2 | | | | | | | |
|---|---|---|---|---|---|---|---|
| Compound No. | $R_1$ | D(OH) | D2 | Characters | Yield | Molecular weight | Mass spectrometry detection M+H |
| 2.1 | $R_{1-1}$ | Exatecan derivative DXd-OH | D2.1 | Light yellow powder | 12 mg | 1090.18 | 1090.48 |
| 2.2 | $R_{1-2}$ (n=5) | Exatecan derivative DXd-OH | D2.1 | Light yellow powder | 8 mg | 1383.49 | 1383.63 |
| 2.3 | $R_{1-3}$ (m=5, n=5) | Exatecan derivative DXd-OH | D2.1 | Light yellow powder | 20 mg | 1804.00 | 1804.02 |
| 2.4 | $R_{1-4}$ (m=5, n=5) | Exatecan derivative DXd-OH | D2.1 | Light yellow powder | 13 mg | 1804.00 | 1804.04 |
| 2.5 | $R_{1-5}$ (m=5) | Exatecan derivative DXd-OH | D2.1 | Light yellow powder | 10 mg | 1510.68 | 1510.71 |
| 2.6 | $R_{1-6}$ (m=5) | Exatecan derivative DXd-OH | D2.1 | Light yellow powder | 25 mg | 1536.72 | 1536.74 |
| 2.7 | $R_{1-14}$ (m=5) | Exatecan derivative DXd-OH | D2.1 | Light yellow powder | 30 mg | 1674.85 | 1674.89 |
| 2.8 | $R_{1-15}$ (m=5) | Exatecan derivative DXd-OH | D2.1 | Light yellow powder | 28 mg | 1648.81 | 1648.77 |
| 2.9 | $R_{1-16}$ (m=5, n=2) | Exatecan derivative DXd-OH | D2.1 | Light yellow powder | 25 mg | 1882.07 | 1882.10 |
| 2.10 | $R_{1-30}$ (n=2) | Exatecan derivative DXd-OH | D2.1 | Light yellow powder | 17 mg | 1262.40 | 1262.51 |
| 2.11 | $R_{1-39}$ (n=2) | Exatecan derivative DXd-OH | D2.1 | Light yellow powder | 21 mg | 1387.53 | 1387.60 |
| 2.12 | $R_{1-1}$ | taxol | D2.2 | white powder | 10 mg | 1450.60 | 1450.63 |
| 2.13 | $R_{1-2}$ (n=5) | taxol | D2.2 | white powder | 15 mg | 1743.92 | 1744.01 |
| 2.14 | $R_{1-3}$ (m=5, n=5) | taxol | D2.2 | white powder | 12 mg | 2164.42 | 2164.45 |

(continued)

| R1-Gly-Gly-Phe-Leu-NH-$CH_2$-D2 | | | | | | | |
|---|---|---|---|---|---|---|---|
| Compound No. | $R_1$ | D(OH) | D2 | Characters | Yield | Molecular weight | Mass spectrometry detection M+H |
| 2.15 | $R_{1-4}$ (m=5, n=5) | taxol | D2.2 | white powder | 14 mg | 2164.42 | 2164.44 |
| 2.16 | $R_{1-5}$ (m=5) | taxol | D2.2 | white powder | 10 mg | 1871.11 | 1871.21 |
| 2.17 | $R_{1-6}$ (m=5) | taxol | D2.2 | white powder | 8 mg | 1897.14 | 1897.24 |
| 2.18 | $R_{1-1}$ | Rapamycin | D2.7 | white powder | 12 mg | 1510.87 | 1511.12 |
| 2.19 | $R_{1-2}$ (n=5) | Rapamycin | D2.7 | white powder | 15 mg | 1804.19 | 1804.23 |
| 2.20 | $R_{1-3}$ (m=5, n=5) | Rapamycin | D2.7 | white powder | 8 mg | 2224.69 | 2224.78 |
| 2.21 | $R_{1-4}$ (m=5, n=5) | Rapamycin | D2.7 | white powder | 14 mg | 2224.69 | 2224.79 |
| 2.22 | $R_{1-5}$ (m=5) | Rapamycin | D2.7 | white powder | 10 mg | 1931.38 | 1931.45 |
| 2.23 | $R_{1-6}$ (m=5) | Rapamycin | D2.7 | white powder | 12 mg | 1957.41 | 1957.53 |

3) Synthesis of Compounds S1, S2, S3, S4, and S5

3.1) Synthesis of Compound S1:

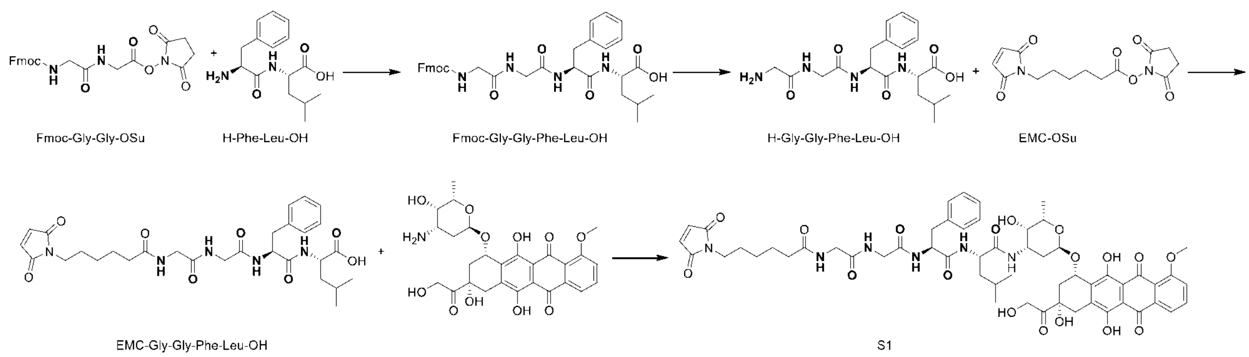


1) Synthesis of Compound Fmoc-Gly-Gly-Phe-Leu-OH

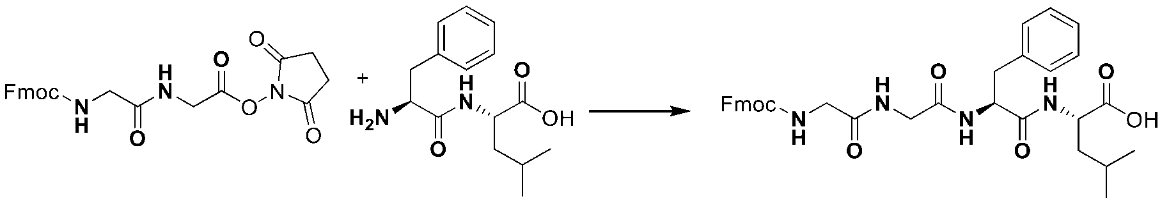

Fmoc-Gly-Gly-OSu H-Phe-Leu-OH Fmoc-Gly-Gly-Phe-Leu-OH

H-Phe-Leu-OH (1.0 eq), and $NaHCO_3$ (5.0 eq) were added to a three-necked flask, and suspended in $THF/H_2O(4/1)$. After stirring under nitrogen protection in an ice bath for 30 min, compound Fmoc-Gly-Gly-OSu (1.0 eq) was added in batches, and the resulting mixture was stirred at room temperature for 5-6 h. The reaction solution was acidified by adding dilute hydrochloric acid, and the obtained crude product was extracted with an organic solvent EtOAc, separated, dried, and subjected to rotary evaporation to remove most of the organic solvent. The residue was slurried with an organic solvent MTBE to obtain the compound Fmoc-Gly-Gly-Phe-Leu-OH as a white solid.

2) Synthesis of Compound H-Gly-Gly-Phe-Leu-OH

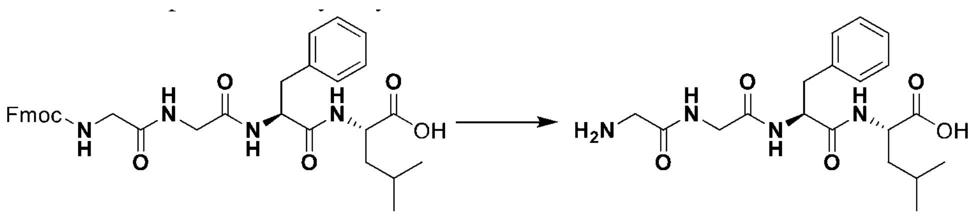

Fmoc-Gly-Gly-Phe-Leu-OH H-Gly-Gly-Phe-Leu-OH

Fmoc-Gly-Gly-Phe-Leu-OH (1.0 eq) was dissolved in DMF. Piperidine (5.0 eq) was added, and the mixture was stirred at room temperature for 2 h. The solvent was evaporated under reduced pressure under an oil pump, and the residue was poured into water. After filtration, the solid was dried, and the resulting crude product was slurried with an organic solvent MTBE to obtain H-Gly-Gly-Phe-Leu-OH as an off-white solid.

3) Synthesis of Compound EMC-Gly-Gly-Phe-Leu-OH

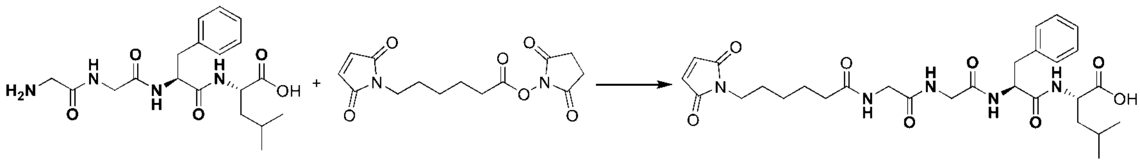

H-Gly-Gly-Phe-Leu-OH EMC-OSu EMC-Gly-Gly-Phe-Leu-OH

To a three-necked flask was added H-Gly-Gly-Phe-Leu-OH(1.0 eq), NaHCO3(5.0 eq), and solvent THF/H2O (4/1), the resulting mixture was stirred in an ice bath under nitrogen protection for 30 min. Then compound EMC-OSu (1.0 eq) was added in batches and the resulting mixture was stirred at room temperature for 10-13 h. The reaction solution was acidified by adding dilute hydrochloric acid, and the obtained crude product was extracted with an organic solvent EtOAc, separated, dried, and subjected to rotary evaporation to remove most of the organic solvent. The residue was slurried with an organic solvent MTBE to obtain the compound EMC-Gly-Gly-Phe-Leu-OH as a white solid.

4) Synthesis of Compound S1

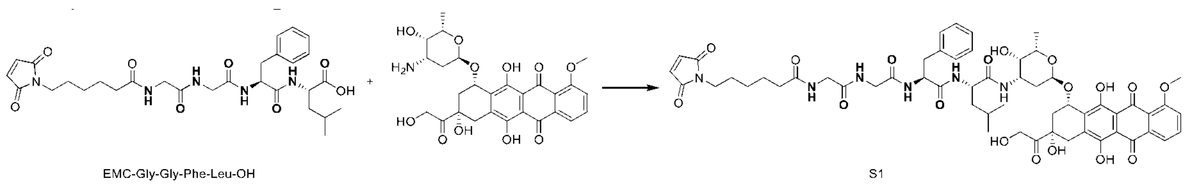

EMC-Gly-Gly-Phe-Leu-OH

S1

EMC-Gly-Gly-Phe-Leu-OH (1.0 eq) and DEPBT (1.2 eq) were added to a three-necked flask and dissolved in DMF solvent. The mixture was stirred in an ice bath under nitrogen protection for 30 min. Then compound doxorubicin (1.0 eq) and DIPEA (2.0 eq) were added separately at 0°C and the resulting mixture was stirred at room temperature for 10-13 h. Most of the organic solvent was removed by rotary evaporation, and the resulting crude product was separated and purified by high pressure preparative chromatography to obtain a red solid powder.

3.2. When the selected toxin molecule is replaced with Exatecan or its derivative DXd-NH2, T785, Melphalan, or MMAE, compounds S2, S3, S4, and S5 can be prepared by a similar method.

4. Synthesis of Compounds S6, S13, S14, S15, and S16

4.1. Synthesis of Compound S6:

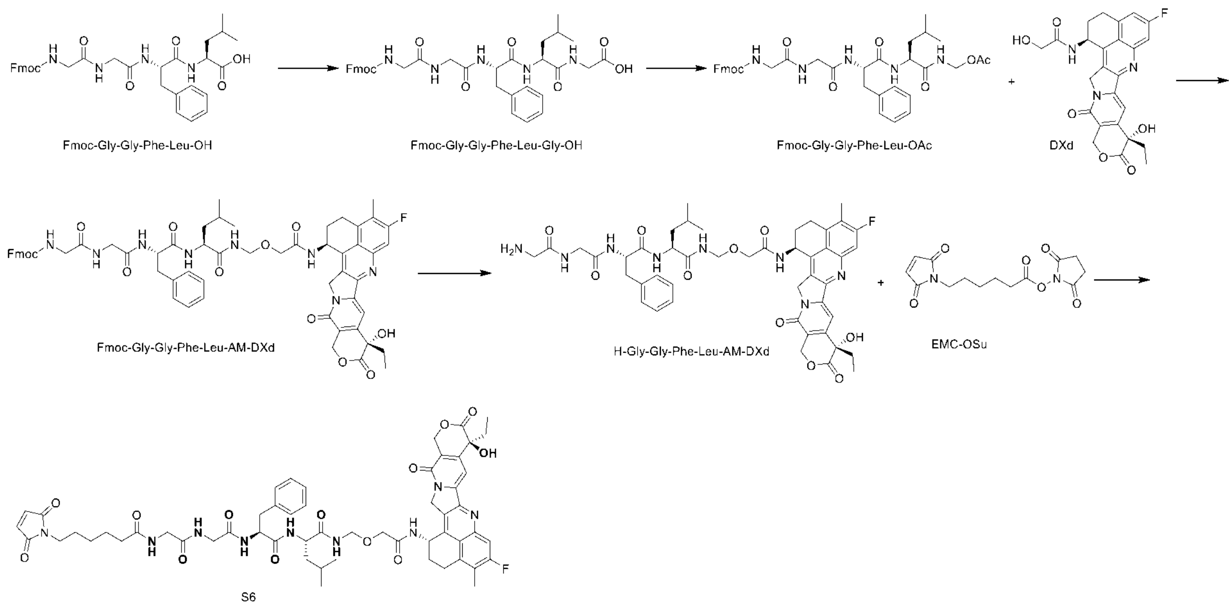

Fmoc-Gly-Gly-Phe-Leu-OH

Fmoc-Gly-Gly-Phe-Leu-Gly-OH

Fmoc-Gly-Gly-Phe-Leu-OAc

DXd

Fmoc-Gly-Gly-Phe-Leu-AM-DXd

H-Gly-Gly-Phe-Leu-AM-DXd

EMC-OSu

S6

1) Synthesis of Compound Fmoc-Gly-Gly-Phe-Leu-Gly-OH

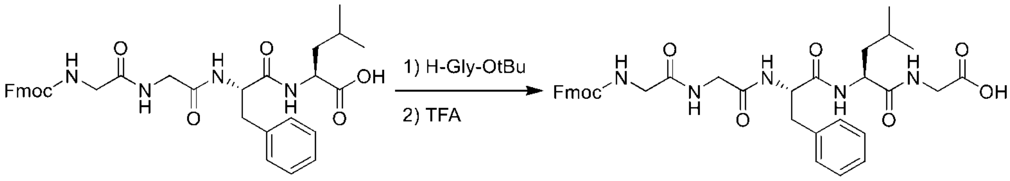


Fmoc-Gly-Gly-Phe-Leu-OH

Fmoc-Gly-Gly-Phe-Leu-Gly-OH

Fmoc-Gly-Gly-Phe-Leu-OH (1.0 eq) was dissolved in DMF, and cooled to 0 °C. H-Gly-OtBu (1.0 eq), HBTU (1.0 eq) and DIPEA (2.0 eq) were added under nitrogen protection, and the mixture was stirred for 2 to 3 hours at room temperature. TLC detection showed that the reaction of raw materials was completed. The reaction solution was diluted with dichloromethane, washed with water, and subjected to liquid seperation successively, the aqueous phase was

extracted with dichloromethane, the organic phases were combined and dried over anhydrous sodium sulfate, spin-dried, and subjected to column chromatography to obtain Fmoc-Gly-Gly-Phe-Leu-Gly-OtBu.

The above crude product was dissolved in DCM, TFA/DCM (20%) was added, and the mixture was stirred at room temperature for 2-3 hours. TLC detection showed that the reaction of raw materials was completed. The reaction solution was concentrated and separated by high pressure preparative chromatography to obtain Fmoc-Gly-Gly-Phe-Leu-Gly-OH.

2) Synthesis of Compound Fmoc-Gly-Gly-Phe-Leu-OAc

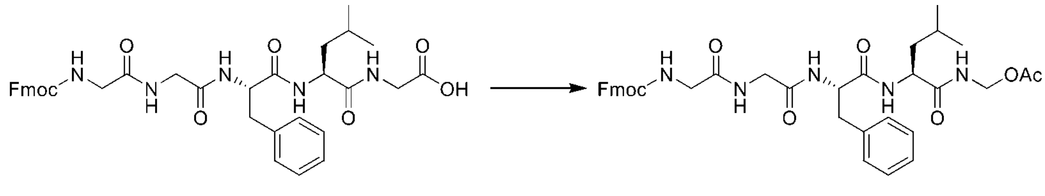

Fmoc-Gly-Gly-Phe-Leu-Gly-OH    Fmoc-Gly-Gly-Phe-Leu-OAc

Fmoc-Gly-Gly-Phe-Leu-Gly-OH (1.0 eq) was dissolved in dichloromethane/tetrahydrofuran (15/5 mL), and cooled to 0 °C. Lead acetate (1.2 eq) and copper acetate (0.2 eq) were added under nitrogen protection, and the resulting mixture was stirred at 40-50 °C under nitrogen protection for 2-3 hours. TLC detection showed that the reaction of raw materials was completed. The reaction solution was diluted with dichloromethane, washed with water, and subjected to liquid seperation successively, the aqueous phase was extracted with dichloromethane, the organic phases were combined and dried over anhydrous sodium sulfate, spin-dried, and subjected to column chromatography to obtain Fmoc-Gly-Gly-Phe-Leu-OAc.

3) Synthesis of Compound Fmoc-Gly-Gly-Phe-Leu-AM-DXd

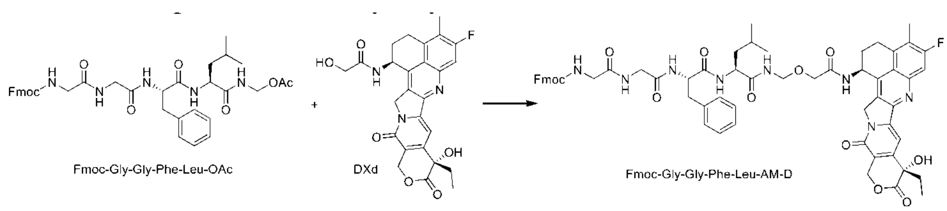


Fmoc-Gly-Gly-Phe-Leu-AM-OAc (1.0 eq), and compound Exatecan derivative DXd-OH(1.0 eq) were added to a three-necked flask, and dissolved in dichloromethane solution. TFA (0.3 eq) was added under nitrogen protection and in an ice bath, and the resulting mixture was stirred at room temperature for 2-3 h. After completion of the reaction, the reaction solution was concentrated and separated by high pressure preparative chromatography to obtain Fmoc-Gly-Gly-Phe-Leu-AM-DXd as a light yellow solid.

Synthesis of Compound H-Gly-Gly-Phe-Leu-AM-DXd

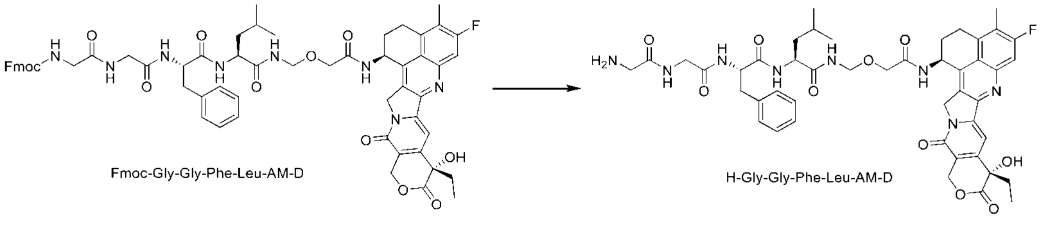


Compound Fmoc-Gly-Gly-Phe-Leu-AM-D (1.0 eq) was dissolved in DMF. Piperidine (2.0 eq) was added, and the

mixture was stirred at room temperature for 2-3 h under nitrogen protection. TLC detection showed that the reaction of raw materials was completed. The reaction solution was redissolved, and the solution was filtered, and then subjected to high pressure liquid chromatography to obtain the compound H-Gly-Gly-Phe-Leu-AM-DXd as a solid product.

4) Synthesis of Compound S6

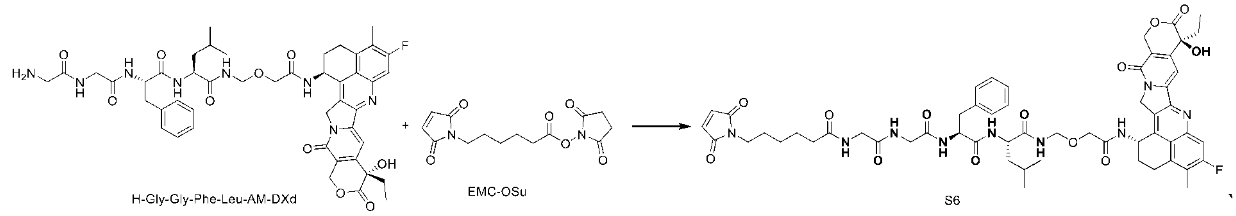
H-Gly-Gly-Phe-Leu-AM-DXd EMC-OSu S6

Compound EMC-OSu (1.0 eq) was dissolved in DMF (10 mL), and added with H-Gly-Gly-Phe-Leu-AM-DXd (1.0 eq). Under nitrogen protection, DIPEA (2.0 eq) was added and the mixture was stirred at room temperature for 1-2 h. TLC detection showed that the reaction of raw materials was completed. The crude reaction solution was filtered, and then subjected to high pressure liquid chromatography to obtain the compound S6 as a light yellow solid product.
4.2. When the selected toxin molecule is replaced by Paclitaxel, SN 38 and Gemcitabine, respectively, compounds S13, S14, S15 and S16 can be prepared by similar methods.

5. Synthesis of Compounds S7, S8, S9, and S10:

5.1. Synthesis of Compound S7 (MI-PEG6-GGFL-AM-DXD):

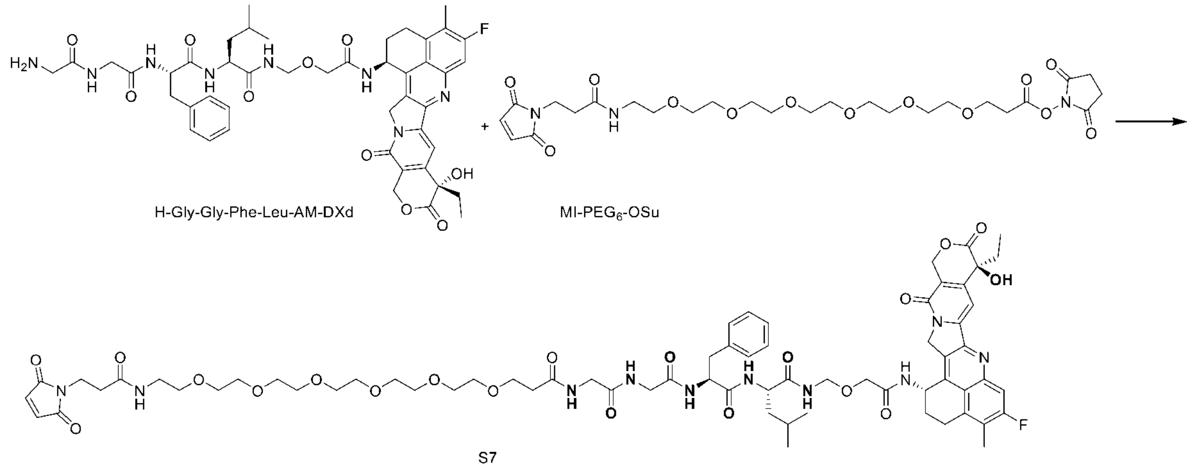
H-Gly-Gly-Phe-Leu-AM-DXd MI-PEG$_6$-OSu

S7

Compound MI-PEG6-OSu (1.0 eq) was dissolved in DMF (10 mL), and added with H-Gly-Gly-Phe-Leu-AM-DXd (1.0 eq). Under nitrogen protection, DIPEA (2.0 eq) was added and the mixture was stirred at room temperature for 1-2 h. TLC detection showed that the reaction of raw materials was completed. The crude reaction solution was filtered, and then subjected to high pressure liquid chromatography to obtain the compound S7 as a light yellow solid product.
5.2. When MI-PEG$_6$-OSu is replaced with the corresponding branched structure, compounds S8, S9 and S10 can be prepared by a similar method.

6. Synthesis of Compounds S11, and S12

6.1. Synthesis of Compound S11:

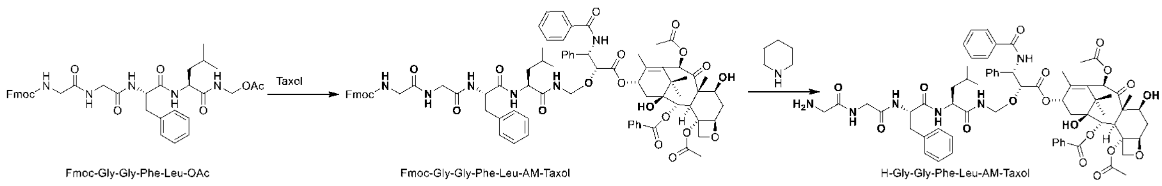

Fmoc-Gly-Gly-Phe-Leu-OAc
Fmoc-Gly-Gly-Phe-Leu-AM-Taxol
H-Gly-Gly-Phe-Leu-AM-Taxol

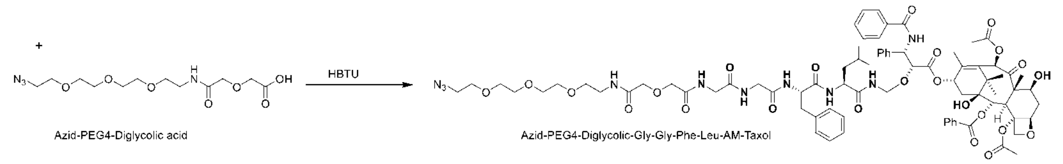

Azid-PEG4-Diglycolic acid
Azid-PEG4-Diglycolic-Gly-Gly-Phe-Leu-AM-Taxol

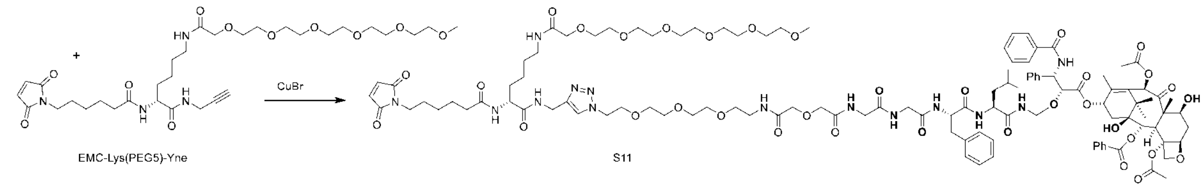

EMC-Lys(PEG5)-Yne
S11

1) Synthesis of Compound Fmoc-Gly-Gly-Phe-Leu-AM-DXd

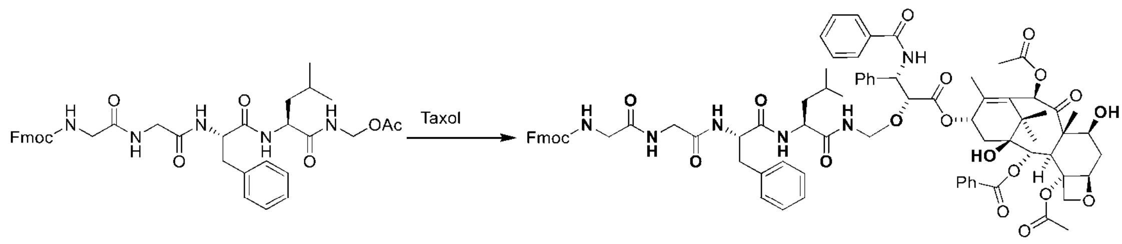

Fmoc-Gly-Gly-Phe-Leu-OAc
Fmoc-Gly-Gly-Phe-Leu-AM-Taxol

Fmoc-Gly-Gly-Phe-Leu-AM-OAc (1.0 eq), and compound Paclitaxel (1.0 eq) were added to a three-necked flask, and dissolved in dichloromethane solution. TFA (0.3 eq) was added under nitrogen protection and in an ice bath, and the resulting mixture was stirred at room temperature for 2-3 h. After completion of the reaction, the reaction solution was concentrated and separated by high pressure preparative chromatography to obtain Fmoc-Gly-Gly-Phe-Leu-AM-Taxol as a white solid.

2) Synthesis of Compound H-Gly-Gly-Phe-Leu-AM-DXd

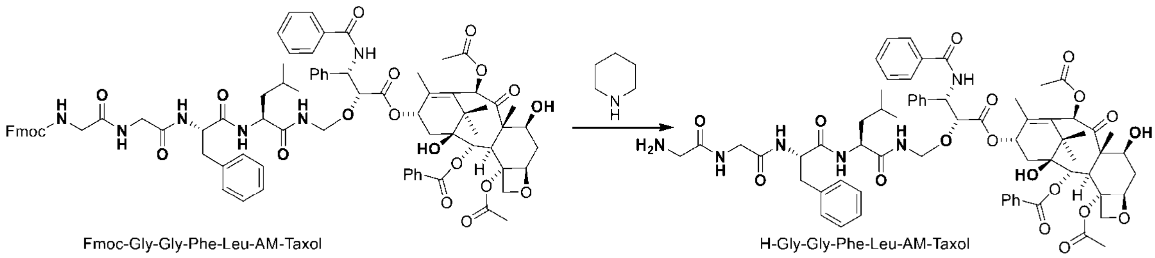
Fmoc-Gly-Gly-Phe-Leu-AM-Taxol
H-Gly-Gly-Phe-Leu-AM-Taxol

Compound Fmoc-Gly-Gly-Phe-Leu-AM-Taxol (1.0 eq) was added to a three-necked flask, and dissolved in DMF. Piperidine (0.2 eq) was added, and the mixture was stirred at room temperature for 2-3 h under nitrogen protection. After completion of the reaction, the reaction solution was concentrated and separated by high pressure preparative chromatography to obtain H-Gly-Gly-Phe-Leu-AM-Taxol as a white solid.

3) Synthesis of Compound H-Gly-Gly-Phe-Leu-AM-DXd

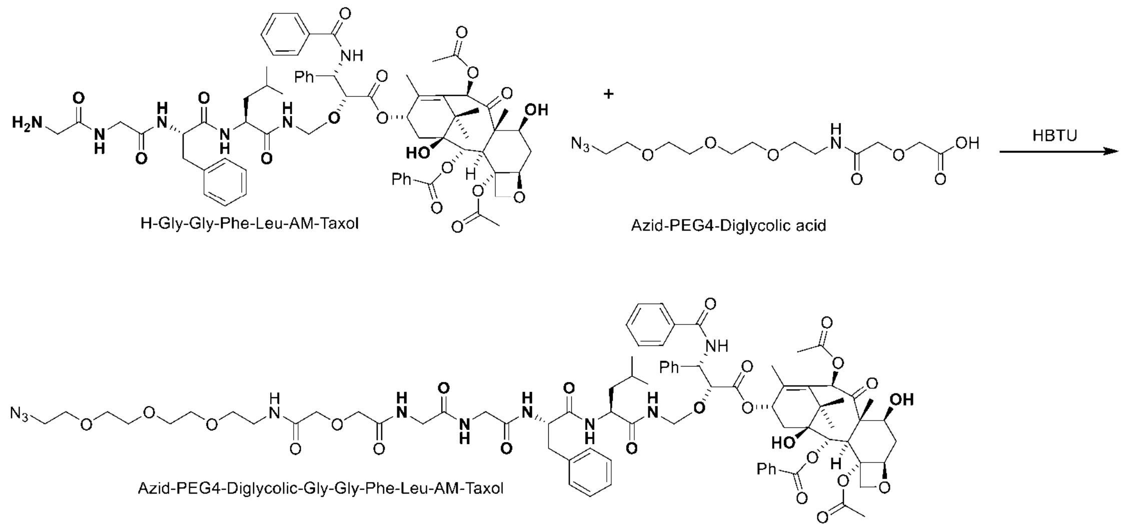


H-Gly-Gly-Phe-Leu-AM-Taxol

Azid-PEG4-Diglycolic acid

Azid-PEG4-Diglycolic-Gly-Gly-Phe-Leu-AM-Taxol

Compound Azid-PEG4-Diglycolic acid (1.0 eq) was dissolved in DMF (10 mL), and added with H-Gly-Gly-Phe-Leu-AM-Taxol (1.0 eq). Under nitrogen protection and in ice bath, HBTU (1.2 eq) and DIPEA (2.0 eq) were added and the mixture was stirred at room temperature for 1-2 h. TLC detection showed that the reaction of raw materials was completed. The reaction solution was redissolved, and the solution was filtered, and then subjected to high pressure liquid chromatography to obtian compound Azid-PEG4-Diglycolic-Gly-Gly-Phe-Leu-AM-Taxol as a white solid product.

4) Synthesis of Compound S11

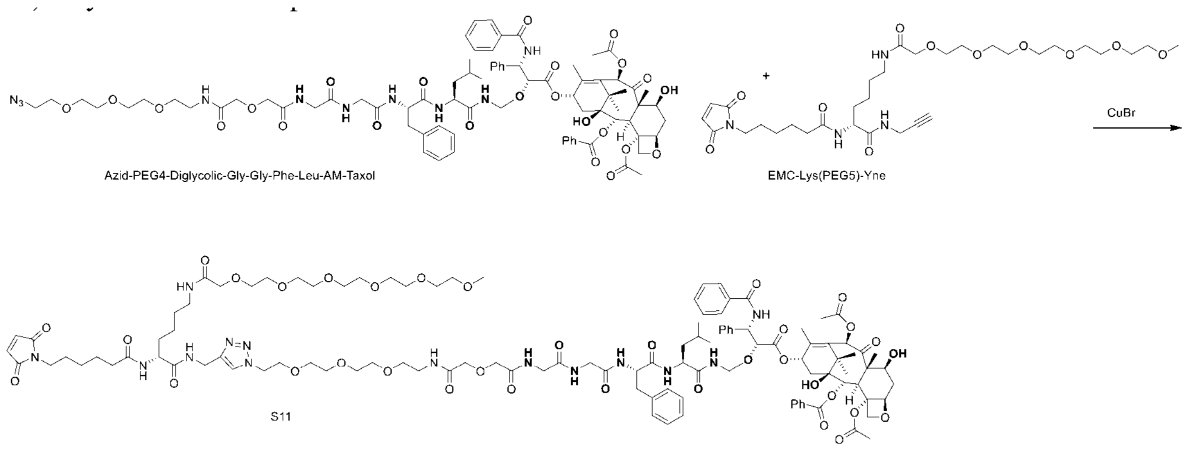


Azid-PEG4-Diglycolic-Gly-Gly-Phe-Leu-AM-Taxol

EMC-Lys(PEG5)-Yne

S11

Compound Azid-PEG4-Diglycolic-Gly-Gly-Phe-Leu-AM-Taxol (1.0 eq) was dissolved in DMSO/H2O (9/1 10 mL), and added with EMC-Lys (PEG5)-Yne(1.0 eq). CuBr (1.2 eq) was added under nitrogen protection and the mixture was stirred at room temperature for 1-2 h. TLC detection showed that the reaction of raw materials was completed. The crude reaction solution was filtered, and then subjected to high pressure liquid chromatography to obtain compound S11 as a white solid product.

6.2. When EMC-Lys(PEG5)-Yne is replaced with EMC-Lys(PEG8)-Yne, compound S12 can be prepared by a similar method.

7. Synthesis of Compounds S17, S18, S30, and S34

7.1 Synthesis of Compound S17:

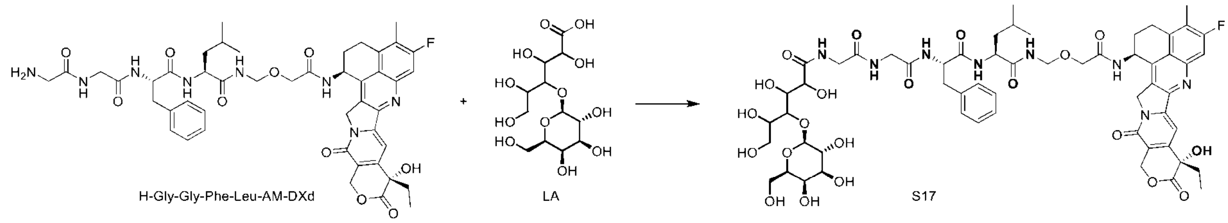

H-Gly-Gly-Phe-Leu-AM-DXd

LA

S17

Compound H-Gly-Gly-Phe-Leu-AM-DXd (1.0 eq) was dissolved in DMF (10 mL), and added with lactobionic acid (2.0 eq). HBTU (1.2 eq) and DIPEA (2.0 eq) were added in ice bath and under nitrogen protection, and the mixture was stirred at room temperature for 1-2 h. TLC detection showed that the reaction of raw materials was completed. The reaction solution was redissolved, and the solution was filtered, and then subjected to high pressure liquid chromatography to obtain compound S17 as a light yellow solid product.

7.2. When lactobionic acid LA is replaced with galactosamine GalNAc, S18 can be prepared by a similar method.

7.3. When lactobionic acid LA is replaced with BCN or DBCO, S30 and S34 can be prepared by a similar method.

8. Synthesis of Compounds S19 and S20

8.1. Synthesis of Compound S19:

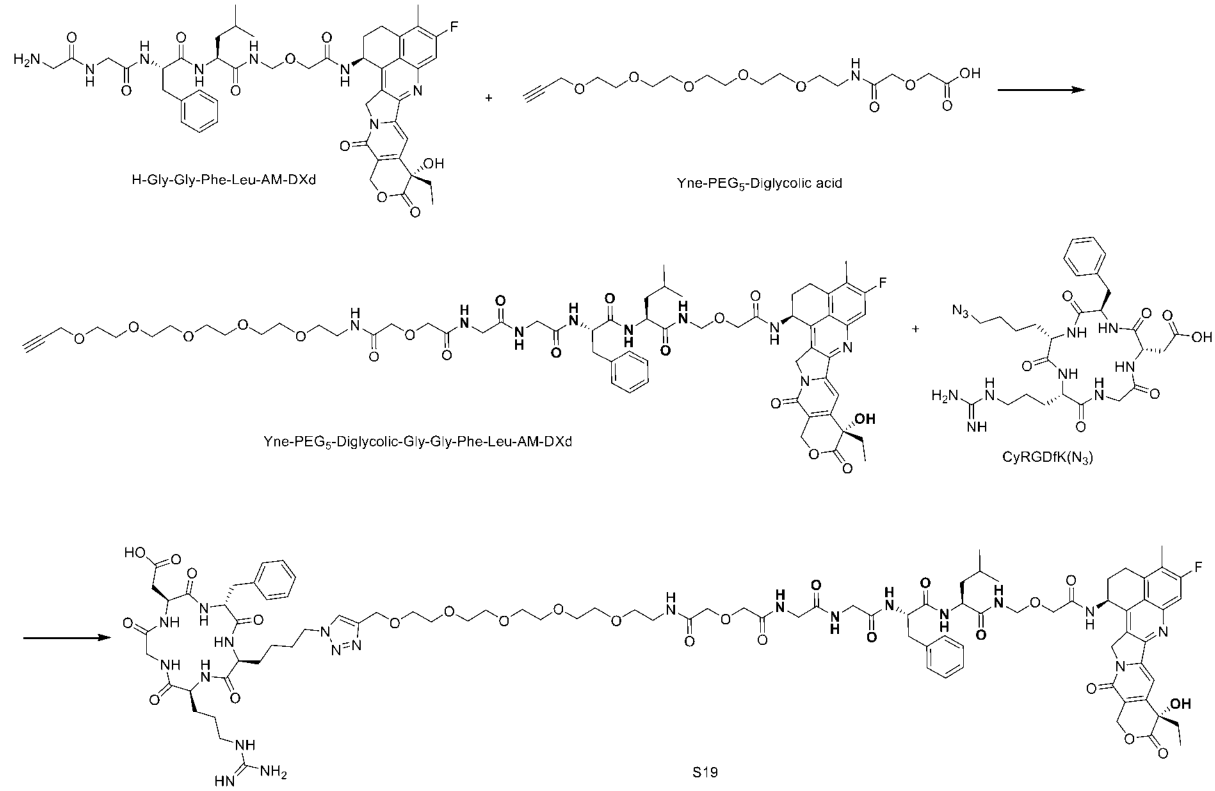

H-Gly-Gly-Phe-Leu-AM-DXd

Yne-PEG$_5$-Diglycolic acid

Yne-PEG$_5$-Diglycolic-Gly-Gly-Phe-Leu-AM-DXd

CyRGDfK(N$_3$)

S19

1) Synthesis of Compound Yne-PEG5-Diglycolic-Gly-Gly-Phe-Leu-AM-DXd

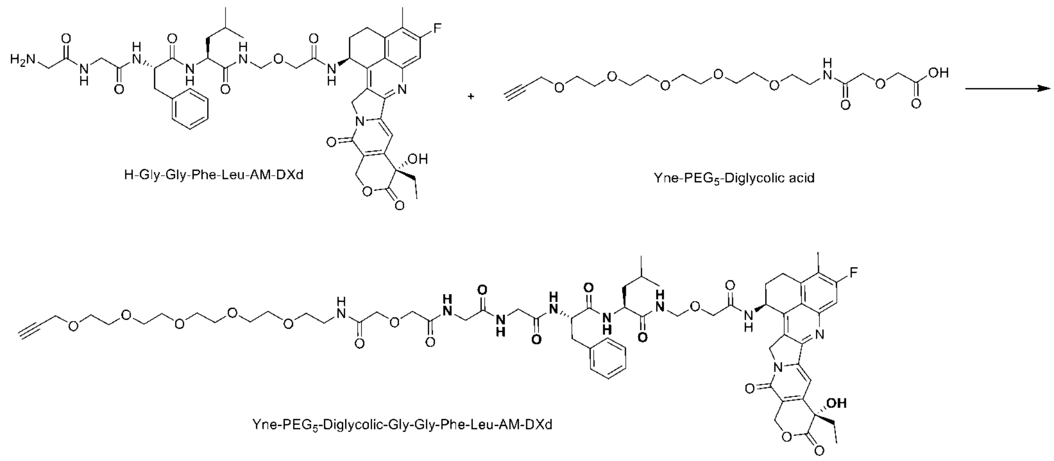

H-Gly-Gly-Phe-Leu-AM-DXd

Yne-PEG$_5$-Diglycolic acid

Yne-PEG$_5$-Diglycolic-Gly-Gly-Phe-Leu-AM-DXd

Compound Yne-PEG5-Diglycolic acid (1.0 eq) was dissolved in DMF (10 mL), and added with H-Gly-Gly-Phe-Leu-AM-DXd (1.0 eq). HBTU (1.2 eq) and DIPEA (2.0 eq) were added under nitrogen protection, and the mixture was stirred at room temperature for 1-2 h. TLC detection showed that the reaction of raw materials was completed. The crude reaction solution was filtered, and then subjected to high pressure liquid chromatography to obtain compound Yne-PEG5-Diglycolic-Gly-Gly-Phe-Leu-AM-DXd as a light yellow solid product.

2) Synthesis of Compound S19

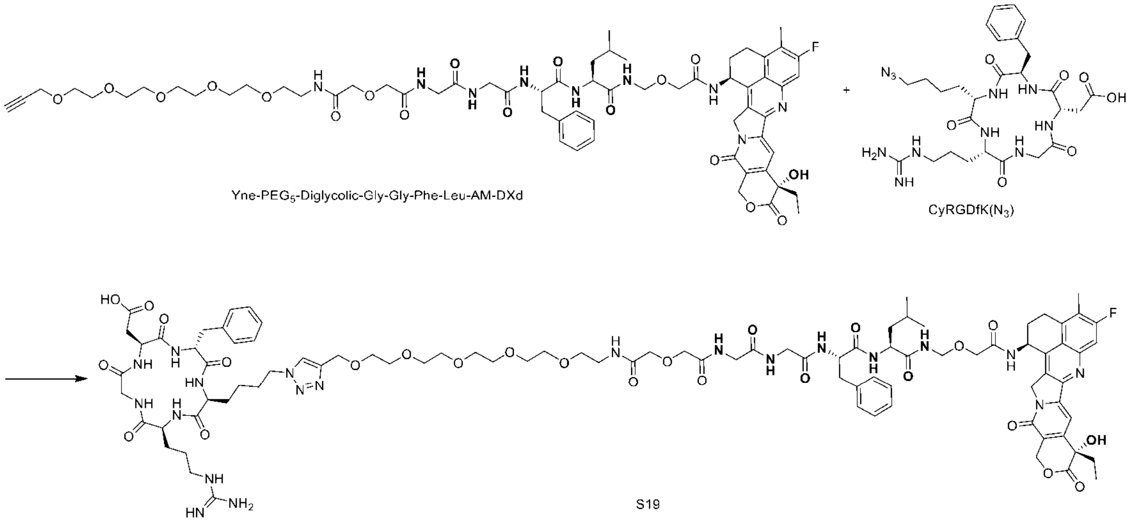

Yne-PEG$_5$-Diglycolic-Gly-Gly-Phe-Leu-AM-DXd

CyRGDfK($N_3$)

S19

Compound CyRGDfK($N_3$) (1.0 eq) was dissolved in DMSO/$H_2O$ (9/1 10 mL), and added with Yne-PEG5-Diglycolic-Gly-Gly-Phe-Leu-AM-DXd (1.0 eq). CuBr (1.2 eq) was added under nitrogen protection, and the mixture was stirred at room temperature for 1-2 h. TLC detection showed that the reaction of raw materials was completed. The crude reaction solution was filtered, and then subjected to high pressure liquid chromatography to obtain compound S19 as a light yellow solid product.

8.2. When CyRGDfK($N_3$) is replaced with CyRGDyK($N_3$), S20 can be prepared by a similar method.

9. Synthesis of Compounds S21 and S22

9.1 Synthesis of Compound S21:

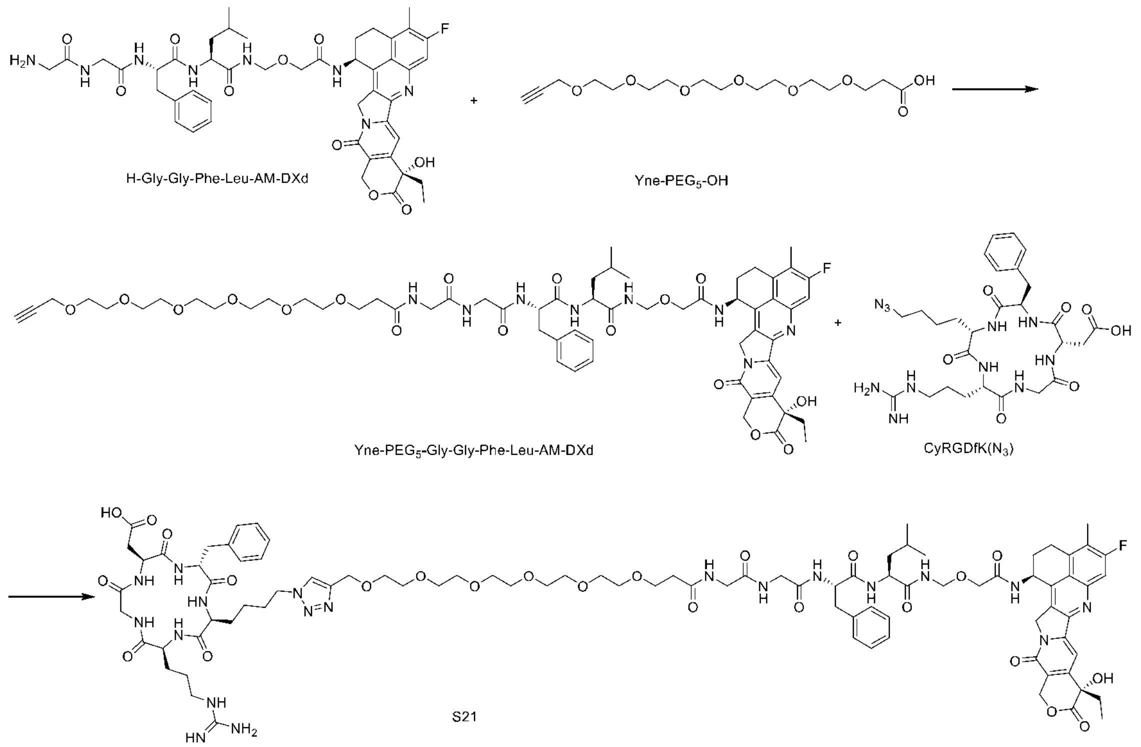

H-Gly-Gly-Phe-Leu-AM-DXd

Yne-$PEG_5$-OH

Yne-$PEG_5$-Gly-Gly-Phe-Leu-AM-DXd

CyRGDfK($N_3$)

S21

1) Synthesis of Compound Yne-PEG5-Gly-Gly-Phe-Leu-AM-DXd

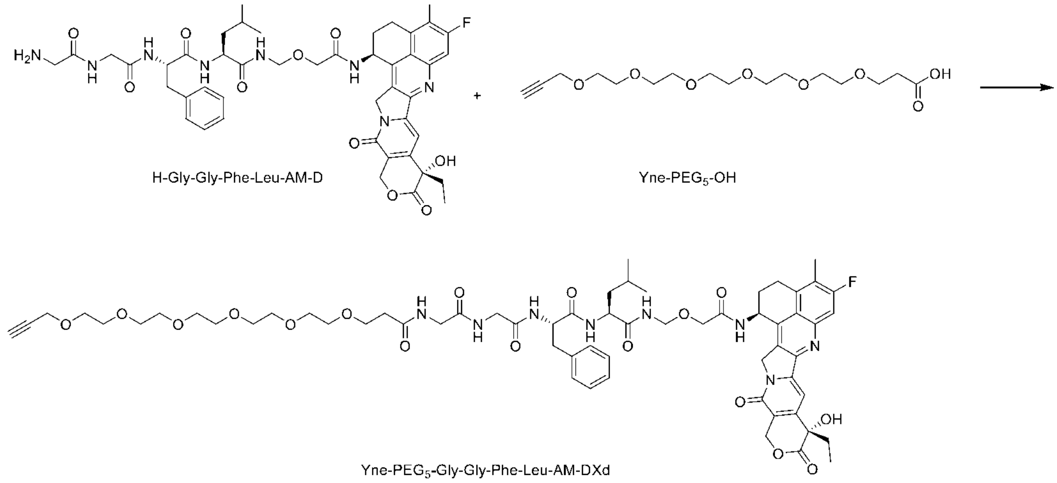

H-Gly-Gly-Phe-Leu-AM-D

Yne-$PEG_5$-OH

Yne-$PEG_5$-Gly-Gly-Phe-Leu-AM-DXd

Compound Yne-PEG5-OH (1.0 eq) was dissolved in DMF (10 mL), and added with H-Gly-Gly-Phe-Leu-AM-DXd (1.0 eq). HBTU (1.2 eq) and DIPEA (2.0 eq) were added under nitrogen protection, and the mixture was stirred at room temperature for 1-2 h. TLC detection showed that the reaction of raw materials was completed. The crude reaction solution was filtered, and then subjected to high pressure liquid chromatography to obtain compound Yne-PEG5-Gly-Gly-Phe-Leu-AM-D as a light yellow solid product.

2) Synthesis of Compound S21

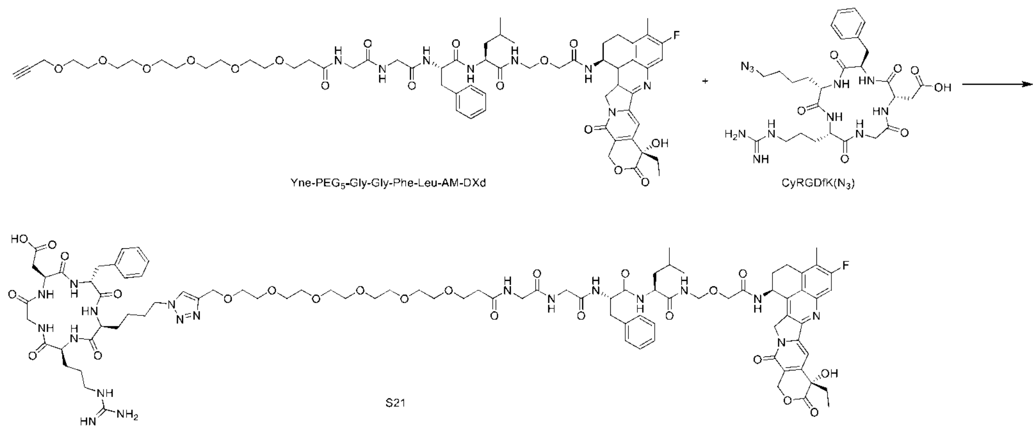

Yne-PEG5-Gly-Gly-Phe-Leu-AM-DXd CyRGDfK(N3)

S21

Compound CyRGDfK($N_3$) (1.0 eq) was dissolved in DMSO/H2O (9/1 10 mL), and added with Yne-PEG5-Gly-Gly-Phe-Leu-AM-DXd (1.0 eq). CuBr (1.2 eq) was added under nitrogen protection, and the mixture was stirred at room temperature for 1-2 h. TLC detection showed that the reaction of raw materials was completed. The crude reaction solution was filtered, and then subjected to high pressure liquid chromatography to obtain compound S21 as a light yellow solid product.

9.2. When CyRGDfK($N_3$) is replaced with CyRGDyK($N_3$), S22 can be prepared by a similar method.

10. Synthesis of Compounds S23, S24, and S25

10.1. Synthesis of Compound S23

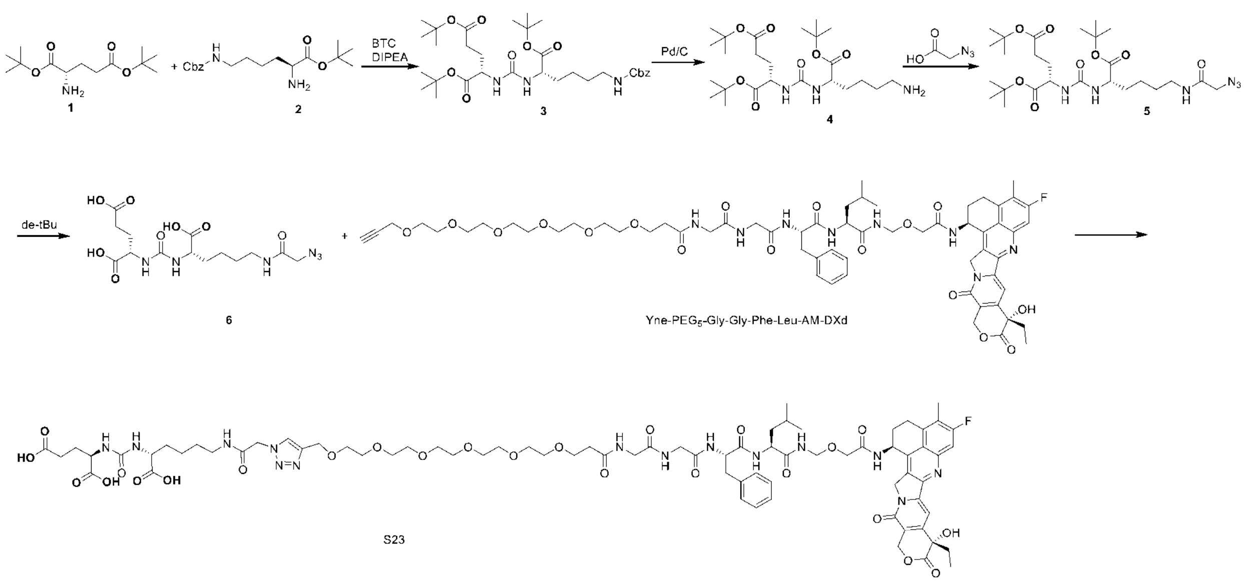


1 2 3 4 5 6

Yne-PEG5-Gly-Gly-Phe-Leu-AM-DXd

S23

1) Synthesis of Compound 3

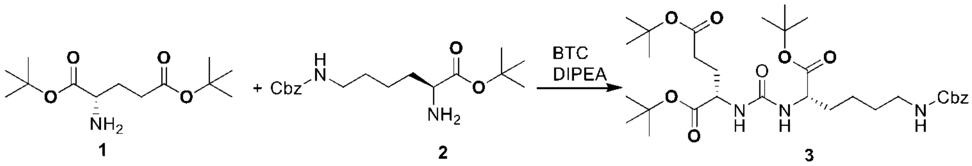


1 2 3

Compound 1 (10.0 g, 1.0 eq) was dissolved in DCM, BTC (0.33 eq) was added at -78°C, and compound 2 (1.1 eq) and triethylamine (3.4 eq) were added 2 h later. The mixture was stirred at room temperature for 16 h, quenched with water, and extracted with ethyl acetate, and the organic solvent was removed by rotary evaporation. The residues were loaded on silica gel and subjected to column chromatography (PE/EtOAc=60:40) to obtain compound 3 (10 g) as a white solid.

2) Synthesis of Compound 4

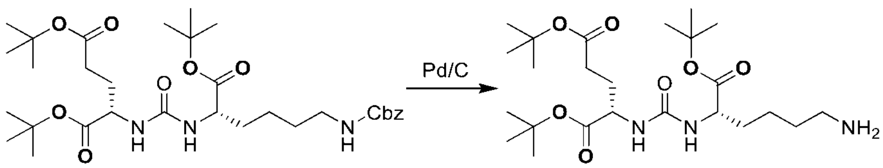


3 4

Compound 3 (10 g, 1.0 eq) was dissolved in DCM, and Pd/C (2 g) was added at room temperature. The mixture was stirred at room temperature overnight. The reaction solution was filtered to remove Pd/C, then loaded on silica gel and subjected to column chromatography (DCM/MeOH = 92/8) to obtain compound 4 (6.5 g) as an off-white solid.

3) Synthesis of Compound 5

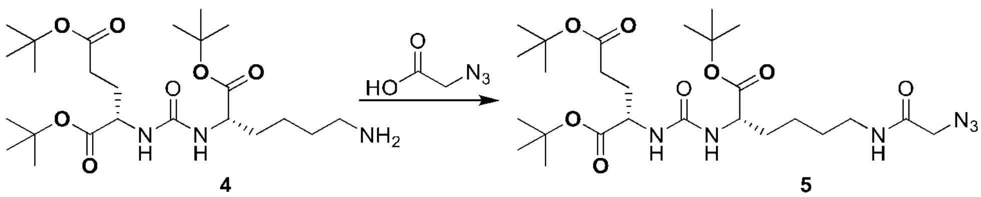

4 5

Compound 4 (720 mg, 1.0 eq) and azidoacetic acid (1.2 eq) were dissolved in DMF, and DIPEA (1.2 eq) and HBTU (1.2 eq) were added at room temperature. The mixture was stirred at room temperature overnight. The reaction solution was quenched with sat. NaHCO3 solution, extracted with ethyl acetate, loaded on silica gel, and subjected to column chromatography (PE/EtOAc =1/1) to obtain (870 mg) a colorless liquid.

4) Synthesis of Compound 6

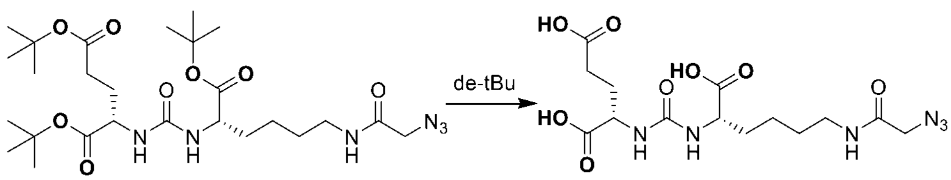


5 6

Compound 6 (150 mg) was dissolved in formic acid (1 mL) and stirred at room temperature for 2 h. LCMS showed that the reaction was complete. The solvent was dried and the product was directly separated by high pressure preparative chromatography to obtain product compound 6 (70 mg).

5) Synthesis of Compound S23

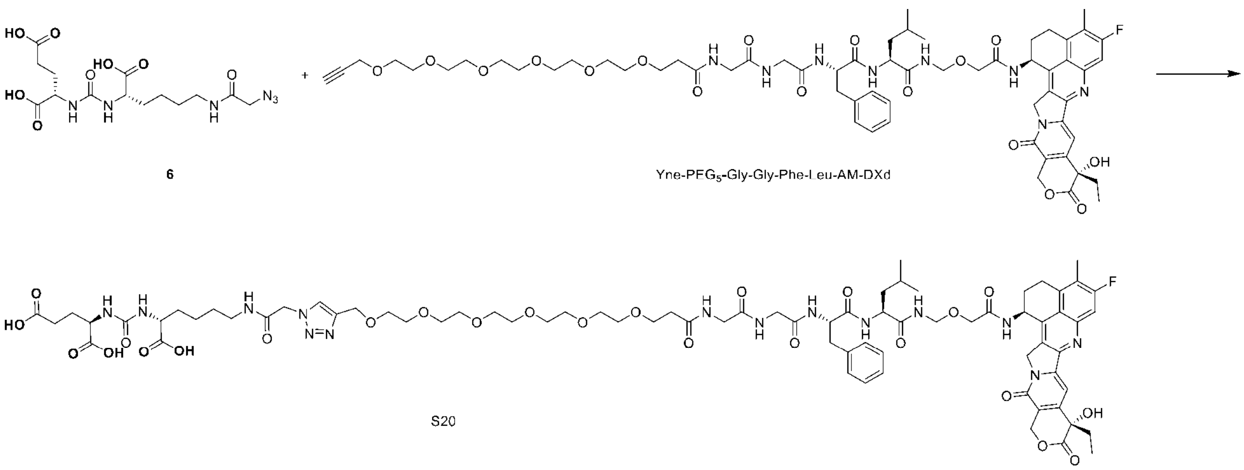

6

Yne-PEG$_5$-Gly-Gly-Phe-Leu-AM-DXd

S20

Compound 6 (1.0 eq) was dissolved in DMSO/H2O (9/1 10 mL), and added with Yne-PEG5-Gly-Gly-Phe-Leu-AM-DXd (1.0 eq). Under nitrogen protection, CuBr (1.2 eq) were added and the mixture was stirred at room temperature for 1-2 h. TLC detection showed that the reaction of raw materials was completed. The reaction solution was filtered, and then subjected to high pressure liquid chromatography to obtain compound S23 as a light yellow solid product.

10.2. When the toxin molecule Exatecan derivative (DXd-OH) in the above compound is replaced by Paclitaxel and Docetaxel, respectively, compounds S24 and S25 can be prepared by similar methods.

11. Synthesis of Compound S26:

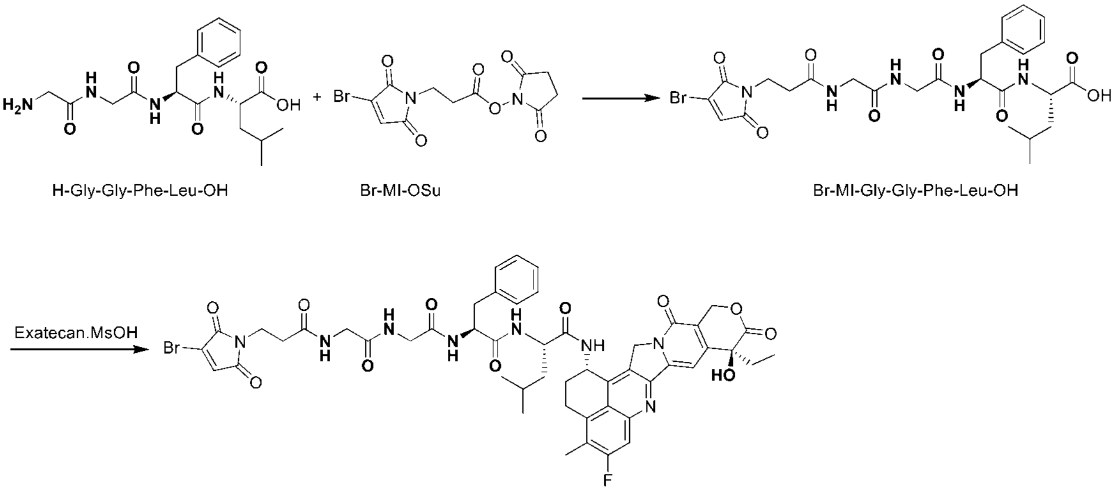


H-Gly-Gly-Phe-Leu-OH

Br-MI-OSu

Br-MI-Gly-Gly-Phe-Leu-OH

1) Synthesis of Compound Br-MI-Gly-Gly-Phe-Leu-OH

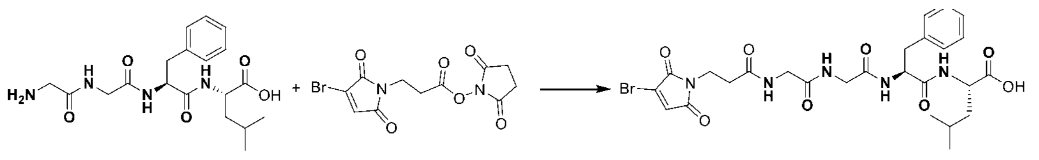

H-Gly-Gly-Phe-Leu-OH

Br-MI-OSu

Br-MI-Gly-Gly-Phe-Leu-OH

To a three-necked flask was added H-Gly-Gly-Phe-Leu-OH (1.0 eq), $NaHCO_3$ (5.0 eq), and solvent THF/$H_2O$ (4/1), and the resulting mixture was stirred in an ice bath under nitrogen protection for 30 min. Then compound Br-MI-OSu (1.0 eq) was added in batches and the resulting mixture was stirred at room temperature for 10-13 h. The reaction solution

was acidified by adding dilute hydrochloric acid, and the obtained crude product was extracted with an organic solvent EtOAc, separated, dried, and subjected to rotary evaporation to remove most of the organic solvent. The residue was slurried with an organic solvent MTBE to obtain compound Br-MI-Gly-Gly-Phe-Leu-OH as a white solid.

2) Synthesis of Compound S26

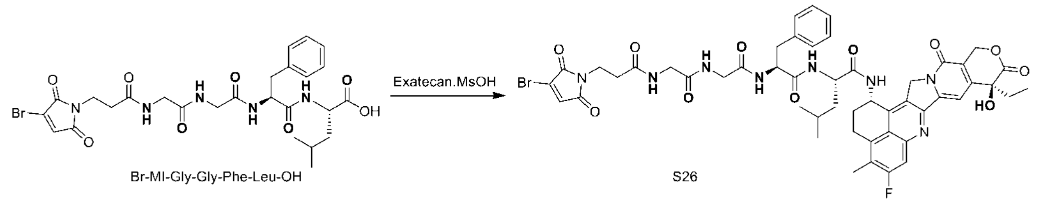

Br-MI-Gly-Gly-Phe-Leu-OH
S26

Br-MI-Gly-Gly-Phe-Leu-OH (1.0 eq) and DEPBT (1.2 eq) were added to a three-necked flask and dissolved in DMF solvent. The mixture was stirred in an ice bath under nitrogen protection for 30 min. Then compound Exatecan (1.0 eq) and DIPEA (3.0 eq) were added separately at 0°C and the resulting mixture was stirred at room temperature for 10-13 h. Most of the organic solvent was removed by rotary evaporation, and the resulting crude product was separated and purified by high pressure preparative chromatography to obtain a light yellow solid powder.

12. Synthesis of Compounds S27, S28, and S29:

12.1. Synthesis of Compound S27:

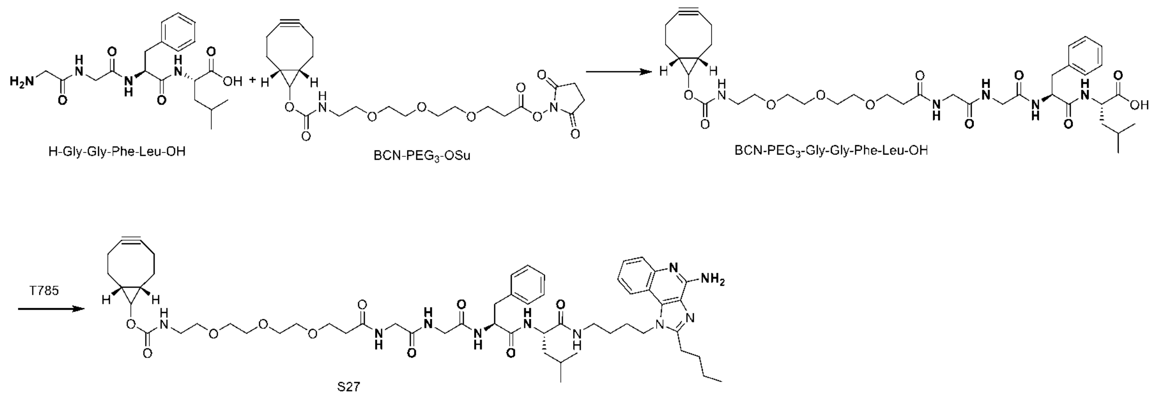

H-Gly-Gly-Phe-Leu-OH
BCN-PEG$_3$-OSu
BCN-PEG$_3$-Gly-Gly-Phe-Leu-OH
S27

1) Synthesis of Compound BCN-PEG3-Gly-Gly-Phe-Leu-OH

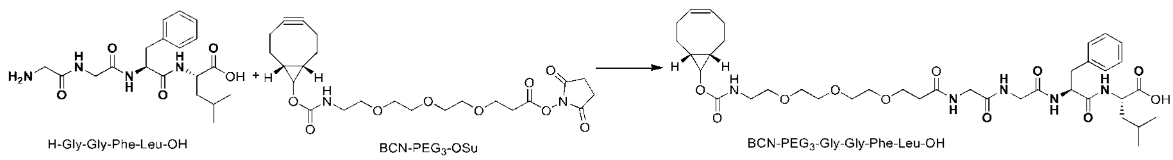
H-Gly-Gly-Phe-Leu-OH
BCN-PEG$_3$-OSu
BCN-PEG$_3$-Gly-Gly-Phe-Leu-OH

To a three-necked flask was added H-Gly-Gly-Phe-Leu-OH(1.0 eq), NaHCO3(5.0 eq), and solvent THF/H2O (4/1), and the resulting mixture was stirred in an ice bath under nitrogen protection for 30 min. Then compound BCN-PEG3-OSu (1.0 eq) was added in batches and the resulting mixture was stirred at room temperature for 10-13 h. The reaction solution was acidified by adding dilute hydrochloric acid, and the obtained crude product was extracted with an organic solvent EtOAc, separated, dried, and subjected to rotary evaporation to remove most of the organic solvent. The residue was slurried with an organic solvent MTBE to obtain compound BCN-PEG3-Gly-Gly-Phe-Leu-OH as a white solid.

2) Synthesis of Compound S27

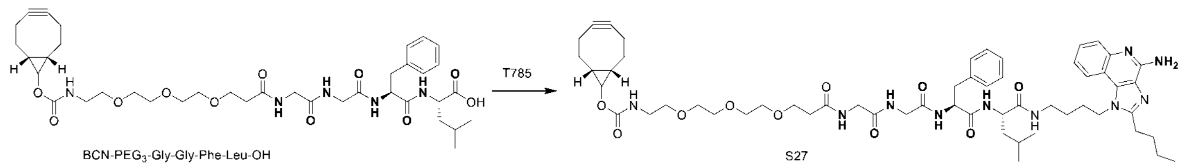


BCN-$PEG_3$-Gly-Gly-Phe-Leu-OH S27

BCN-PEG3-Gly-Gly-Phe-Leu-OH (1.0 eq) and DEPBT (1.2 eq) were added to a three-necked flask and dissolved in DMF solvent. The mixture was stirred in an ice bath under nitrogen protection for 30 min. Then compound T785 (1.0 eq) and DIPEA (2.0 eq) were added separately at 0°C and the resulting mixture was stirred at room temperature for 10-13 h. Most of the organic solvent was removed by rotary evaporation, and the resulting crude product was separated and purified by high pressure preparative chromatography to obtain a white solid powder.

12.2. When T785 is replaced with Exatecan derivative DXd-NH2 and MMAE respectively, compounds S28 and S29 can be prepared by a similar method.

13. Synthesis of Compounds S31, S32, and S33:

13.1 Synthesis of Compound S31

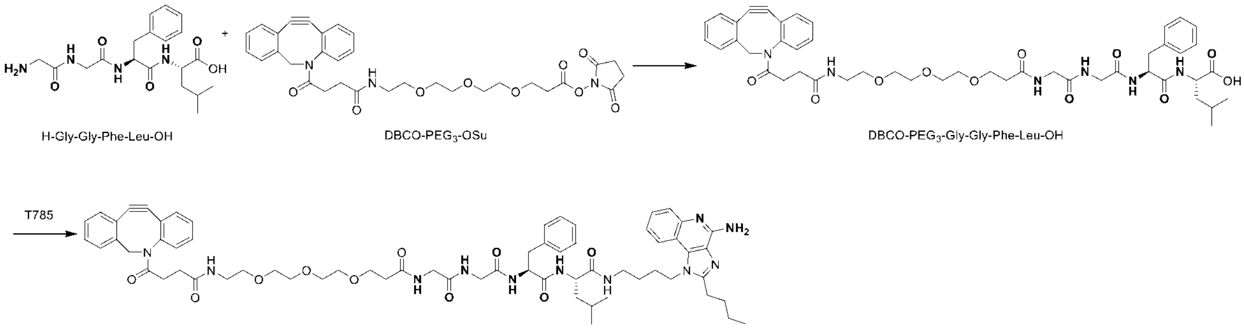


H-Gly-Gly-Phe-Leu-OH + DBCO-$PEG_3$-OSu DBCO-$PEG_3$-Gly-Gly-Phe-Leu-OH

S31

1) Synthesis of Compound DBCO-PEG3-Gly-Gly-Phe-Leu-OH

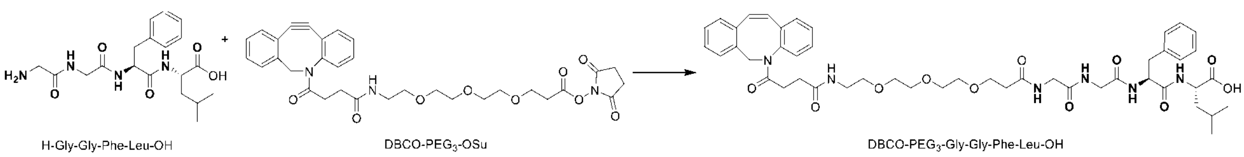

H-Gly-Gly-Phe-Leu-OH + DBCO-$PEG_3$-OSu DBCO-$PEG_3$-Gly-Gly-Phe-Leu-OH

To a three-necked flask was added H-Gly-Gly-Phe-Leu-OH (1.0 eq), $NaHCO_3$ (5.0 eq), and solvent THF/$H_2O$ (4/1), and the resulting mixture was stirred in an ice bath under nitrogen protection for 30 min. Then compound DBCO-PEG3-OSu (1.0 eq) was added in batches and the resulting mixture was stirred at room temperature for 10-13 h. The reaction solution was acidified by adding dilute hydrochloric acid, and the obtained crude product was extracted with an organic solvent EtOAc, separated, dried, and subjected to rotary evaporation to remove most of the organic solvent. The residue was slurried with an organic solvent MTBE to obtain compound DBCO-PEG3-Gly-Gly-Phe-Leu-OH as a white solid.

2) Synthesis of Compound S31

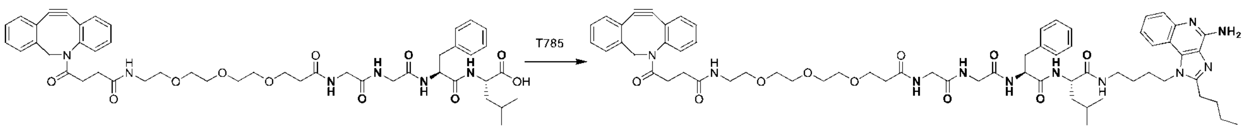


EMC-Gly-Gly-Phe-Leu-OH (1.0 eq) and DEPBT (1.2 eq) were added to a three-necked flask and dissolved in DMF solvent. The mixture was stirred in an ice bath under nitrogen protection for 30 min. Then compound T785 (1.0 eq) and DIPEA (2.0 eq) were added separately at 0°C and the resulting mixture was stirred at room temperature for 10-13 h. Most of the organic solvent was removed by rotary evaporation, and the resulting crude product was separated and purified by high pressure preparative chromatography to obtain a white solid powder.

112.2. When T785 is replaced with Exatecan derivative DXd-NH2 and MMAE respectively, compounds S32 and S33 can be prepared by a similar method.

14. Synthesis of Leu-AM-DXd:

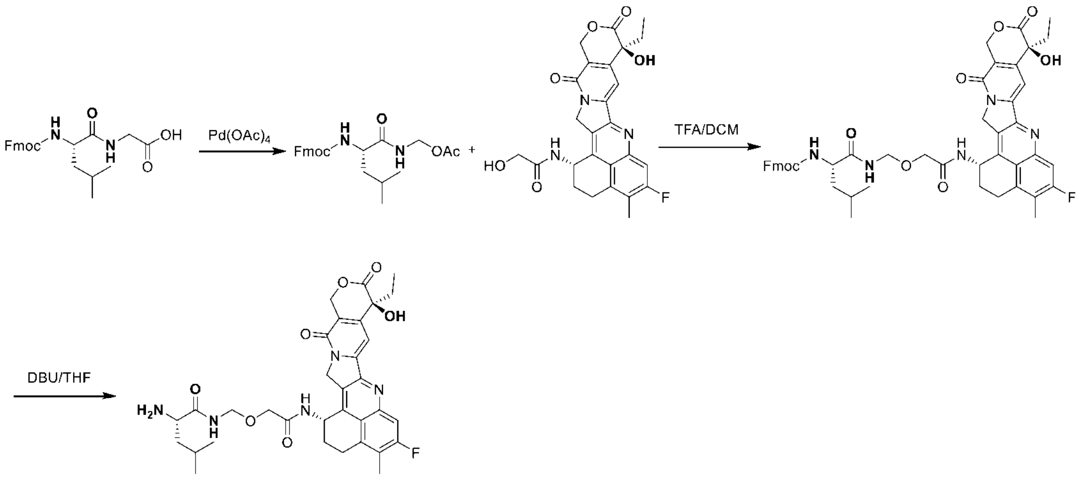


Leu-AM-DXd

Step 1: Synthesis of Compound Fmoc-Leu-OAc

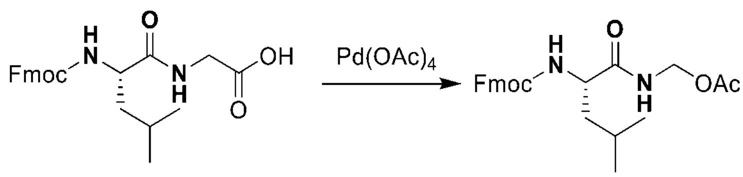


Fmoc-Leu-Gly-OH (1.0 eq) was dissolved in dichloromethane/tetrahydrofuran (15/5 mL), and cooled to 0 °C. Lead acetate (1.2 eq) and copper acetate (0.2 eq) were added under nitrogen protection, and the resulting mixture was stirred at 40-50 °C under nitrogen protection for 2-3 hours. TLC detection showed that the reaction of raw materials was completed. The reaction solution was diluted with dichloromethane, washed with water, and subjected to liquid seperation successively; the aqueous phase was extracted with dichloromethane, and the organic phases were combined and dried over anhydrous sodium sulfate, spin-dried, and subjected to column chromatography to obtain Fmoc-Leu-OAc.

Step 2: Synthesis of Compound Fmoc-Leu-AM-DXd

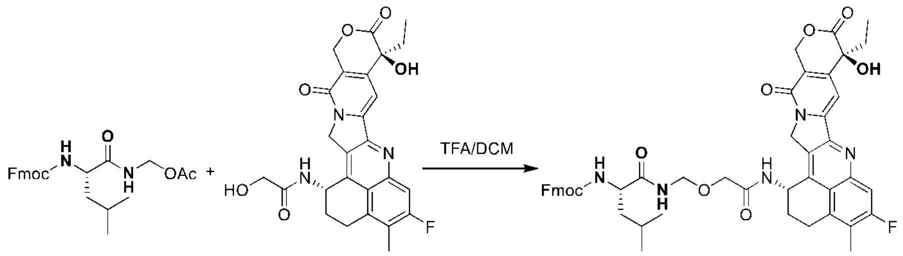


Fmoc-Leu-AM-OAc (1.0 eq), and compound DXd (1.0 eq) were added to a three-necked flask, and dissolved in dichloromethane solution. TFA (0.3 eq) was added under nitrogen protection and in an ice bath, and the resulting mixture was stirred at room temperature for 2-3 h. After completion of the reaction, the reaction solution was concentrated and separated by high pressure preparative chromatography to obtain Fmoc-Leu-AM-DXd as a light yellow solid.

Step 3: Synthesis of Compound Leu-AM-DXd

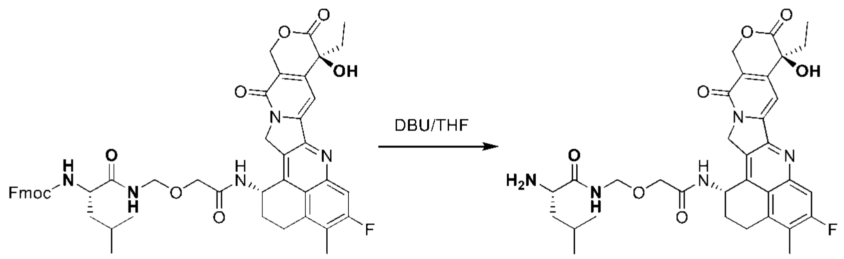


Leu-AM-DXd

Compound Fmoc-Leu-AM-D (1.0 eq) was dissolved in Tetrahydrofuran. DBU (1.0 eq) was added, and the mixture was stirred at room temperature for 2-3 h under nitrogen protection. TLC detection showed that the reaction of raw materials was completed. The reaction solution was redissolved with methanol, and the solution was filtered, and then subjected to high pressure liquid chromatography to obtain compound Leu-AM-DXd as a yellow solid product.

**Example 4 Quantification of DXD**

One method to test whether a drug conjugate is cleaved by Cathepsin involves adding Cathepsin enzyme to the drug conjugate and performing an enzymatic cleavage reaction, while simultaneously monitoring the content of each substance in the mixture to determine the rate and progress of the enzymatic cleavage reaction.

**Test conditions**

1.1 Preparation of reference solution:

About 25 mg of DXD reference substance was accurately weighed and placed in a 5 mL volumetric flask. DMSO was added to the scale mark and the mixture was mixed to prepare a 10 mM solution which was marked as the standard stock solution. The standard stock solution was taken and prepared into standard solutions with concentrations of 20nmol/mL, 10nmol/mL, 5nmol/mL, 2nmol/mL, 1nmol/mL, and 0.2nmol/mL. 10uL was taken and injected into HPLC, the peak area was recorded. The linear equation was calculated by using the peak area as the ordinate and the concentration as the abscissa.

1.2 chromatographic conditions:

chromatographic column: Agilent Eclipse Plus C18 5um, 4.6*250mm
Fluorescence detector: Em=380nm, Ex=460nm
Mobile phase A: 0.1%TFA/H2O B: ACN
Flow rate: 1ml/min

Injection volume: 10uL

Time gradient:

| Time (min) | A (%) | B (%) |
|---|---|---|
| 0 | 80 | 20 |
| 5 | 80 | 20 |
| 20 | 40 | 60 |
| 25 | 10 | 90 |
| 25.1 | 80 | 20 |
| 30 | 80 | 20 |

**Example 5 L-AM-DXD cannot be cleaved by cathepsin L.**

1. Preparation of experimental solution:

Assay Buffer: 50mM MES, 5mM DTT, 1mM EDTA, 0.005% (w/v) Brij-35, pH6.0.
Cathepsin L: 100 μg/mL in assay buffer, incubated on ice for 15 min.

Preparation of sample solution: An appropriate amount of L-AM-DXD was weighed and added with a certain amount of DMSO solution to make the concentration of the stock solution being 10 umol/mL. Then 10uL of the stock solution was taken and added with 80uL of DMSO to dilute to 1 umol/mL. Then pure water was added in a ratio of 1:4 to dilute the sample solution to a concentration of 0.2 umol/mL.

2. Enzymic cleavage reaction:

50uL of the sample solution was taken, added with 150uL of the incubated cathepsin L solution at a volume ratio of 1:3 (v:v), and digested at 37°C; another 50uL of the sample solution was taken, and added with 150uL of the assay buffer solution at a volume ratio of 1:3 (v:v). 30uL of the solutions were taken and added into the injection bottle at 0h, 2h, 4h, and 20h, respectively, and injected. The HPLC method is as described in 1.2.

3. Experimental results: The experimental results were as shown in Fig. 1.

4. Conclusions:

After 20 hours of action of cathepsin L on L-AM-DXD, the DXD produced (calculated according to the external standard method of the DXD standard curve) only accounted for 1.45% of the reaction substrate L-AM-DXD, and most of it was not removed. It can be concluded that L-AM-DXD cannot be cleaved by cathepsin L.

In Examples 6 to 13, GGFL-AM-DXD and MI-PEG6-GGFL-AM-DXD can be used interchangeably, and GGFG-AM-DXD and EMC-GGFG-AM-DXD can be used interchangeably.

**Example 6 Linker screening experiments revealed that MI-PEG6-GGFL-AM-DXD (S7) can be cleaved by cathepsin L, indicating that GGF could be recognized and cleaved by cathepsin L after being connected to L. Comparison found that the activation efficiency was better than that of EMC-GGFG-AM-DXD.**

1. Preparation of experimental solution:

Assay Buffer: 50mM MES, 5mM DTT, 1mM EDTA, 0.005% (w/v) Brij-35, pH6.0.
Cathepsin L: 100 μg/mL in assay buffer, incubated on ice for 15 min.

Preparation of sample solution: An appropriate amount of EMC-GGFG-AM-DXD (purchased from Shanghai

Haoyuan Pharmaceutical) and MI-PEG6-GGFL-AM-DXD were respectively weighed and added with a certain amount of DMSO solution to make the concentration of the stock solution thereof being 10 umol/mL. Then 10uL was taken and added with 80uL of DMSO to dilute to 1 umol/mL. Then pure water was added in a ratio of 1:4 to dilute the sample solution to a concentration of 0.2 umol/mL.

2. Enzymatic cleavage reaction:

50uL of the sample solution was taken, added with 150uL of the incubated cathepsin L solution at a volume ratio of 1:3 (v:v), and digested at 37°C; another 50uL of the sample solution was taken, added with 150uL of the assay buffer solution at a volume ratio of 1:3 (v:v). 30uL were taken and added into the injection bottle at 0h, 2h, 4h, and 20h, respectively, and injected. The HPLC method is as described in 1.2.

3. Experimental results: The results were as shown in Fig. 2 and 3.

4. Conclusions:

The DXD produced by MI-PEG6-GGFL-AM-DXD and EMC-GGFG-AM-DXD after 20 hours of action of cathepsin L were calculated (calculated according to the external standard method of DXD standard curve). The amount of DXD produced by enzymic cleavage of MI-PEG6-GGFL-AM-DXD accounted for 98.3% of the reaction substrate, that is, the enzymatic cleavage activation efficiency was 98.3%. The amount of DXD produced by enzymatic cleavage of EMC-GGFG-AM-DXD accounted for 27.8% of the reaction substrate, that is, the enzymatic cleavage activation efficiency was 27.8%. It can be seen that the enzymatic cleavage of MI-PEG6-GGFL-AM-DXD by cathepsin L was better than that of EMC-GGFG-AM-DXD, demonstrating that L-AM-DXD cannot be cleaved by cathepsin L, but can be cleaved by cathepsin L after connecting to GGF.

The results of this enzymatic cleavage experiment showed that the enzymatic cleavage efficiency of compounds S1-S34 in the present invention within 20 h reached more than 90%, which was significantly better than that of the control compound EMC-GGFG-AM-DXd. The specific data were as shown in Table F1:

Table F1

| Compound No. | Enzymatic cleavage efficiency % | Compound No. | Enzymatic cleavage efficiency % |
|---|---|---|---|
| S1 | 90.5 | S18 | 98.3 |
| S2 | 91.1 | S19 | 98.0 |
| S3 | 91.0 | S20 | 97.8 |
| S4 | 92.3 | S21 | 98.0 |
| S5 | 93.1 | S22 | 98.2 |
| S6 | 98.0 | S23 | 98.0 |
| S7 | 98.3 | S24 | 97.7 |
| S8 | 98.0 | S25 | 97.5 |
| S9 | 97.5 | S26 | 95.1 |
| S10 | 96.8 | S27 | 94.5 |
| S11 | 98.0 | S28 | 95.1 |
| S12 | 98.1 | S29 | 96.3 |
| S13 | 98.0 | S30 | 98.1 |
| S14 | 98.0 | S31 | 97.5 |
| S15 | 95.1 | S32 | 98.0 |
| S16 | 92.3 | S33 | 95.1 |
| S17 | 98.5 | S34 | 98.1 |

**Example 7 Linker screening experiments found that MI-PEG6-GGFL-AM-DXD could not be cleaved by cathepsin B, while EMC-GGFG-AM-DXD could be activated by cathepsin B, indicating that cathepsin L is the only**

**enzyme that activates MI-PEG6-GGFL-AM-DXD.**

1. Preparation of experimental solution:

Activation buffer: 25mM MES, 5mM DTT, pH 5.0
Assay buffer: 25 mM MES, pH 5.0.
Cathepsin B: 100 μg/mL in activation buffer, incubated at room temperature for 15 minutes

Preparation of sample solution: An appropriate amount of MI-PEG6-GGFL-AM-DXD and EMC-GGFG-AM-DXD were respectively weighed and added with a certain amount of DMSO solution to make the concentration of the stock solution thereof being 10 umol/mL. Then 10uL of the stock solution was respectively taken and added with 80uL of DMSO to dilute to 1 umol/mL. Then pure water was added in a ratio of 1:4 to dilute the sample solution to a concentration of 0.2 umol/mL.

2. Enzymatic cleavage reaction:

50uL of the sample solution was taken, added with 150uL of the incubated cathepsin B solution at a volume ratio of 1:3 (v:v), and digested at 37°C; another 50uL of the sample solution was taken, and added with 150uL of the assay buffer solution at a volume ratio of 1:3 (v:v). 30uL of the solutions were taken and added into the injection bottle at 0h, 2h, 4h, and 20h, respectively, and injected. The HPLC method is as described in 1.2 of Example 4.

3. Experimental results: The experimental results were as shown in Fig. 4 and Fig. 5.

4. Conclusions:

The DXD produced (calculated according to the external standard method of DXD standard curve) by MI-PEG6-GGFL-AM-DXD and EMC-GGFG-AM-DXD after 20 hours of action of cathepsin B were compared. The amount of DXD produced by enzymatic cleavage of MI-PEG6-GGFL-AM-DXD accounted for 3.75% of the reaction substrate. The amount of DXD produced by enzymatic cleavage of EMC-GGFG-AM-DXD accounted for 45.2% of the reaction substrate, and there was also a part of GFG-AM-DXD fragment. It can be seen that Cathepsin B was more sensitive to EMC-GGFG-AM-DXD, but insensitive to MI-PEG6-GGFL-AM-DXD, and cathepsin L was the only enzyme that activates MI-PEG6-GGFL-AM-DXD.

**Example 8 Stability test of MI-PEG6-GGFL-AM-DXD and EMC-GGFG-AM-DXD in human plasma. MI-PEG6-GGFL-AM-DXD is more stable than EMC-GGFG-AM-DXD.**

1. Preparation of experimental solution:

PBS buffer: pH = 7.2
Protein precipitation agent: DMSO/MEOH = 1:1

Preparation of sample solution: An appropriate amount of MI-PEG6-GGFL-AM-DXD and GGFG-AM-DXD were weighed and added with a certain amount of DMSO solution to make the concentration of the stock solution thereof being 10 umol/mL. Then 10uL of the stock solution was taken and added with 80uL of DMSO to dilute to 1 umol/mL. Then pure water was added in a ratio of 1:4 to dilute the sample solution to a concentration of 0.2 umol/mL.

2. Stability in human plasma:

An appropriate amount of sample was weighed and prepared into a 1 mM solution with DMSO. Human plasma was added at a volume ratio of 1:9 and the solution was incubated in a 37°C incubator. Samples were taken after 0, 2, 4, 6, and 20 h. DMSO/MEOH (1:1) was added at a volume ratio of 1:3 to remove protein. The solution was centrifuged at 12,000 rpm for 5 min and the supernatant was collected. The HPLC method was as described in 1.2 of Example 4.
3. Experimental results: The experimental results were as shown in Fig. 6 and 7.
4. Conclusions: The DXD produced by MI-PEG6-GGFL-AM-DXD and EMC-GGFG-AM-DXD after being placed in human plasma for 20 hours were calculated (calculated according to the external standard method of DXD standard

curve). The DXD produced by MI-PEG6-GGFL-AM-DXD (S7) and EMC-GGFG-AM-DXD accounted for 1.75% and 2.88% of the substrate, respectively, that is, the substrate contents were 98.3% and 97.1%, respectively, indicating that MI-PEG6-GGFL-AM-DXD has better stability.

The results of this plasma stability test showed that the substrate content of the compound of the present invention can reach more than 98% or even 100% within 20 hours (that is, the generated load was ≤98% or even no load is detected), indicating that the connection performance between the linker and the load exhibited good stability. wherein the load was a group of a drug having cytotoxicity or a drug carrying cytotoxicity such as DXD and the like. The specific data were as shown in Table F2:

Table F2

| Compound No. | Substrate content % | Generated load % | Compound No. | Substrate content % | Generated load % |
|---|---|---|---|---|---|
| S1 | 100 | 0.0% | S18 | 98.2 | 1.8% |
| S2 | 100 | 0.0% | S19 | 98.0 | 2.0% |
| S3 | 100 | 0.0% | S20 | 98.3 | 1.7% |
| S4 | NT | NT | S21 | 98.2 | 1.8% |
| S5 | 100 | 0.0% | S22 | 98.3 | 1.7% |
| S6 | 98.0 | 2.0% | S23 | 98.1 | 1.9% |
| S7 | 98.3 | 1.7% | S24 | NT | NT |
| S8 | 98.0 | 2.0% | S25 | NT | NT |
| S9 | 98.5 | 1.5% | S26 | 100 | 0.0% |
| S10 | 98.8 | 1.2% | S27 | 100 | 0.0% |
| S11 | NT | NT | S28 | 100 | 0.0% |
| S12 | 98.1 | 1.9% | S29 | 100 | 0.0% |
| S13 | 98.0 | 2.0% | S30 | 98.2 | 1.8% |
| S14 | 98.0 | 2.0% | S31 | NT | NT |
| S15 | NT | NT | S32 | 100 | 0.0% |
| S16 | NT | NT | S33 | 100 | 0.0% |
| S17 | 98.5 | 1.5% | S34 | 98.3 | 1.7% |

**Example 9 MI-PEG6-GGFL-AM-DXD has high stability in Monkey heart homogenate and is superior to GGFG-AM-DXD.**

1. Preparation of experimental solution:

Assay buffer: 25 mM MES, pH 5.0.
Protein precipitation agent: DMSO/MEOH = 1:1

Preparation of sample solution: An appropriate amount of MI-PEG6-GGFL-AM-DXD and EMC-GGFG-AM-DXD were weighed and added with a certain amount of DMSO solution to make the concentration of the stock solution thereof being 10 umol/mL. Then 10uL of the stock solution were taken and added with 80uL of DMSO to dilute to 1 umol/mL. Then pure water was added in a ratio of 1:4 to dilute the sample solution to a concentration of 0.2 umol/mL.

Preparation of heart homogenate:

An appropriate amount of fast frozen heart by liquid nitrogen was placed in a 4 mL EP tube and weighed. Assay buffer with pH 5.0 was added at a mass-to-volume ratio of 1:5. The tissue was homogenized using a tissue grinder for 3 min. The heart homogenate was then centrifuged at 12,000 rpm for 5 min. The supernatant was collected and stored in a

-40°C refrigerator for later use.

2. Stability of heart homogenate:

The sample solution and the heart homogenate were respectively taken at a volume ratio of 1:9 and shaken thoroughly. Another sample solution and buffer solution (pH 5.0) were also shaken thoroughly at a volume ratio of 1:9 as a blank control solution. The solution was placed in a 37°C constant temperature water bath. 30uL was taken at 0h, 2h, 4h, and 20h, and added with 90uL of protein precipitation agent and shaken thoroughly. The resulting solution was centrifuged at 12000 rpm for 5 min, and the supernatant was taken and added into an injection bottle and injected. The HPLC method was as described in 1.2 of Example 4.

3. Experimental results: The experimental results were as shown in Fig. 8 and 9.

4. Conclusions:

The DXD produced by MI-PEG6-GGFL-AM-DXD and EMC-GGFG-AM-DXD after being placed in monkey heart homogenate for 20 hours were calculated (calculated according to the external standard method of DXD standard curve). The DXD produced by MI-PEG6-GGFL-AM-DXD and EMC-GGFG-AM-DXD accounted for 0% and 7.41% of the substrate, respectively. It can be seen that MI-PEG6-GGFL-AM-DXD did not release in the heart homogenate, while a part of EMC-GGFG-AM-DXD released, indicating that MI-PEG6-GGFL-AM-DXD has no cardiotoxicity.

**Example 10 MI-PEG6-GGFL-AM-DXD has high stability in monkey liver homogenate and is superior to EMC-GGFG-AM-DXD.**

1. Preparation of experimental solution:

Assay buffer: 25 mM MES, pH 5.0.
Protein precipitation agent: DMSO/MEOH = 1:1

Preparation of sample solution: An appropriate amount of MI-PEG6-GGFL-AM-DXD and EMC-GGFG-AM-DXD were weighed and added with a certain amount of DMSO solution to make the concentration of the stock solution thereof being 10 umol/mL. Then 10uL of the stock solution were taken and added with 80uL of DMSO to dilute to 1 umol/mL. Then pure water was added in a ratio of 1:4 to dilute the sample solution to a concentration of 0.2 umol/mL.

Preparation of liver homogenate:

An appropriate amount of fast frozen liver by liquid nitrogen was placed in a 4 mL EP tube and weighed. Assay buffer with pH 5.0 was added at a mass-to-volume ratio of 1:5. The tissue was homogenized using a tissue grinder for 3 min. The liver homogenate was then centrifuged at 12,000 rpm for 5 min. The supernatant was collected and stored in a -40°C refrigerator for later use.

2. Stability of liver homogenate:

The sample solution and the liver homogenate were respectively taken at a volume ratio of 1:9 and shaken thoroughly. Another sample solution and buffer solution (pH 5.0) were also shaken thoroughly at a volume ratio of 1:9 as a blank control solution. The solution was placed in a 37°C constant temperature water bath. 30uL was taken at 0h, 2h, 4h, and 20h, and added with 90uL of protein precipitation agent and shaken thoroughly. The solution was centrifuged at 12000 rpm for 5 min, and the supernatant was taken and added into an injection bottle and injected. The HPLC method was as described in 1.2 of Example 4.

3. Experimental results: The experimental results were as shown in Fig. 10 and Fig. 11.

4. Conclusions: After MI-PEG6-GGFL-AM-DXD and EMC-GGFG-AM-DXD being placed in liver homogenate for 20 hours, the amount of DXD produced by EMC-GGFG-AM-DXD accounted for 27.6% of the substrate, and the amount of DXD produced by MI-PEG6-GGFL-AM-DXD accounted for 5.7% of the substrate, indicating that GGFL-AM-DXD was more stable than GGFG-AM-DXD in liver homogenate.

**Example 11 GGFL-AM-DXD has a high activation efficiency in human gastric cancer tumor homogenate and is better than GGFG-AM-DXD.**

1. Preparation of experimental solution:

Assay buffer: 25 mM MES, pH 5.0.
Protein precipitation agent: DMSO/MEOH = 1:1

Preparation of sample solution: An appropriate amount of MI-PEG6-GGFL-AM-DXD and GGFG-AM-DXD were weighed and added with a certain amount of DMSO solution to make the concentration of the stock solution thereof being 10 umol/mL. Then 10uL were taken and added with 80uL of DMSO to dilute to 1 umol/mL. Then pure water was added in a ratio of 1:4 to dilute the sample solution to a concentration of 0.2 umol/mL.

Preparation of tumor homogenate 1:

An appropriate amount of fast-frozen tumor by liquid nitrogen was placed in a 4 mL EP tube and weighed. Assay buffer with pH 5.0 was added at a mass-to-volume ratio of 1:5. The tissue was homogenized using a tissue grinder for 3 min. The tumor homogenate was then centrifuged at 12,000 rpm for 5 min. The supernatant was collected and stored in a -40°C refrigerator for later use.

2. Activation of tumor homogenate 1:

The sample solution and the tumor homogenate 1 were respectively taken at a volume ratio of 1:9 and shaken thoroughly. Another sample solution and buffer solution (pH 5.0) were also shaken thoroughly at a volume ratio of 1:9 as a blank control solution. The solution was placed in a 37°C constant temperature water bath. 30uL was taken at 0h, 2h, 4h, and 20h, and added with 90uL of protein precipitation agent and shaken thoroughly. The solution was centrifuged at 12000 rpm for 5 min, and the supernatant was taken and added into an injection bottle and injected. The HPLC method was as described in 1.2 of Example 4.

3. Experimental results: The experimental results were as shown in Fig. 12 and 13.

4. Conclusions:

After GGFL-AM-DXD and GGFG-AM-DXD being placed in tumor homogenate 1 for a certain period of time, the amount of DXD produced by GGFG-AM-DXD at 48 hours accounted for 29.1% of the substrate, the amount of DXD produced by GGFL-AM-DXD reached 94.2% after 20 hours, showing that GGFL-AM-DXD released more readily in tumor homogenate 1 than GGFG-AM-DXD.

**Example 12 GGFL-AM-DXD has a high activation efficiency in CT26 tumor homogenate and is better than GGFG-AM-DXD.**

1. Preparation of experimental solution:

Assay buffer: 25 mM MES, pH 5.0.
Protein precipitation agent: DMSO/MEOH = 1:1

Preparation of sample solution: An appropriate amount of MI-PEG6-GGFL-AM-DXD and EMC-GGFG-AM-DXD were weighed and added with a certain amount of DMSO solution to make the concentration of the stock solution thereof being 10 umol/mL. Then 10uL were taken and added with 80uL of DMSO to dilute to 1 umol/mL. Then pure water was added in a ratio of 1:4 to dilute the sample solution to a concentration of 0.2 umol/mL.

Preparation of tumor homogenate 2:

An appropriate amount of fast-frozen tumor by liquid nitrogen was placed in a 4 mL EP tube and weighed. Assay buffer with pH 5.0 was added at a mass-to-volume ratio of 1:5. The tissue was homogenized using a tissue grinder for 3 min. The tumor homogenate was then centrifuged at 12,000 rpm for 5 min. The supernatant was collected and stored in a -40°C refrigerator for later use.

2. Activation of tumor homogenate 2:

The sample solution and the tumor homogenate 2 were respectively taken at a volume ratio of 1:9 and shaken thoroughly. Another sample solution and buffer solution (pH 5.0) were also shaken thoroughly at a volume ratio of 1:9 as a blank control solution. The solution was placed in a 37°C constant temperature water bath. 30uL was taken at 0h, 2h, 4h, and 20h, and added with 90uL of protein precipitation agent and shaken thoroughly. The solution was centrifuged at 12000 rpm for 5 min, and the supernatant was taken and added into an injection bottle and injected. The HPLC method was as described in 1.2 of Example 4.

3. Experimental results: The experimental results were as shown in Fig. 14 and Fig. 15.

4. Conclusions: After GGFL-AM-DXD and GGFG-AM-DXD being placed in tumor homogenate 2 for a certain period of time, the amount of DXD produced by GGFG-AM-DXD at 20 hours accounted for 42.5% of the substrate, and the amount of DXD produced by GGFL-AM-DXD reached 98.7% within 20 hours, showing that GGFL-AM-DXD released more readily in tumor homogenate 2 than GGFG-AM-DXD.

## Example 13 Antibody Drug conjugate

### 1. General Preparation Methods for Antibody-Drug Conjugate

The antibody targets were selected from the following: HER2, CD19, CD20, EGFR, CD22, CD3, TROP2, Glycoprotein NMB, Guanylyl cyclase C, CEA, AXL, GCC, CD79b, PSMA, ENPP3, Mesothelin, CD138, NaPi2b, CD56, CD74, FOLR1, DLL3, CEACAM5, CD142, SLAMF7, CD25, SLTRK6, CD37, CD70, AGS-22, C4.4A, FGFR2, Ly6E, MUC16, BCMA, pCadherin, Ephrin-A, LAMP1, MUC1, PDL1, HER2, NY-ESO-1, BCMA, WT1, MUC1, CD20, CD23, ROR1, CD123, CD33, CD44v6, CD174, CD30, CD133, cMet, FAP, EphA2, GD2, GPC3, IL-13Ra2, LewisY, SS1, CD171, EGFR, EGFRvIII, VEGFR2, NY-ESO-1, MUC-1, and MAGE-A3.

TCEP (tris(2-carboxyethyl)phosphine hydrochloride, 40 e.q.) was added to any of the above antibodies for reduction for 8 hours, and any of the compounds S1-S25 prepared in Examples 1-3 (40 e.q.) was added and reacted for 16 hours to form the corresponding antibody-drug conjugate.

### 2. *In vivo* therapeutic effects of antibody-drug conjugates on cancer tumors

According to the aforementioned general method, MI-PEG6-GGFL-AM-DXD and MI-PEG6-GGFG-AM-DXD were additionally coupled to HER2 antibody, and then its treatment in gastric cancer tumor homogenate was compared.

The *in vivo* tumor treatment experimental method was as follows:

Human gastric cancer cell line NCI-N87 was subcutaneously transplanted into NCG mice to construct an *in vivo* model.

NCI-N87 cells were cultured according to the culture conditions, and the cells were collected at the exponential growth phase and counted. Each mouse was inoculated on the right flank with $5\times10^6$ human gastric cancer cell NCI-N87 (suspended in 0.1 ml basal culture medium). After inoculation, mice were randomly divided into groups based on body weight and tumor volume when tumors grew to 70-150 $mm^3$ (8 days after tumor implantation). The study was divided into the following groups:

PBS group,
HER2-GGFG-DXD (5 mg/kg) group,
HER2-GGFL-DXD (5 mg/kg) group.

Treatment started on day 1. Twice a week until day 25, mice were weighed and tumor volumes were measured. The formula for calculating was:

$$\text{Tumor volume} = 1/2 \times \text{length} \times \text{width} \times \text{width}\ (mm^3).$$

$$\text{Tumor inhibition rate TGI}\ (\%) = 100\% - (Tt - T0) / (Ct - C0) \times 100\%;$$

$$\text{Anti-tumor rate}\ T/C(\%)=(Tt/T0)/(Ct/C0)\times100\%.$$

T0 is the tumor volume of the experimental antibody group during different dosing periods;
Tt is the tumor volume of the experimental antibody group during each measurement period; C0 is the tumor volume of the PBS group during cage dosing; CT is the mean tumor volume of the PBS group at each measurement period.

The experimental results, as shown in Figure 16, showed that the anti-HER2 antibody-coupled Dxd drug candidate molecule based on the GGFL linker has a better curative effect on gastric cancer tumors than the GGFG linker, and has a good inhibitory effect, with 5 of the 6 mice cured, while only 2 of the mice with the GGFG linker cured.

**Example 14: drug efficacy study of Drug Conjugates S7 (MI-PEG6-GGFL-AM-DXd), S8, S10, S18, S19, S23, and DXd at the same dose on Human Fibrosarcoma**

1. Animals: Nude mice, 6-8 weeks old, all female (Shanghai SLAC Laboratory Animal Co., Ltd.).

2. Generation of Tumor Models

1) The corresponding cells were purchased from American Type Culture Collection (ATCC) and authenticated according to the instructions provided by ATCC. The cells were cultured in Dulbecco's modified Eagle's medium (DMEM for short) containing 10% fetal bovine serum at 37°C and 5% carbon dioxide. Cells were passaged every 3 days and used within 15 generations.

2) Tumor generation: $5\times10^6$ corresponding cells were subcutaneously injected into the back of the nude mice. When the tumor grew to about 100 $mm^3$, the mice were randomly divided into groups and the treatment began. The first day was counted from the day treatment began.

3) Treatment Course

A dose of 1/10 MTD (5 μmol/kg) was used, administered once a week for 3 weeks.

4) Grouping and outcome measures were as shown in Table 4.

Table 4: Effects of the corresponding compounds and the control group on tumor suppression

| Group | Number of animals | Tumor volume($mm^3$) | Anti-tumor rate% |
|---|---|---|---|
| | | Day 20 | Day 20 |
| Saline (blank group) | 6 | Over 1000 | 0 |
| DXd (Control Group) | 6 | 200 | 80% |
| S7 (MI-PEG6-GGFL-AM-DXd) | 6 | 20 | 98% |
| S8 | 6 | 15 | 98% |
| S10 | 6 | 25 | 98% |
| S18 | 6 | 40 | 95% |
| S19 | 6 | 30 | 95% |
| S23 | 6 | 35 | 95% |

5) Results and Discussion: Compared with DXd, S7 (MI-PEG6-GGFL-AM-DXd), S8, S10, S18, S19, and S23 exhibited significantly improved treatment efficacy and can almost achieve the effect of curing tumors.

**Example 15 Antibody glycosylation site drug conjugate**

The antibody targets were selected from the following: HER2, CD19, CD20, EGFR, CD22, CD3, TROP2, Glycoprotein NMB, Guanylyl cyclase C, CEA, AXL, GCC, CD79b, PSMA, ENPP3, Mesothelin, CD138, NaPi2b, CD56, CD74, FOLR1, DLL3, CEACAM5, CD142, SLAMF7, CD25, SLTRK6, CD37, CD70, AGS-22, C4.4A, FGFR2, Ly6E, MUC16, BCMA, pCadherin, Ephrin-A, LAMP1, MUC1, PDL1, HER2, NY-ESO-1, BCMA, WT1, MUC1, CD20, CD23, ROR1, CD123, CD33, CD44v6, CD174, CD30, CD133, cMet, FAP, EphA2, GD2, GPC3, IL-13Ra2, LewisY, SS1, CD171, EGFR, EGFRvIII, VEGFR2, NY-ESO-1, MUC-1, and MAGE-A3.

β1,4-galactosidase was added to any of the above antibodies and incubated for 24 h. β-1,4-galactosyltransferase and UDP-C2 keto-Gal were added to the purified Protein A antibody and incubated for 48 h. The medium was changed to 50 mM NaOAc (pH 4.5), and Aminoo-azide (10 e.q.) was added and incubated for 8 h. After filtration, the above-mentioned linker-payload containing an alkyne group (10 e.q.) was added and reacted for 4 h.

All documents mentioned in the present invention are cited as references in this application, just as each document is individually cited as a reference. In addition, it should be understood that, after reading the above teaching content of the present invention, those skilled in the art can make various changes or modifications to the present invention, and these equivalent forms also fall within the scope defined by the appended claims of the present application.

## Claims

**1.** A conjugate or a stereoisomer thereof, or a pharmaceutically acceptable salt thereof, wherein the conjugate is of formula (II);

R1-L-R2 formula (II);

wherein

L is a linker (-GGFL-) as shown in formula (I);

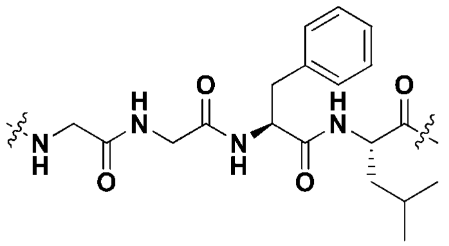

formula (I);

R1 is a group having one or more functions selected from the group consisting of:

activation of the tumor microenvironment, improvment of the water solubility of tumor-targeting drugs, and providing a coupling site to an antibody element;
R2 is a group derived from a drug having cytotoxicity or a drug carrying cytotoxicity.

**2.** The conjugate of claim 1, wherein

the conjugate is selected from the following Table B1:

Table B1

| | |
|---|---|
| 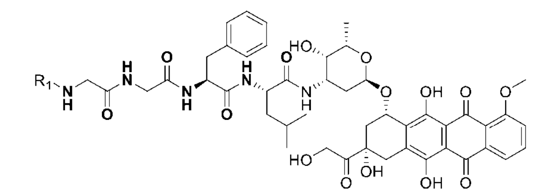 | 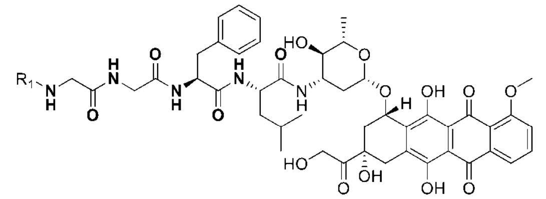 |
| 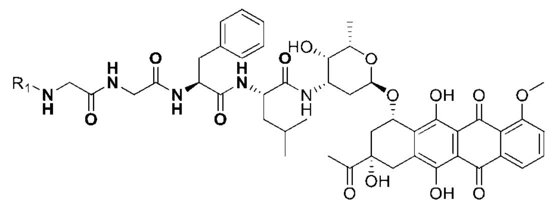 | 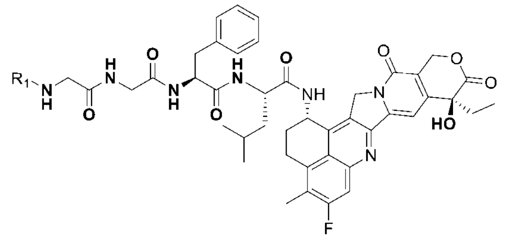 |
| 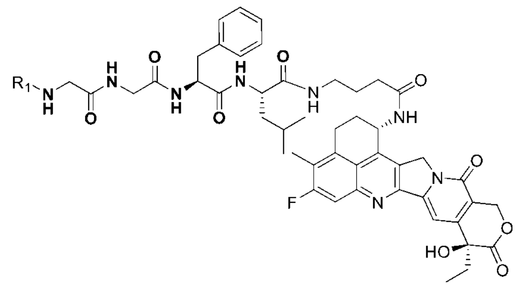 | 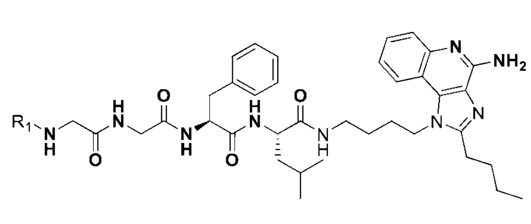 |

| | |
|---|---|
| 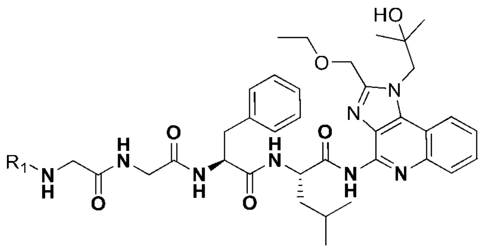 | 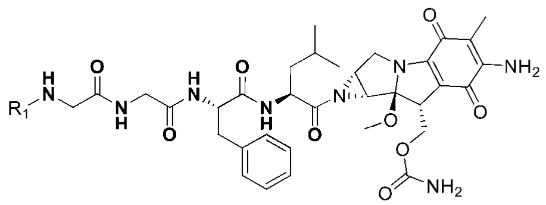 |
| 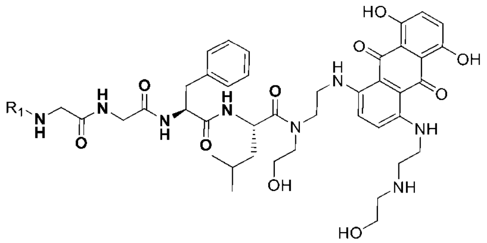 | 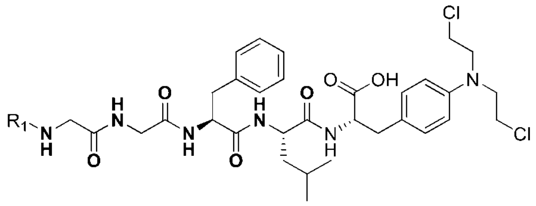 |
| 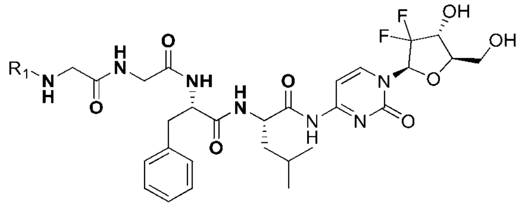 | |
| 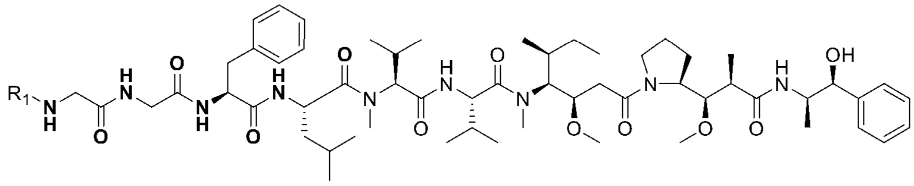 | |
| 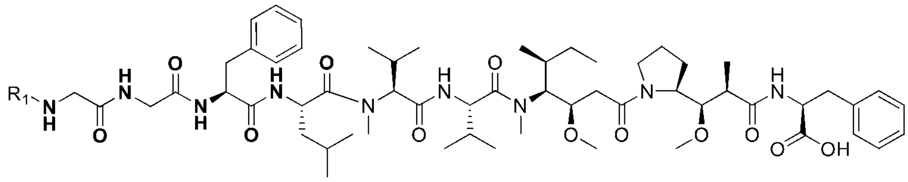 | |
| 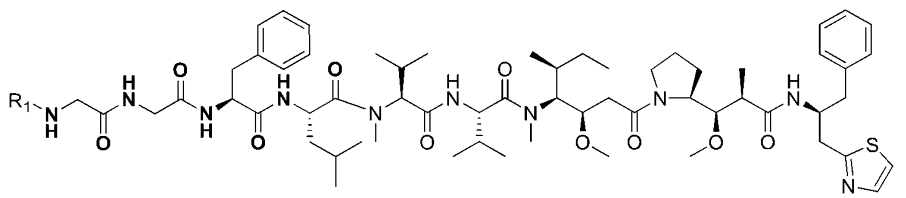 | |

or, the conjugate is selected from the following Table B2:

Table B2

| | |
|---|---|
| 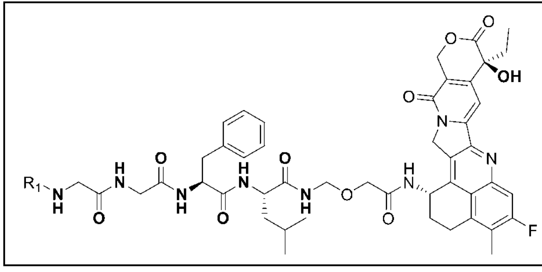 | 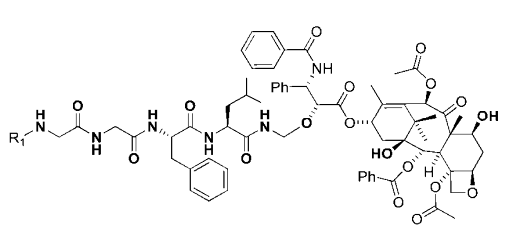 |

| | |
|---|---|
| 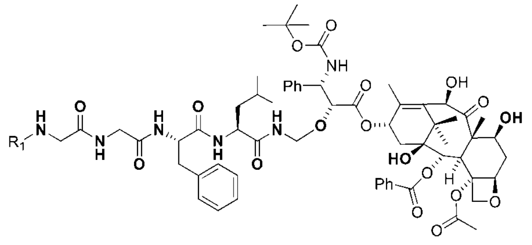 | 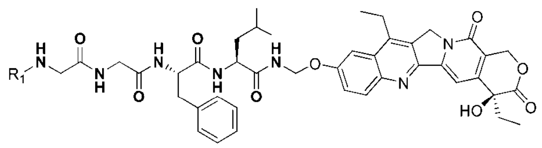 |
| 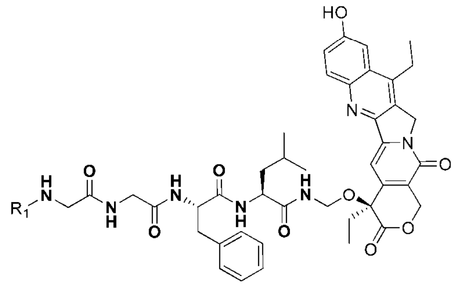 | 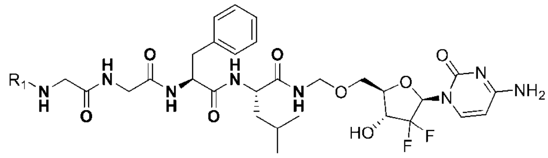 |
| 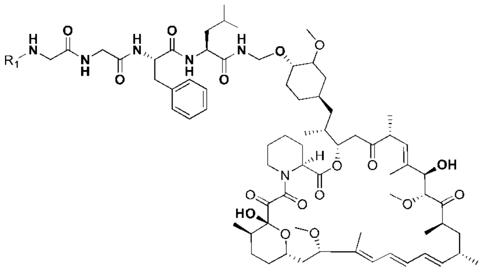 | 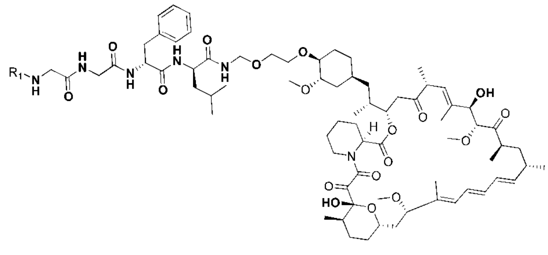 |

**3.** The conjugate of claim 1, wherein

(i) providing a coupling site to an antibody means including a reactive group that can be coupled to an antibody in R1, and the reactive group that can be coupled to antibodies is selected from the group consisting of:

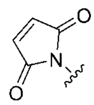 , 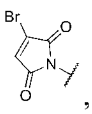 , 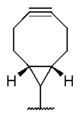 , 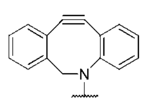 , and 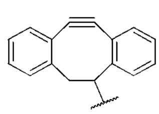 ; and/or

(ii) the activating group in the tumor microenvironment is selected from the group consisting of:

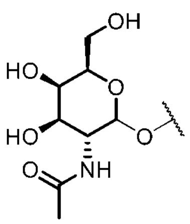

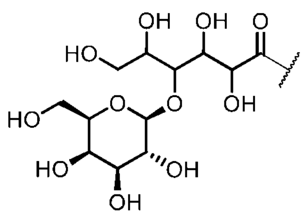

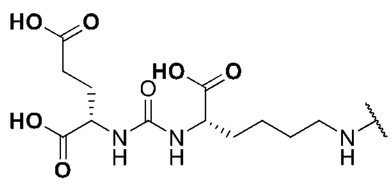

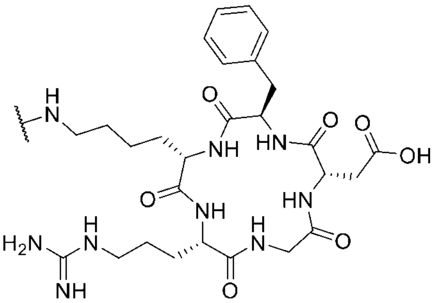 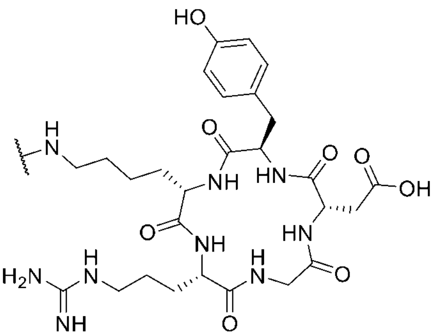 .

**4.** The conjugate of claim 1, wherein

R1 is selected from the following Table C1-A and C1-B:

Table C1-A

| $R_{1-1}$ | $R_{1-7}$ | $R_{1-8}$ |
|---|---|---|
| 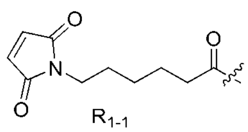 $R_{1-1}$ | 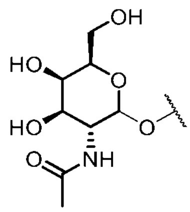 $R_{1-7}$ | 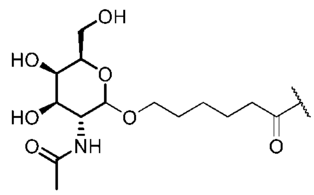 $R_{1-8}$ |
| $R_{1-9}$ | $R_{1-10}$ | $R_{1-11}$ |
| 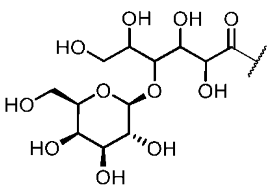 $R_{1-9}$ | 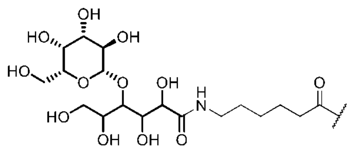 $R_{1-10}$ | 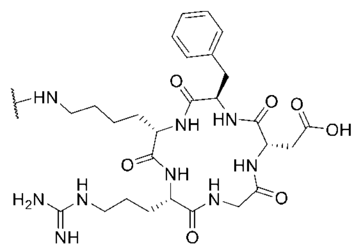 $R_{1-11}$ |
| $R_{1-12}$ | $R_{1-13}$ | |
| 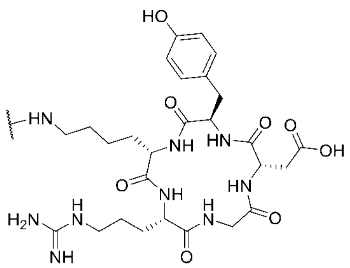 $R_{1-12}$ | 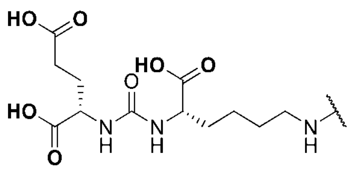 $R_{1-13}$ | |
| $R_{1-25}$ | $R_{1-29}$ | $R_{1-37}$ |

(continued)

| | | |
|---|---|---|
| 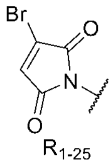 R1-25 | 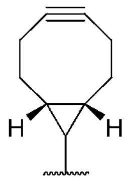 R1-29 | 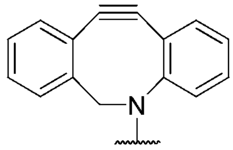 R1-37 |
| R1-42 | R1-43 | R1-45 |
| 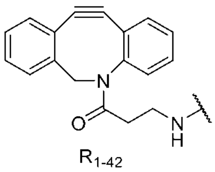 R1-42 | 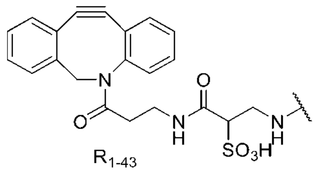 R1-43 | 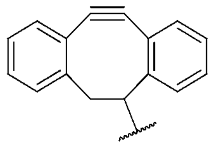 R1-45 |
| R1-44 | | |
| 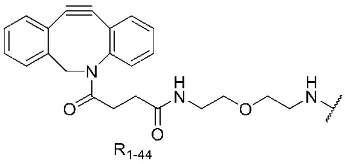 R1-44 | | |

Table C1-B

| | | |
|---|---|---|
| $R_{1-2}$ | $R_{1-3}$ | $R_{1-4}$ |
| 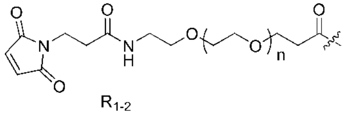 $R_{1-2}$ | 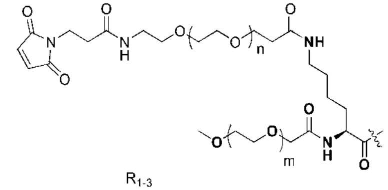 $R_{1-3}$ | 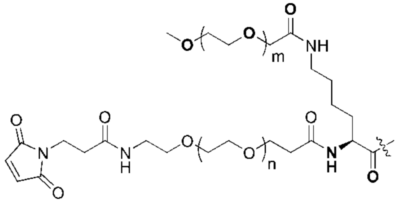 $R_{1-4}$ |
| $R_{1-5}$ | $R_{1-6}$ | |
| 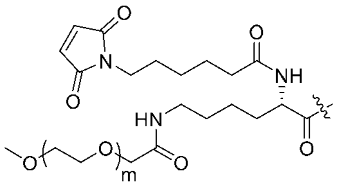 $R_{1-5}$ | 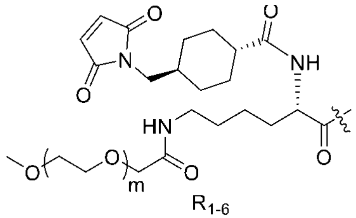 $R_{1-6}$ | |
| $R_{1-26}$ | $R_{1-27}$ | $R_{1-28}$ |
| 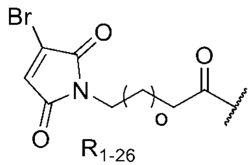 $R_{1-26}$ | 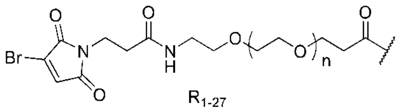 $R_{1-27}$ | 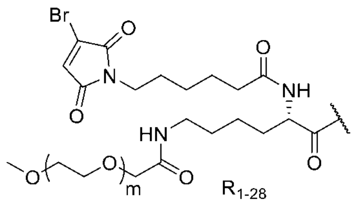 $R_{1-28}$ |
| $R_{1-30}$ | $R_{1-31}$ | $R_{1-32}$ |
| 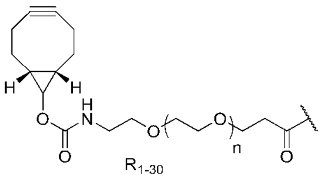 $R_{1-30}$ | 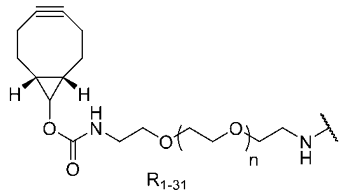 $R_{1-31}$ | 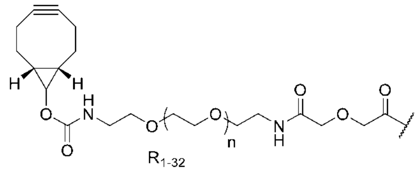 $R_{1-32}$ |
| $R_{1-33}$ | $R_{1-34}$ | $R_{1-35}$ |

(continued)

| | | |
|---|---|---|
| 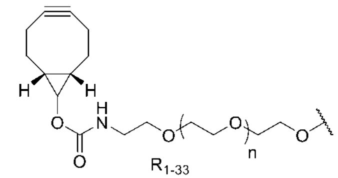 R1-33 | 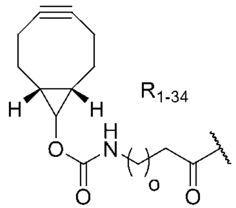 R1-34 | 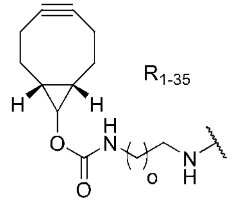 R1-35 |
| R1-36 | | |
| 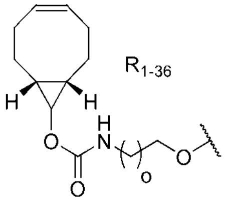 R1-36 | | |
| R1-38 | R1-39 | R1-40 |
| 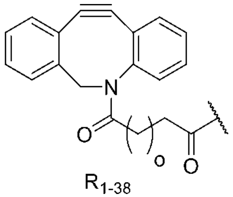 R1-38 | 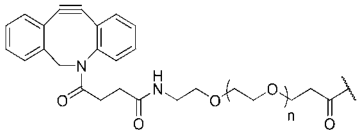 R1-39 | 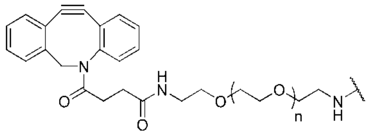 R1-40 |
| R1-41 | | |
| 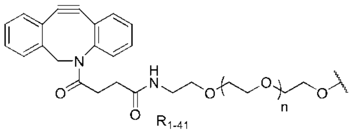 R1-41 | | |

In Table C1-B, o is 0, or an integer from 1 to 20; m and n are each independently 0, or an integer from 0 to 20; or,

R1 is selected from the following Table C2-A and C2-B:

Table C2-A

| | |
|---|---|
| $R_{1-17}$ | 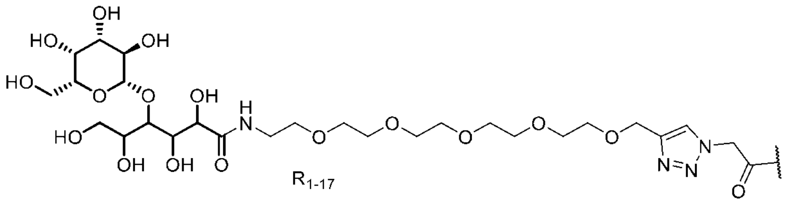 $R_{1-17}$ |
| $R_{1-18}$ | 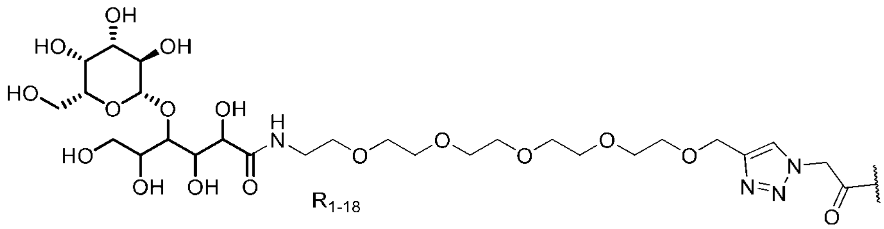 $R_{1-18}$ |

Table C2-B

| | |
|---|---|
| $R_{1-14}$ | 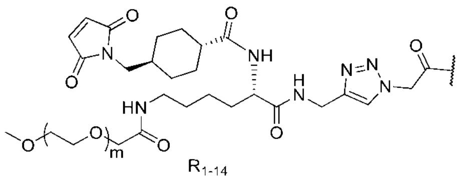 $R_{1-14}$ |
| $R_{1-15}$ | 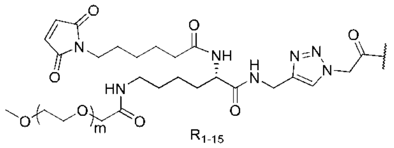 $R_{1-15}$ |
| $R_{1-16}$ | 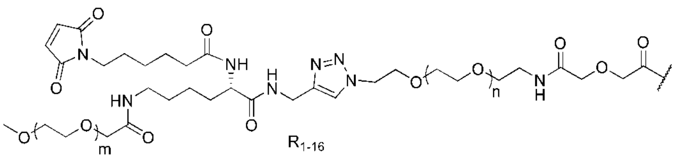 $R_{1-16}$ |
| $R_{1-19}$ | 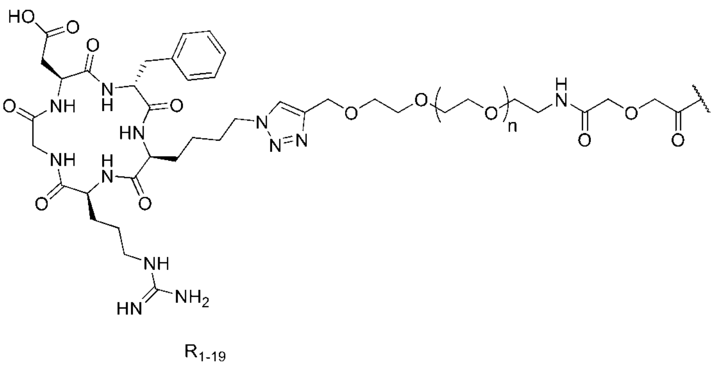 $R_{1-19}$ |

(continued)

| | |
|---|---|
| $R_{1-20}$ | 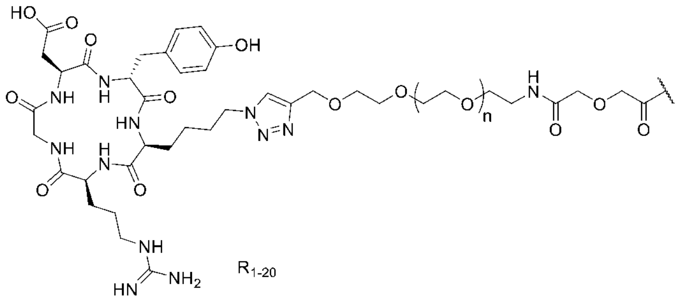 |
| $R_{1-21}$ | 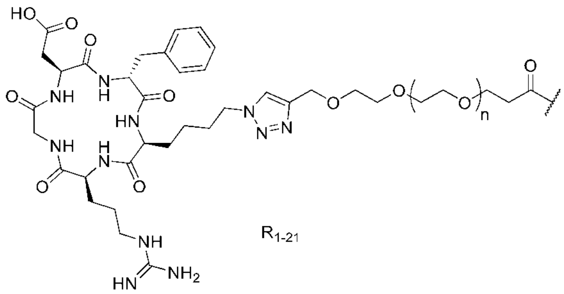 $R_{1-21}$ |
| $R_{1-22}$ | 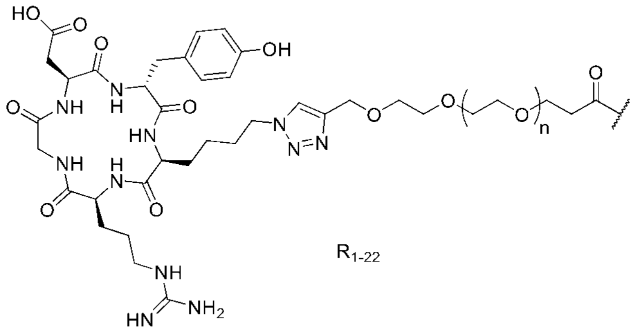 $R_{1-22}$ |
| $R_{1-23}$ | 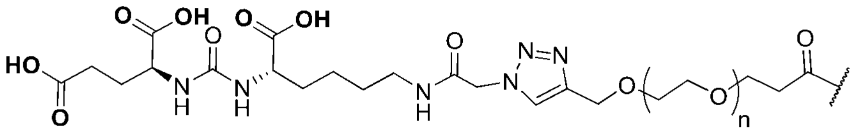 $R_{1-23}$ |
| $R_{1-24}$ | 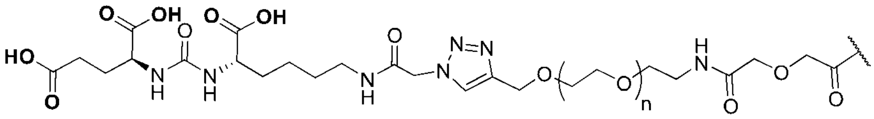 $R_{1-24}$ |

in Table C2-B, m and n are each independently 0, or an integer from 0 to 20.

**5.** The conjugate of claim 1 or the stereoisomer thereof or the pharmaceutically acceptable salt thereof, wherein the conjugate is selected from Table A1, Table A2 and Table A3:

Table A1

| | |
|---|---|
| S1 | 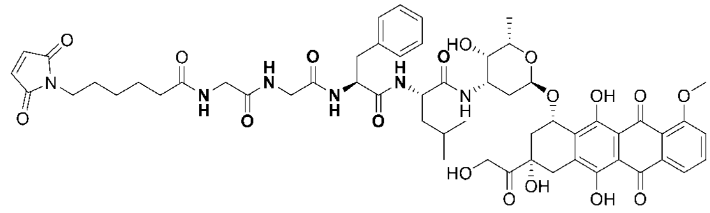 |
| S2 | 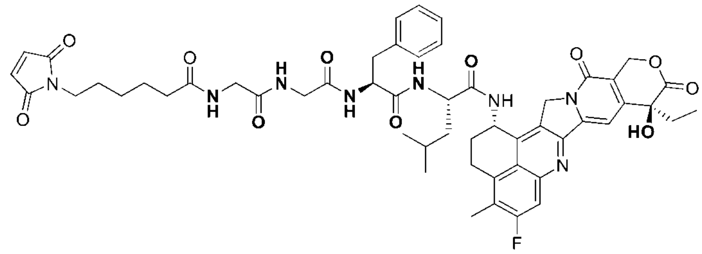 |
| S3 | 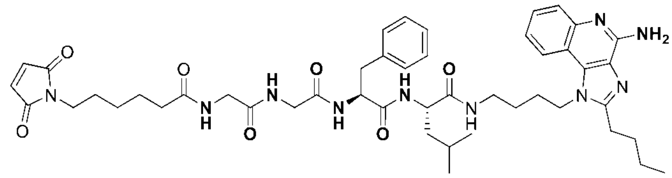 |
| S4 | 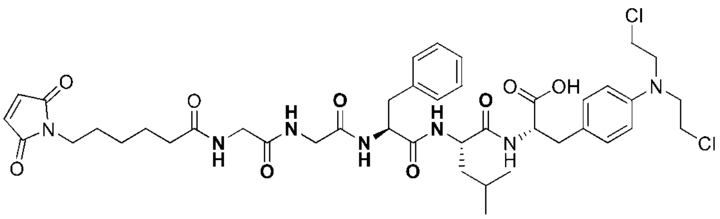 |
| S5 | 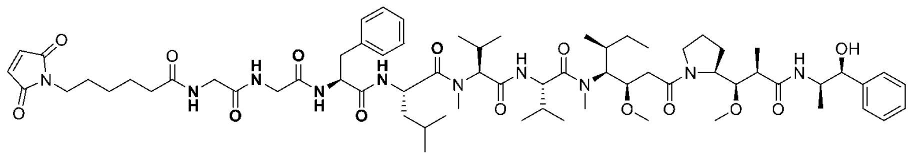 |
| S6 | 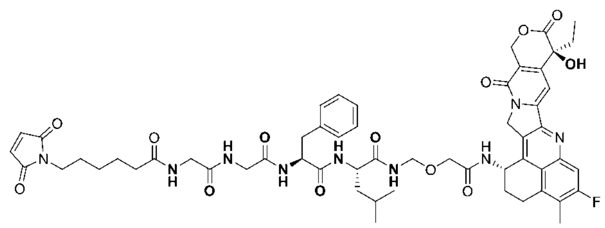 |
| S7 | 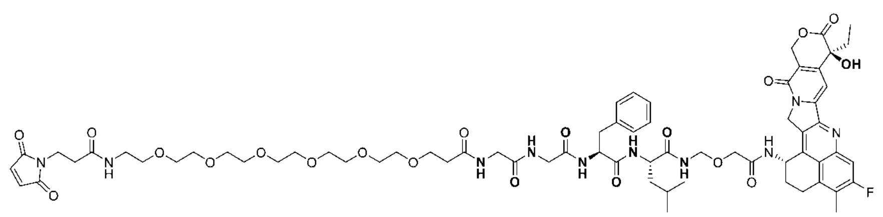 |

(continued)

| | |
|---|---|
| S8 | 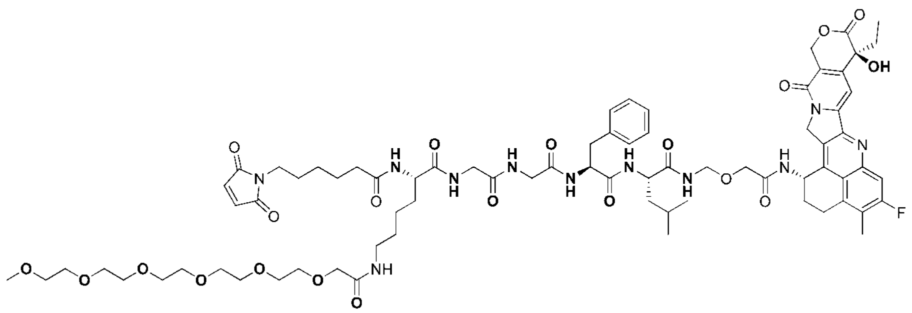 |
| S9 | 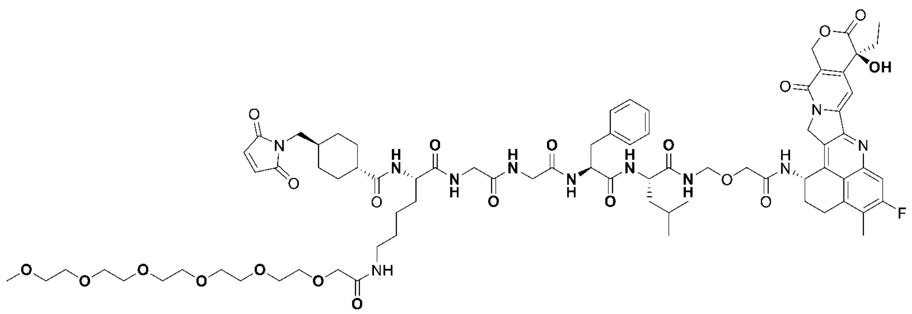 |
| S10 | 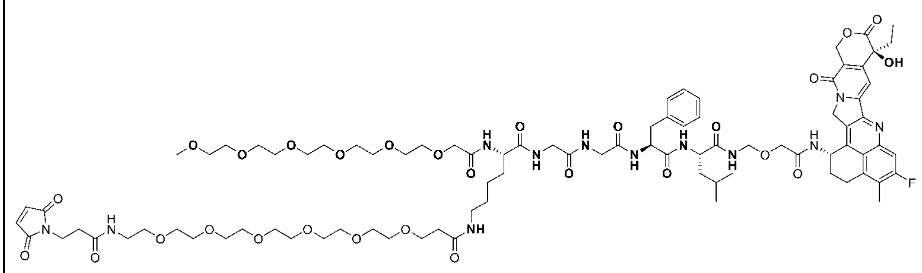 |
| S11 | 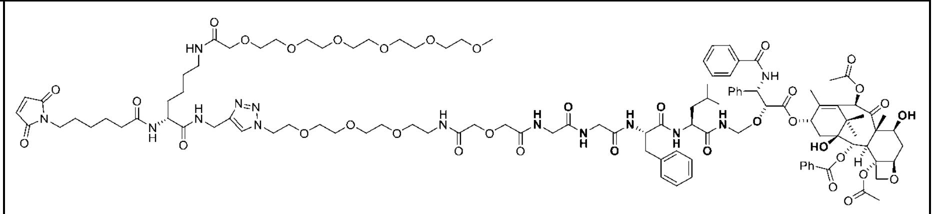 |
| S12 | 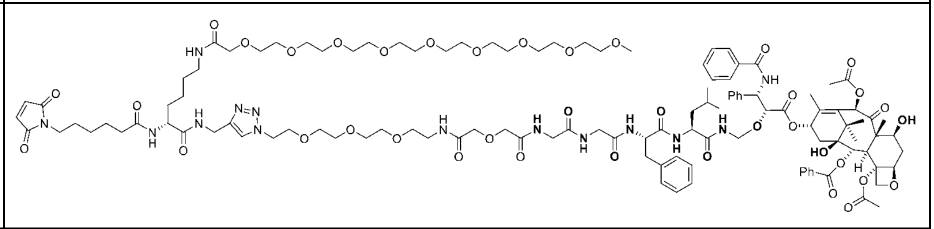 |
| S13 | 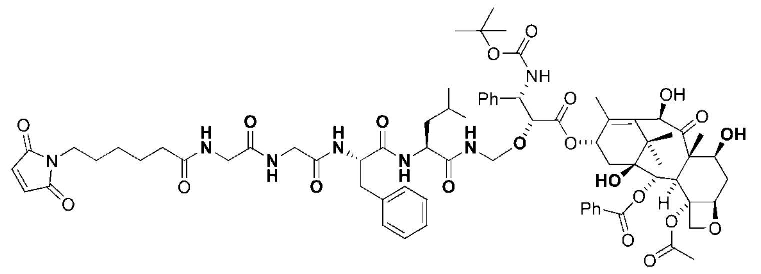 |

(continued)

| | |
|---|---|
| S14 | 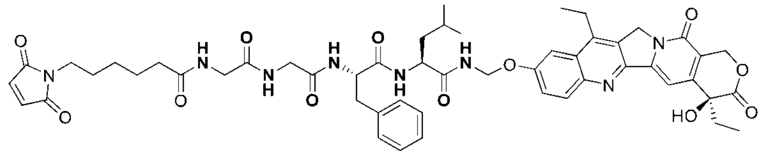 |
| S15 | 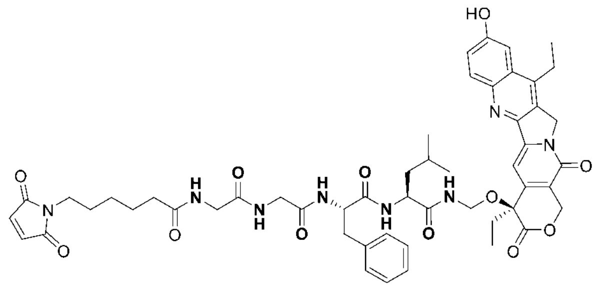 |
| S16 | 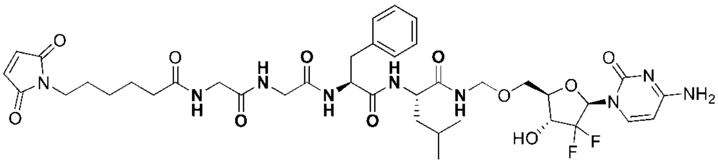 |
| S17 | 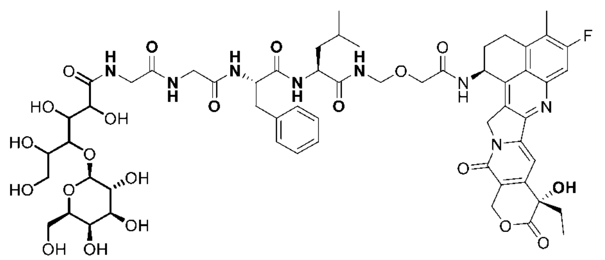 |
| S18 | 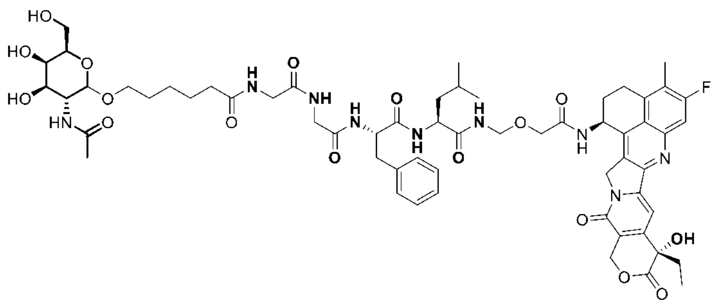 |
| S19 | 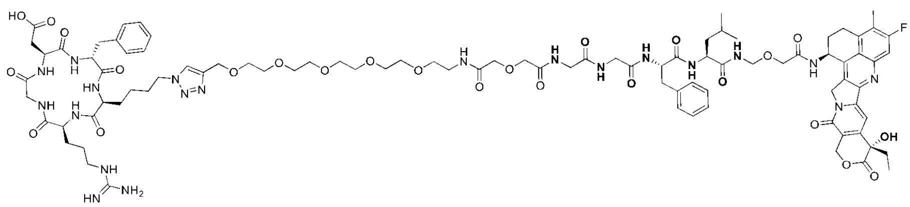 |
| S20 | 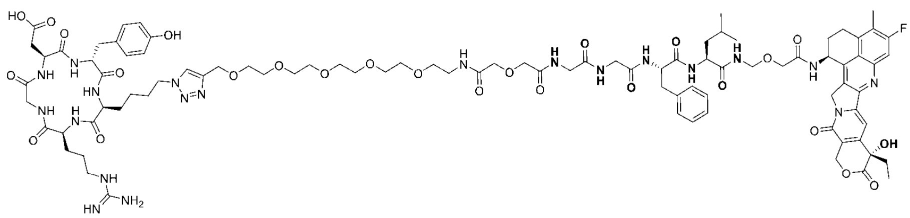 |

(continued)

| | |
|---|---|
| S21 | 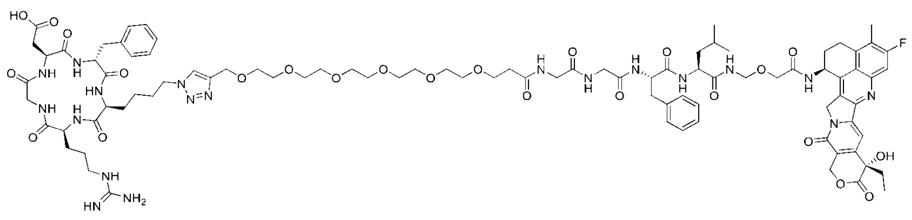 |
| S22 | 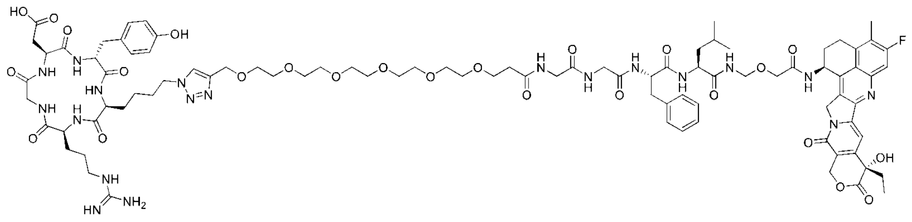 |
| S23 | 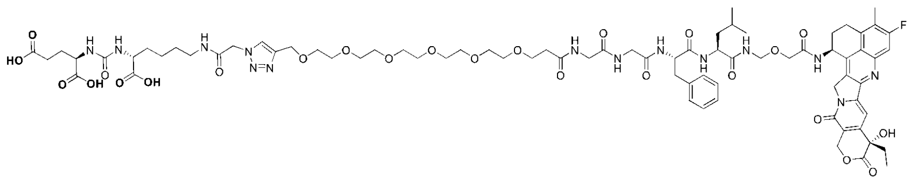 |
| S24 | 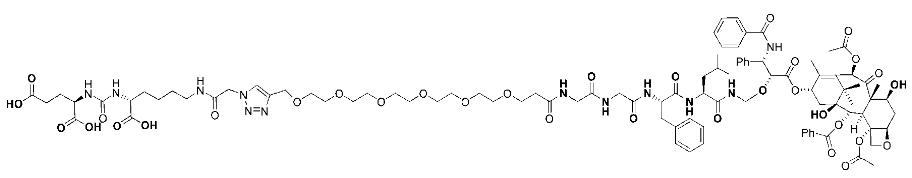 |
| S25 | 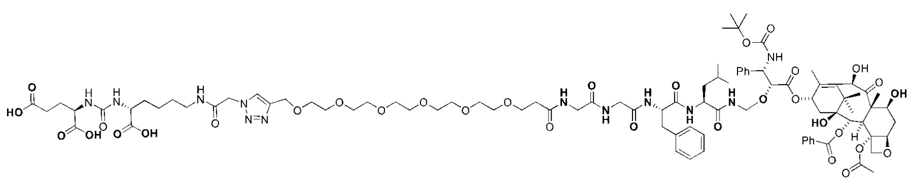 |
| S26 | 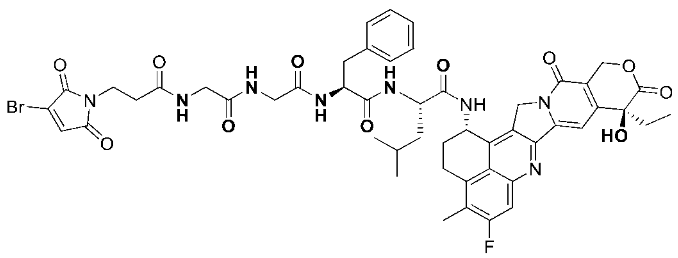 |
| S27 | 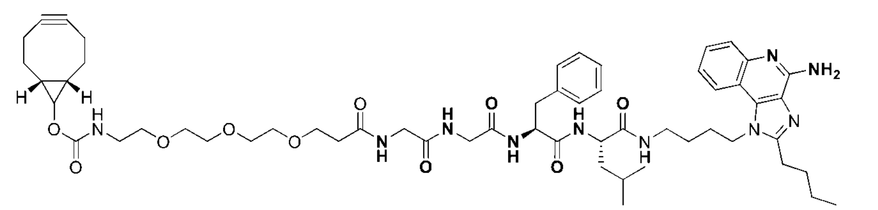 |

(continued)

| | |
|---|---|
| S28 | 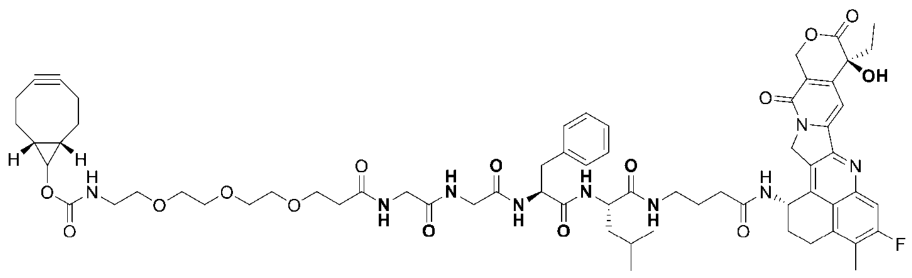 |
| S29 | 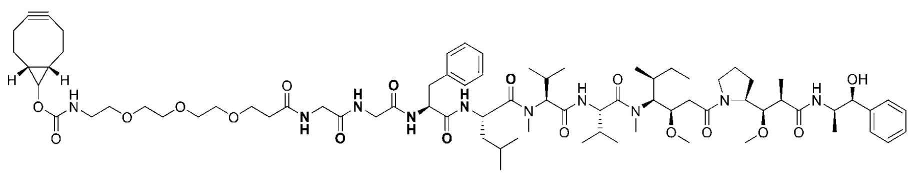 |
| S30 | 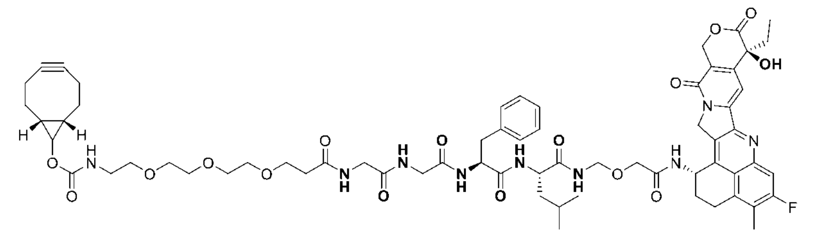 |
| S31 | 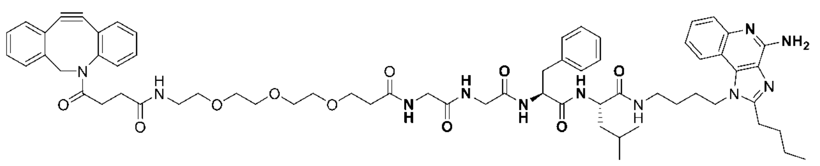 |
| S32 | 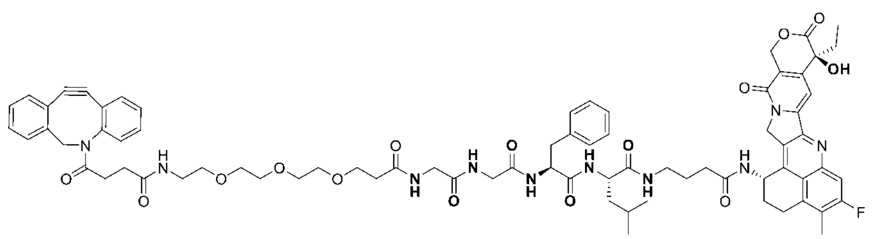 |
| S33 | 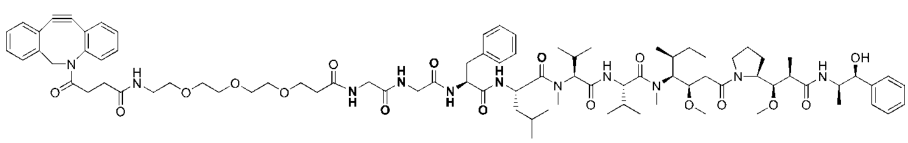 |
| S34 | 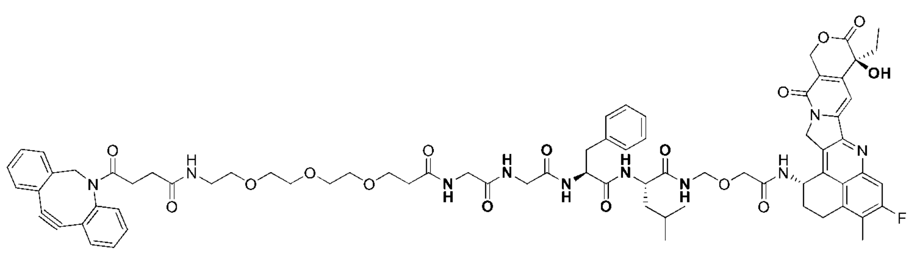 |

**6.** An antibody conjugate or a stereoisomer thereof or a pharmaceutically acceptable salt thereof, wherein the antibody conjugate is as shown in formula (III),

R3-(R1'-L-R2)$_x$ Formula (III);

wherein

R3 refers to an antibody moiety,
R1'-L-R2 refers to a group formed by the conjugation of the conjugate of formula (II)with the antibody element through the R1 group; wherein, R1, L, and R2 are as defined in claim 1; R1' is a group formed by the conjugation of R1 with the antibody element; and
x is 1 to 8.

**7.** The antibody conjugate of claim 6, wherein the antibody is selected from the group consisting of: HER2, CD19, CD20, EGFR, CD22, CD3, TROP2, Glycoprotein NMB, Guanylyl cyclase C, CEA, AXL, GCC, CD79b, PSMA, ENPP3, Mesothelin, CD138, NaPi2b, CD56, CD74, FOLR1, DLL3, CEACAM5, CD142, SLAMF7, CD25, SLTRK6, CD37, CD70, AGS-22, C4.4A, FGFR2, Ly6E, MUC16, BCMA, pCadherin, Ephrin-A, LAMP1, MUC1, PDL1, HER2, NY-ESO-1, BCMA, WT1, MUC1, CD20, CD23, ROR1, CD123, CD33, CD44v6, CD174, CD30, CD133, cMet, FAP, EphA2, GD2, GPC3, IL-13Ra2, LewisY, SS1, CD171, EGFR, EGFRvIII, VEGFR2, NY-ESO-1, MUC-1 , and MAGE-A3, or combinations thereof.

**8.** A pharmaceutical composition, wherein the pharmaceutical composition comprises: (i) the conjugate or the stereoisomer thereof or the pharmaceutically acceptable salt thereof according to any one of claims 1 to 5, or the antibody conjugate or the stereoisomer thereof or the pharmaceutically acceptable salt thereof according to any one of claims 6 to 7; and (ii) pharmaceutically acceptable carriers.

**9.** Use of the conjugate of claim 1, or the antibody conjugate of claim 6 in the preparation of a medicament for treating or preventing tumor or inflammation.

**10.** The use of claim 9, wherein the tumor is selected from the group consisting of: bladder cancer, brain cancer, breast cancer, cervical cancer, colorectal cancer, esophageal cancer, kidney cancer, liver cancer, lung cancer, nasopharyngeal cancer, pancreatic cancer, prostate cancer, skin cancer, stomach cancer, uterine cancer, ovarian cancer, testicular cancer, and blood cancer, or a combination thereof.

**11.** A linker, wherein the linker is as shown in formula (I):

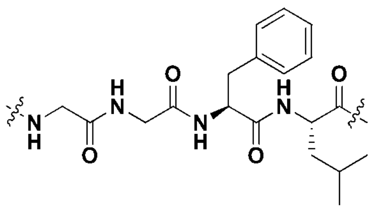

Formula (I).

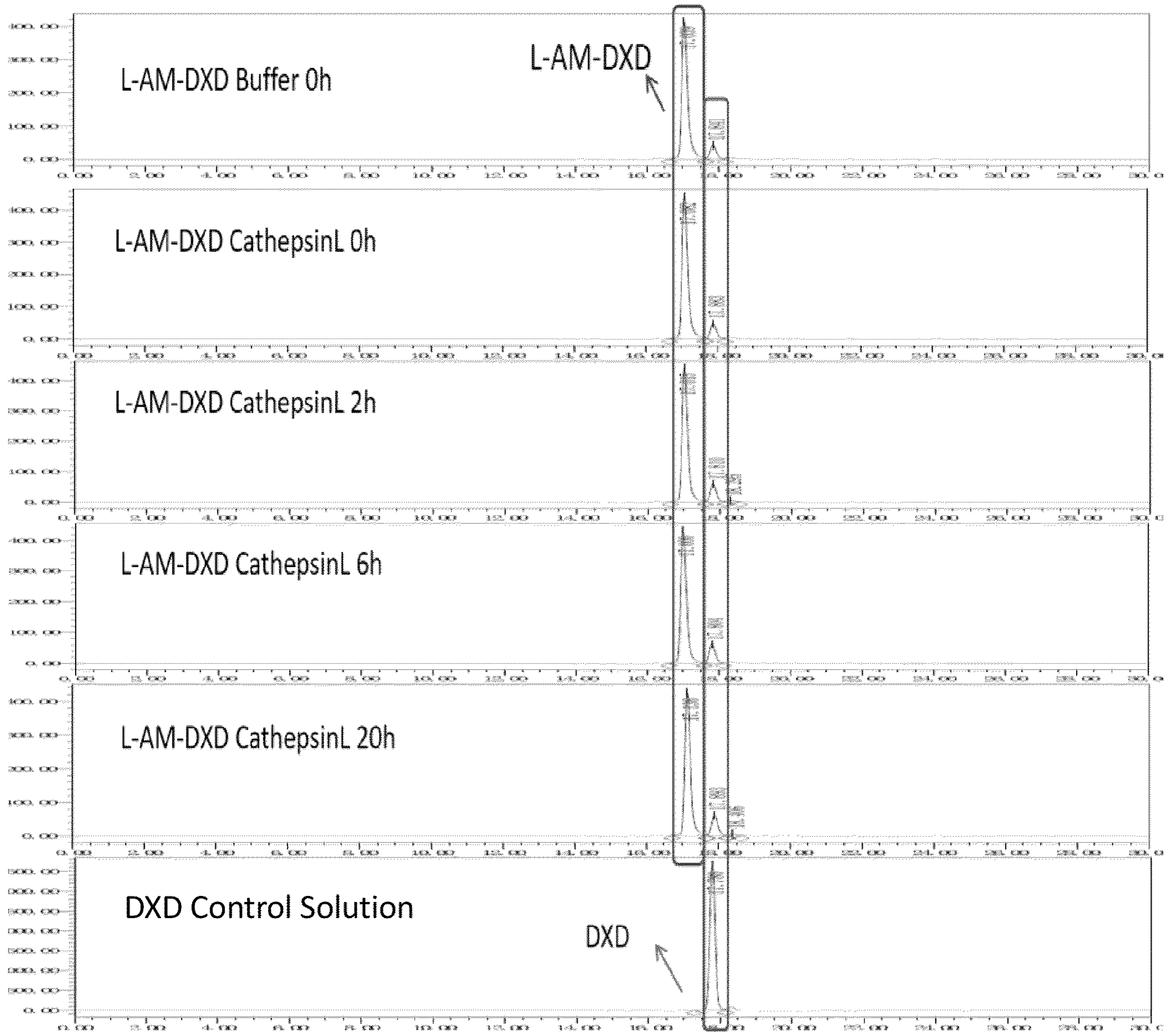
L-AM-DXD Buffer 0h
L-AM-DXD
L-AM-DXD CathepsinL 0h
L-AM-DXD CathepsinL 2h
L-AM-DXD CathepsinL 6h
L-AM-DXD CathepsinL 20h
DXD Control Solution
DXD

FIG. 1

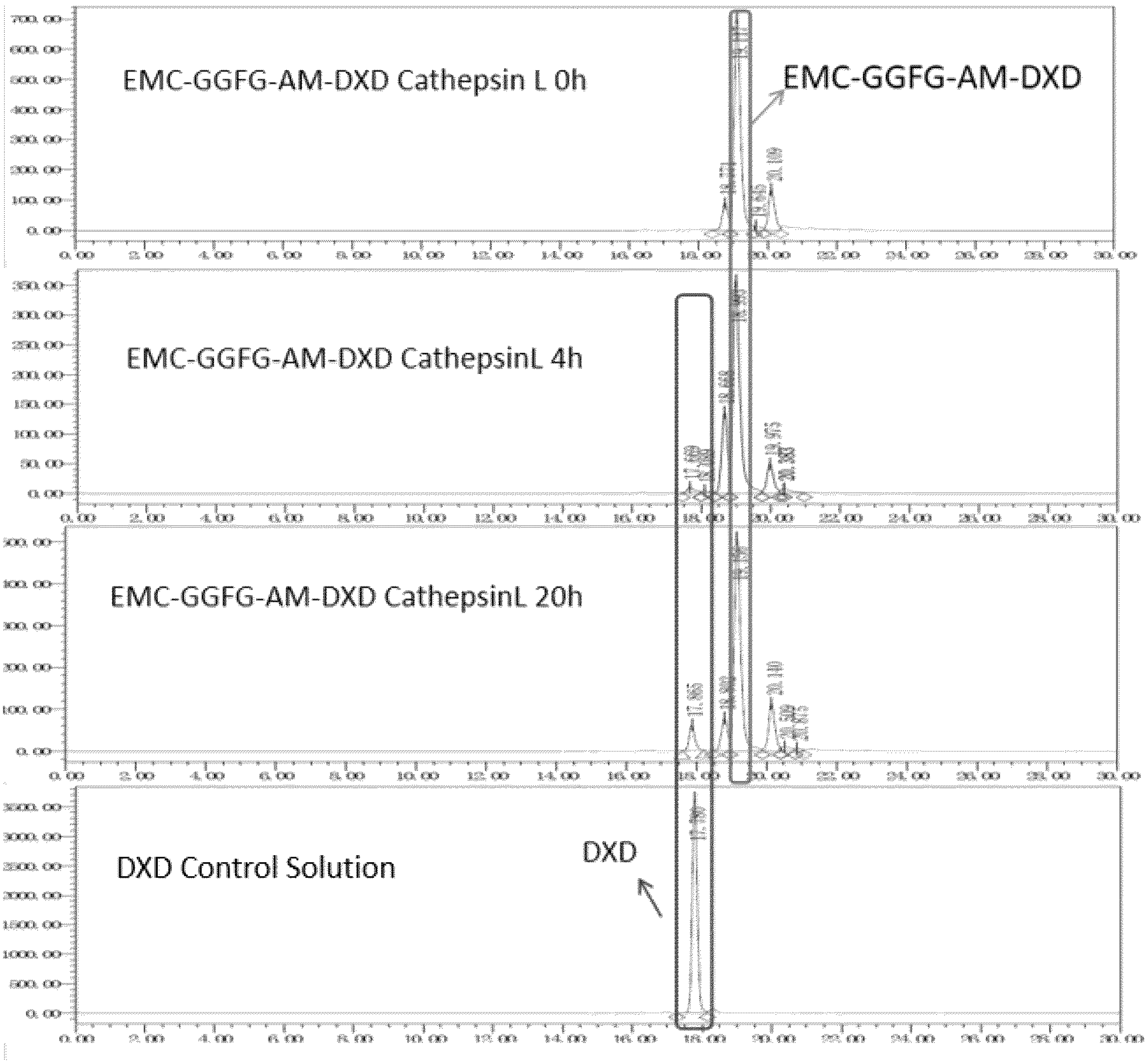
EMC-GGFG-AM-DXD Cathepsin L 0h
EMC-GGFG-AM-DXD
EMC-GGFG-AM-DXD CathepsinL 4h
EMC-GGFG-AM-DXD CathepsinL 20h
DXD Control Solution
DXD

FIG. 2

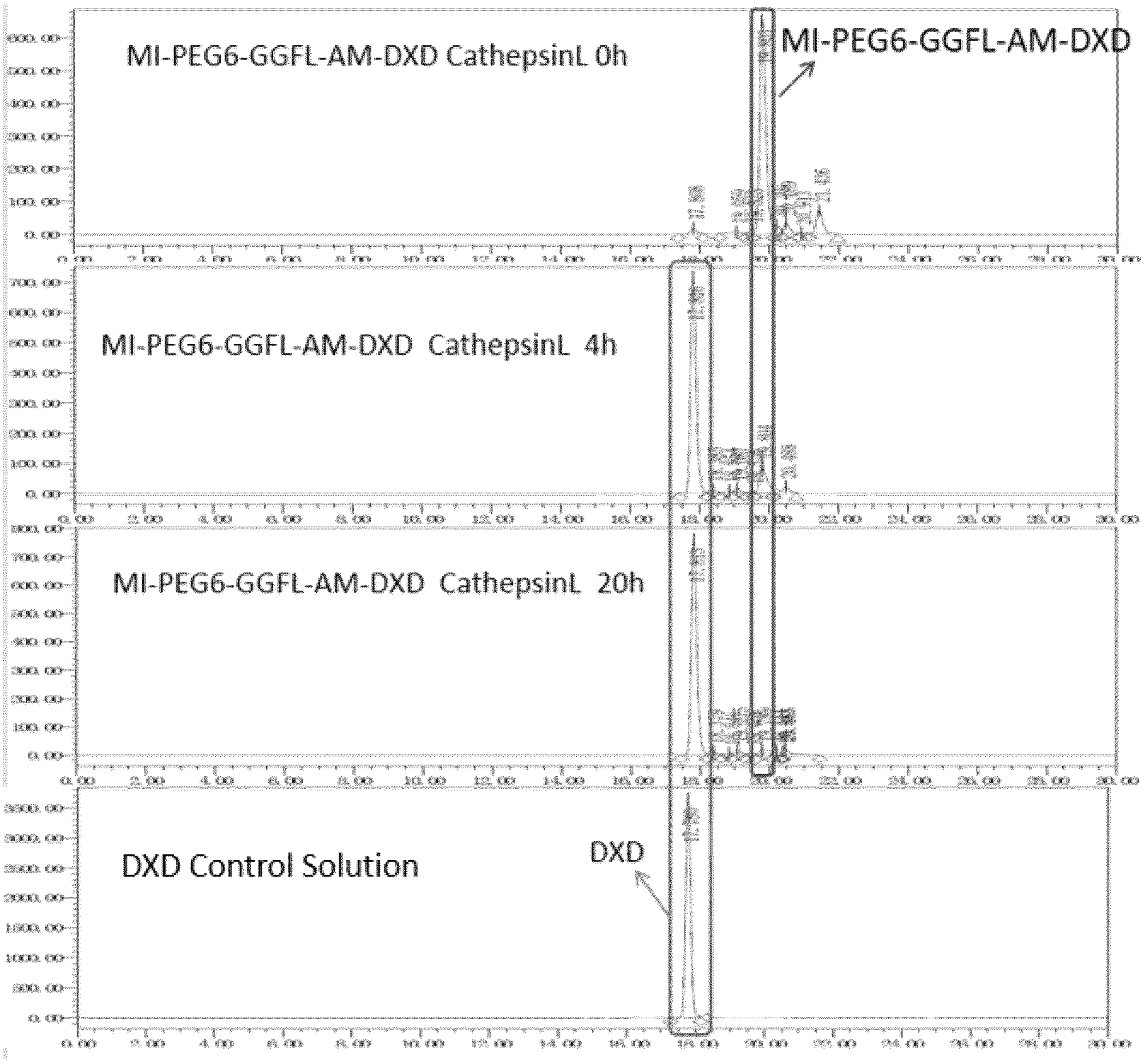
MI-PEG6-GGFL-AM-DXD CathepsinL 0h
MI-PEG6-GGFL-AM-DXD
MI-PEG6-GGFL-AM-DXD CathepsinL 4h
MI-PEG6-GGFL-AM-DXD CathepsinL 20h
DXD Control Solution
DXD

FIG. 3

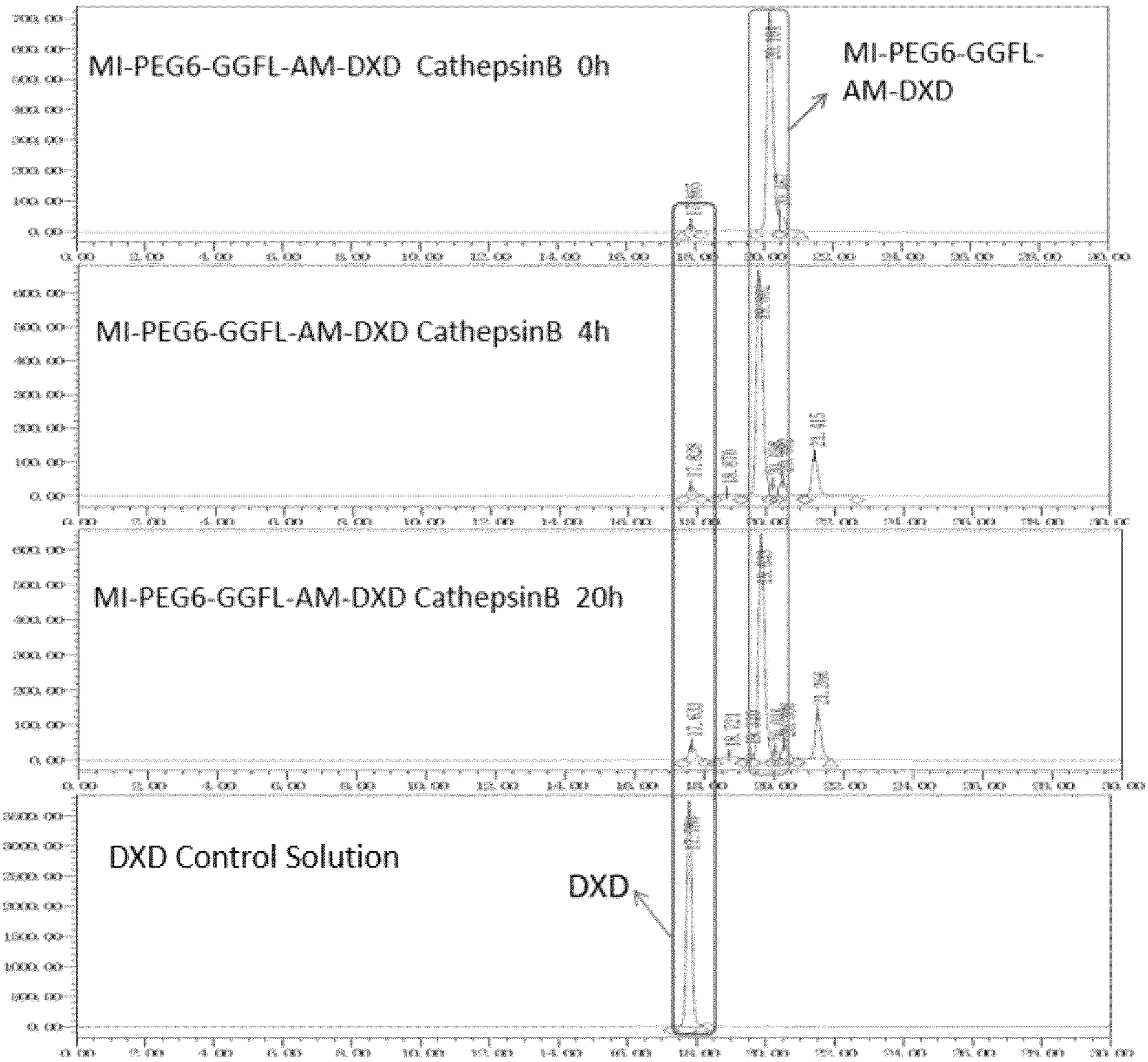
MI-PEG6-GGFL-AM-DXD CathepsinB 0h
MI-PEG6-GGFL-AM-DXD
MI-PEG6-GGFL-AM-DXD CathepsinB 4h
MI-PEG6-GGFL-AM-DXD CathepsinB 20h
DXD Control Solution
DXD

FIG. 4

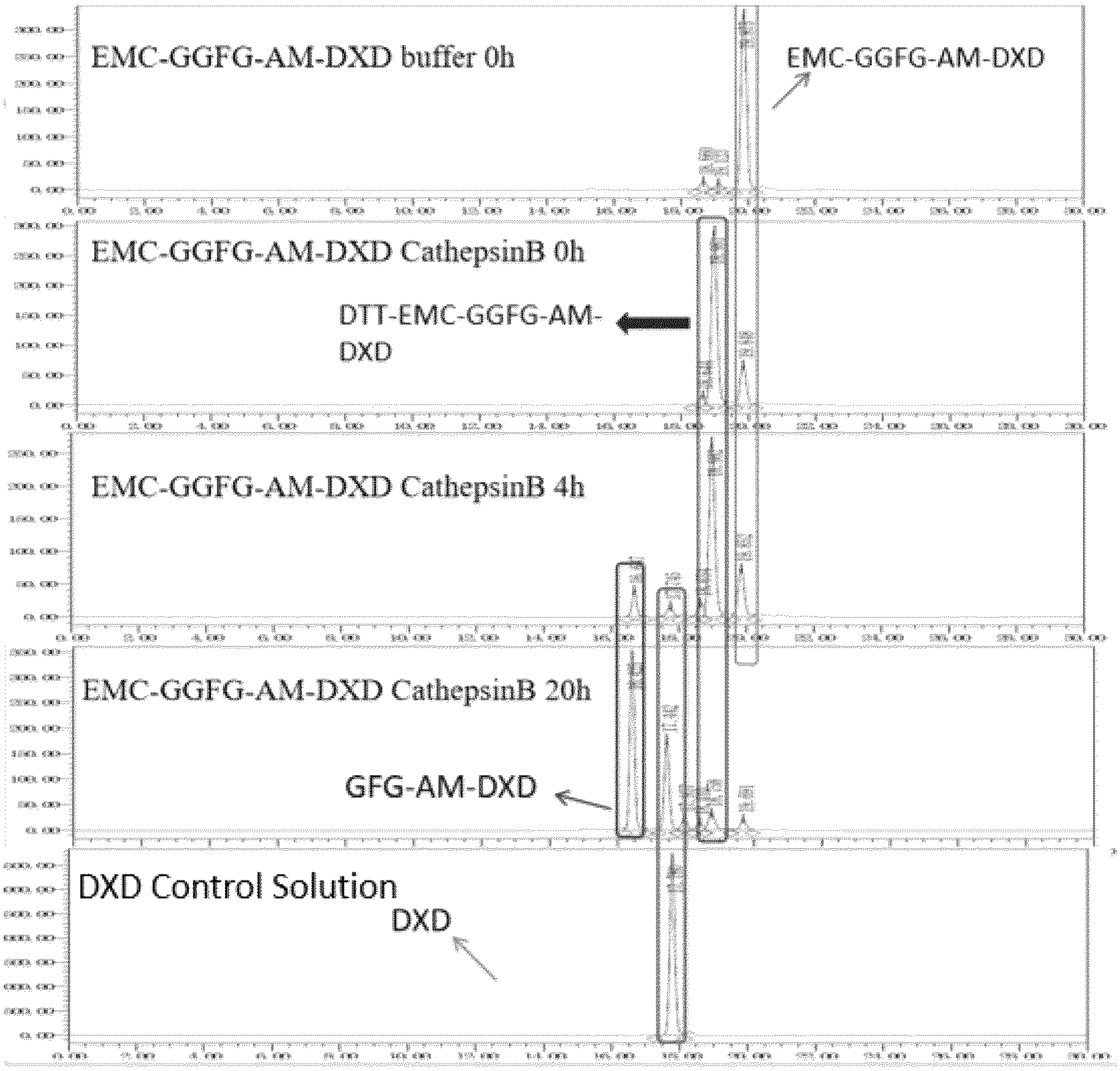
EMC-GGFG-AM-DXD buffer 0h
EMC-GGFG-AM-DXD
EMC-GGFG-AM-DXD CathepsinB 0h
DTT-EMC-GGFG-AM-DXD
EMC-GGFG-AM-DXD CathepsinB 4h
EMC-GGFG-AM-DXD CathepsinB 20h
GFG-AM-DXD
DXD Control Solution
DXD

FIG. 5

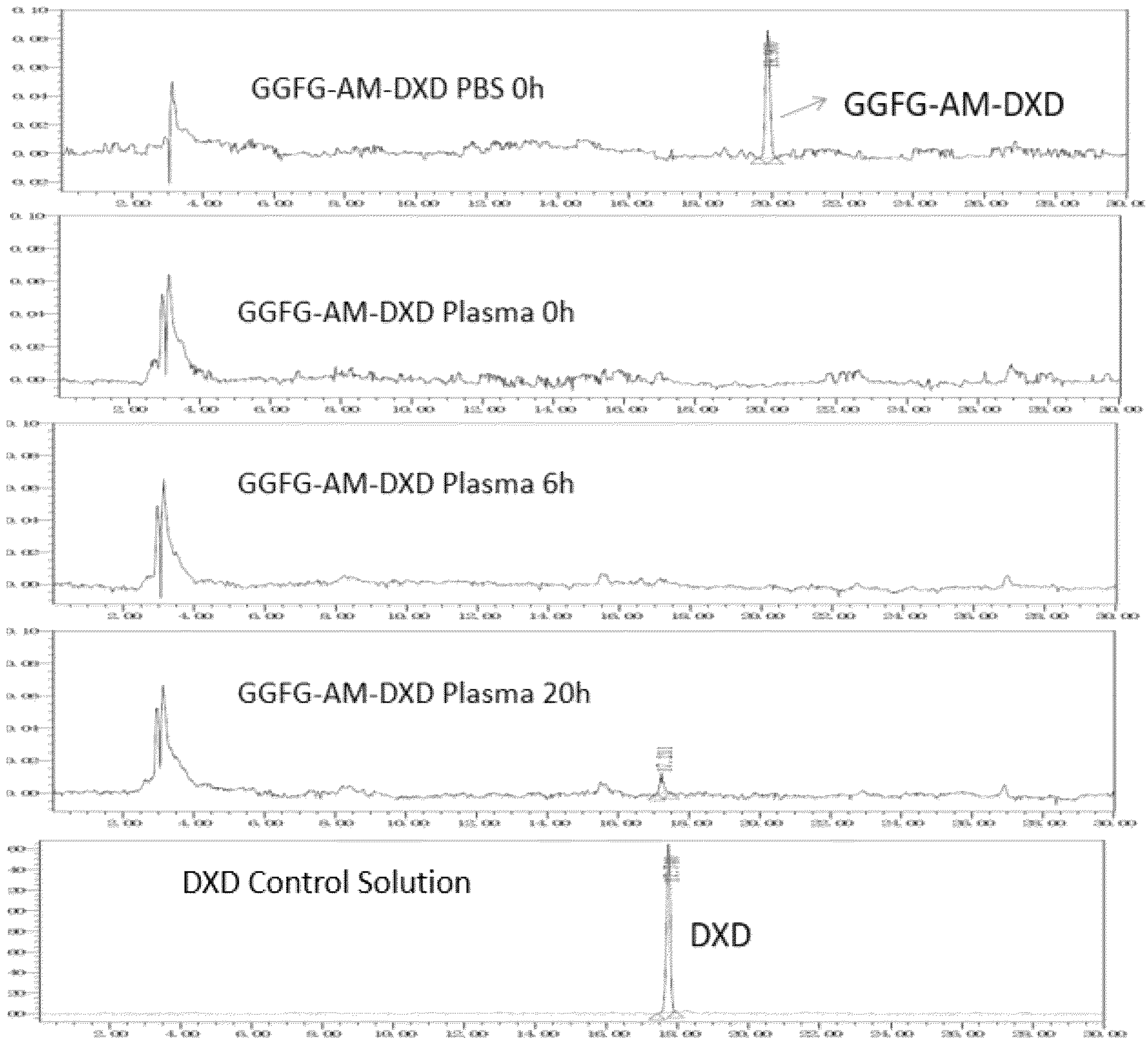
GGFG-AM-DXD PBS 0h
GGFG-AM-DXD
GGFG-AM-DXD Plasma 0h
GGFG-AM-DXD Plasma 6h
GGFG-AM-DXD Plasma 20h
DXD Control Solution
DXD

FIG. 6

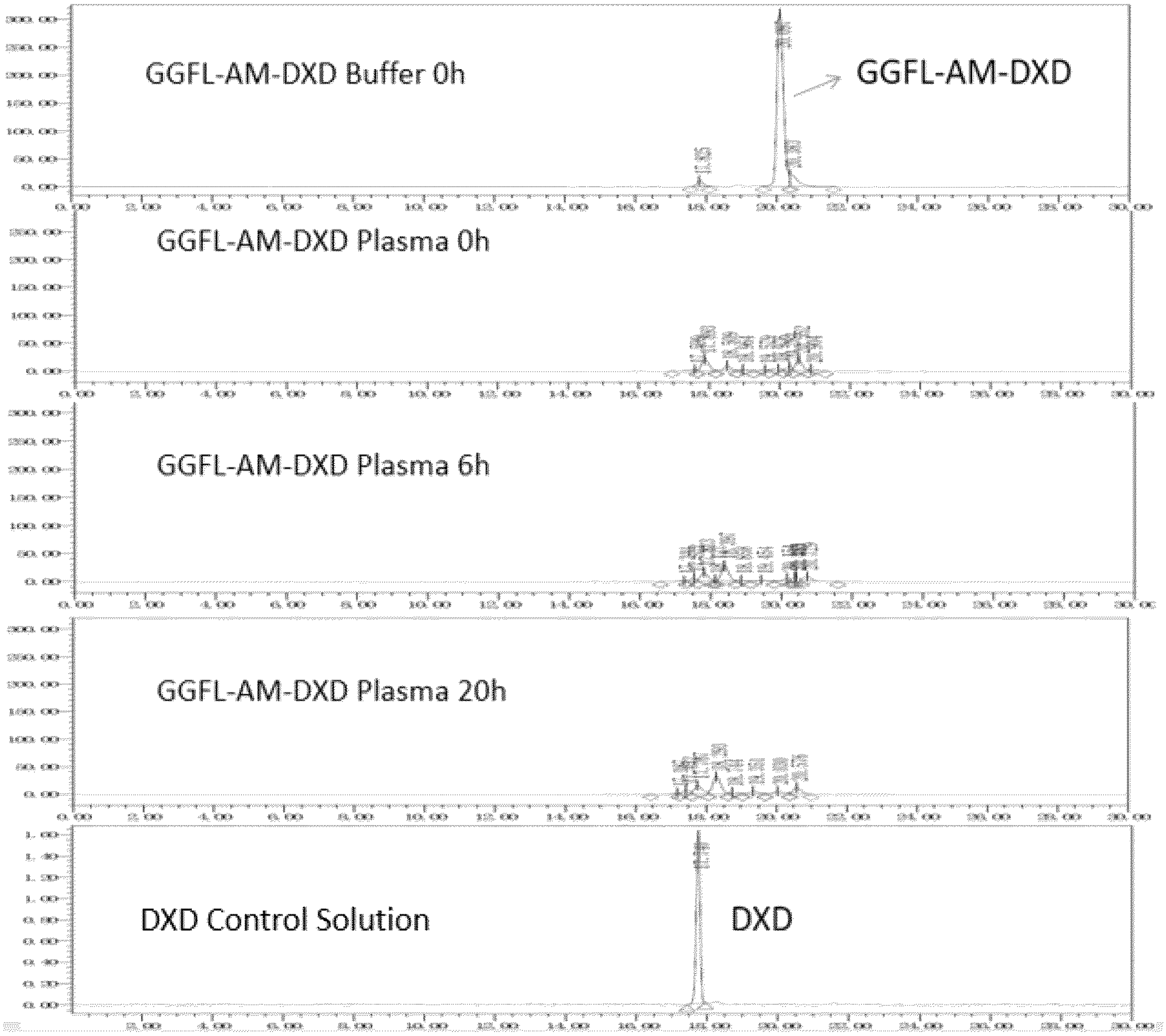
GGFL-AM-DXD Buffer 0h
GGFL-AM-DXD
GGFL-AM-DXD Plasma 0h
GGFL-AM-DXD Plasma 6h
GGFL-AM-DXD Plasma 20h
DXD Control Solution
DXD

FIG. 7

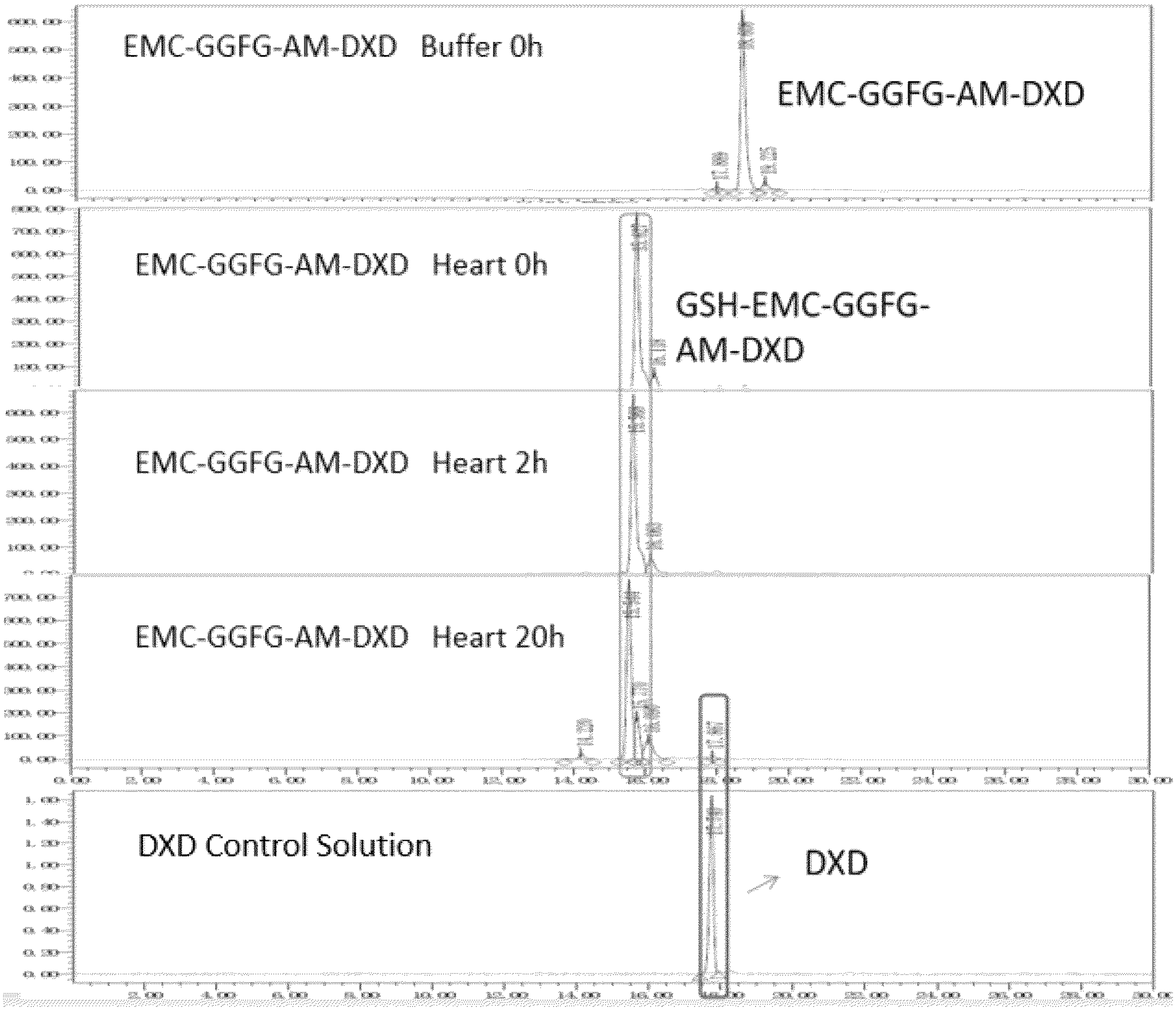
EMC-GGFG-AM-DXD Buffer 0h
EMC-GGFG-AM-DXD
EMC-GGFG-AM-DXD Heart 0h
GSH-EMC-GGFG-AM-DXD
EMC-GGFG-AM-DXD Heart 2h
EMC-GGFG-AM-DXD Heart 20h
DXD Control Solution
DXD

FIG. 8

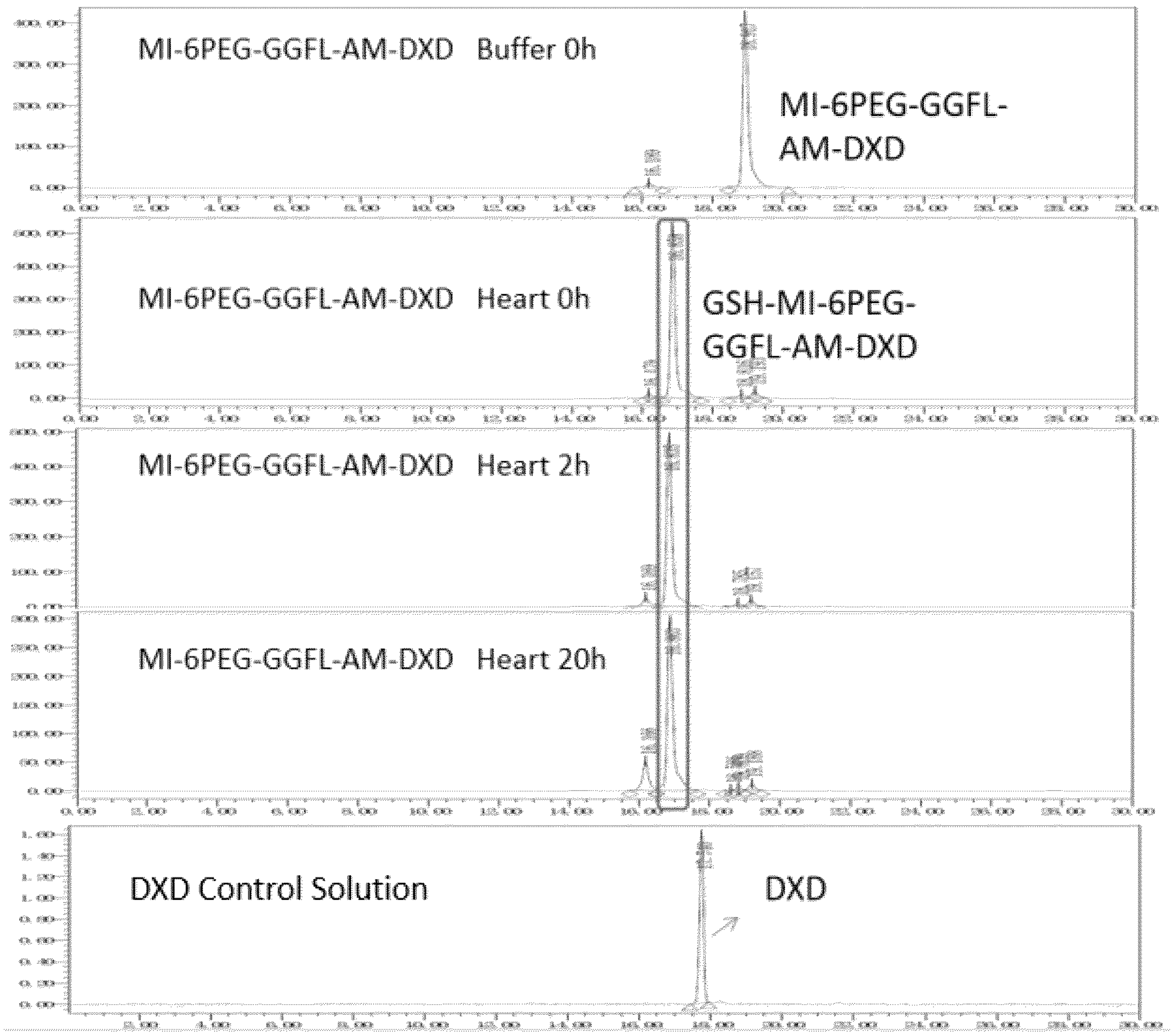
MI-6PEG-GGFL-AM-DXD Buffer 0h
MI-6PEG-GGFL-AM-DXD
MI-6PEG-GGFL-AM-DXD Heart 0h
GSH-MI-6PEG-GGFL-AM-DXD
MI-6PEG-GGFL-AM-DXD Heart 2h
MI-6PEG-GGFL-AM-DXD Heart 20h
DXD Control Solution
DXD

FIG. 9

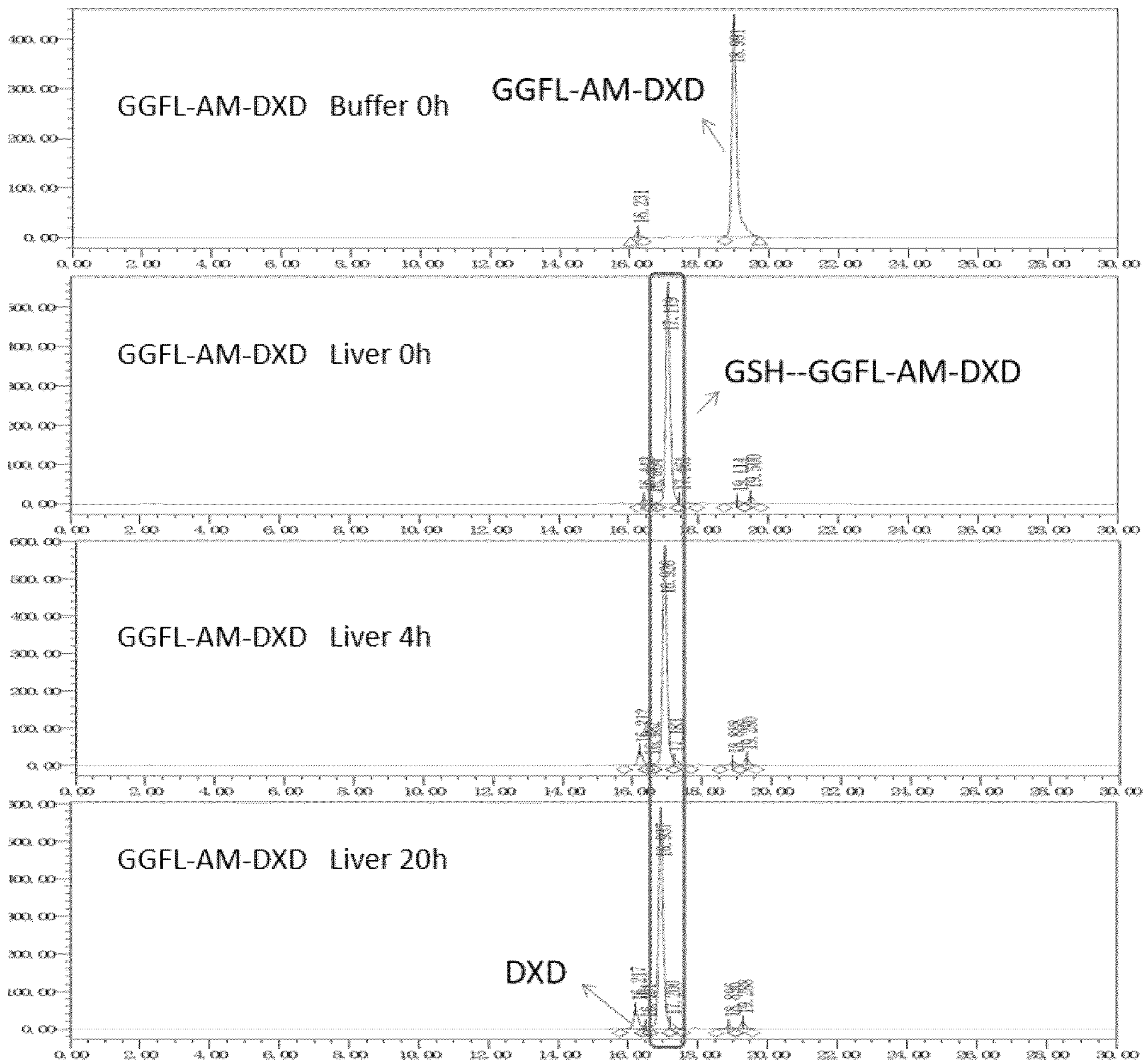
GGFL-AM-DXD Buffer 0h
GGFL-AM-DXD
GGFL-AM-DXD Liver 0h
GSH--GGFL-AM-DXD
GGFL-AM-DXD Liver 4h
GGFL-AM-DXD Liver 20h
DXD

FIG. 10

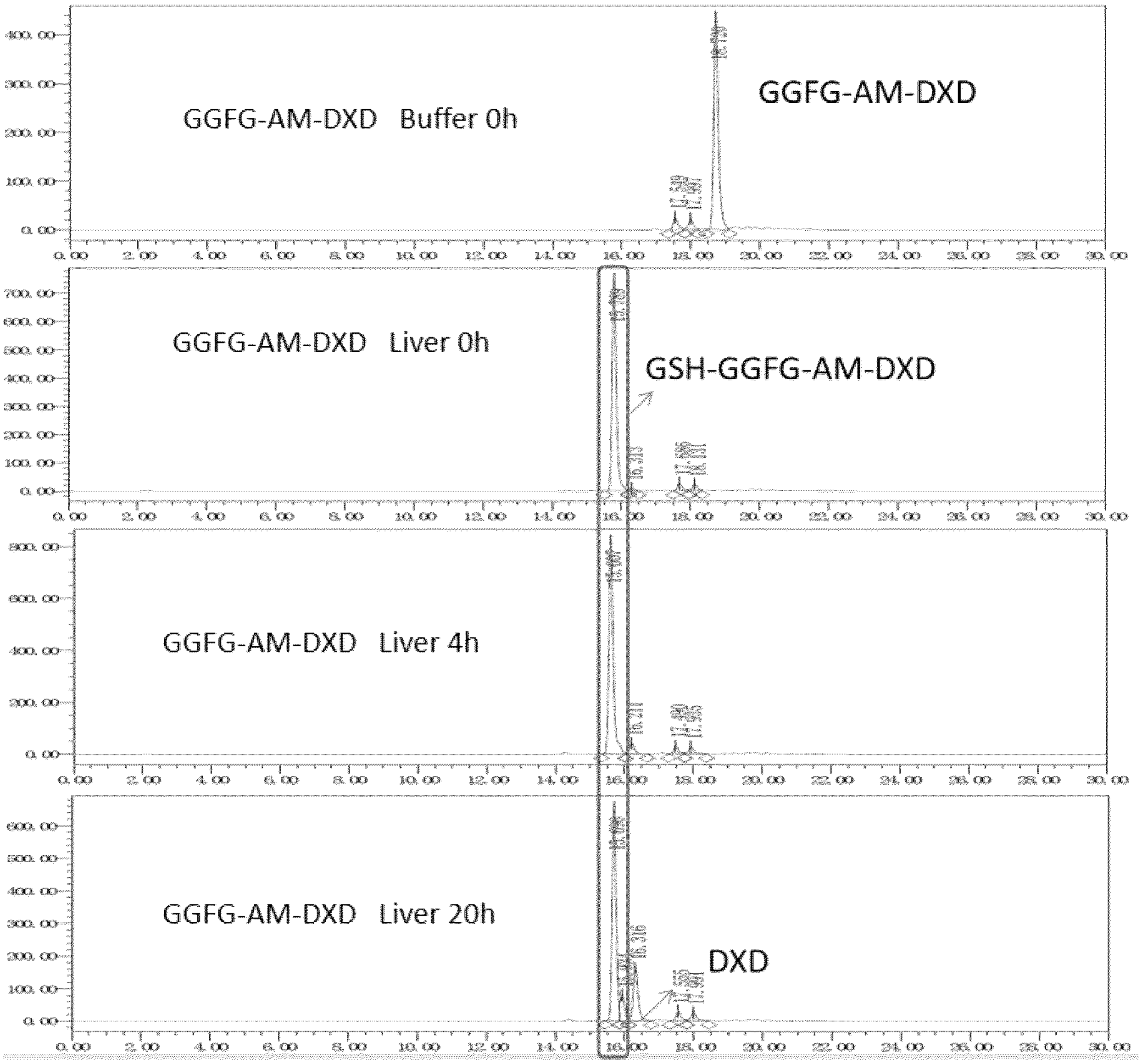
GGFG-AM-DXD Buffer 0h
GGFG-AM-DXD
GGFG-AM-DXD Liver 0h
GSH-GGFG-AM-DXD
GGFG-AM-DXD Liver 4h
GGFG-AM-DXD Liver 20h
DXD

FIG. 11

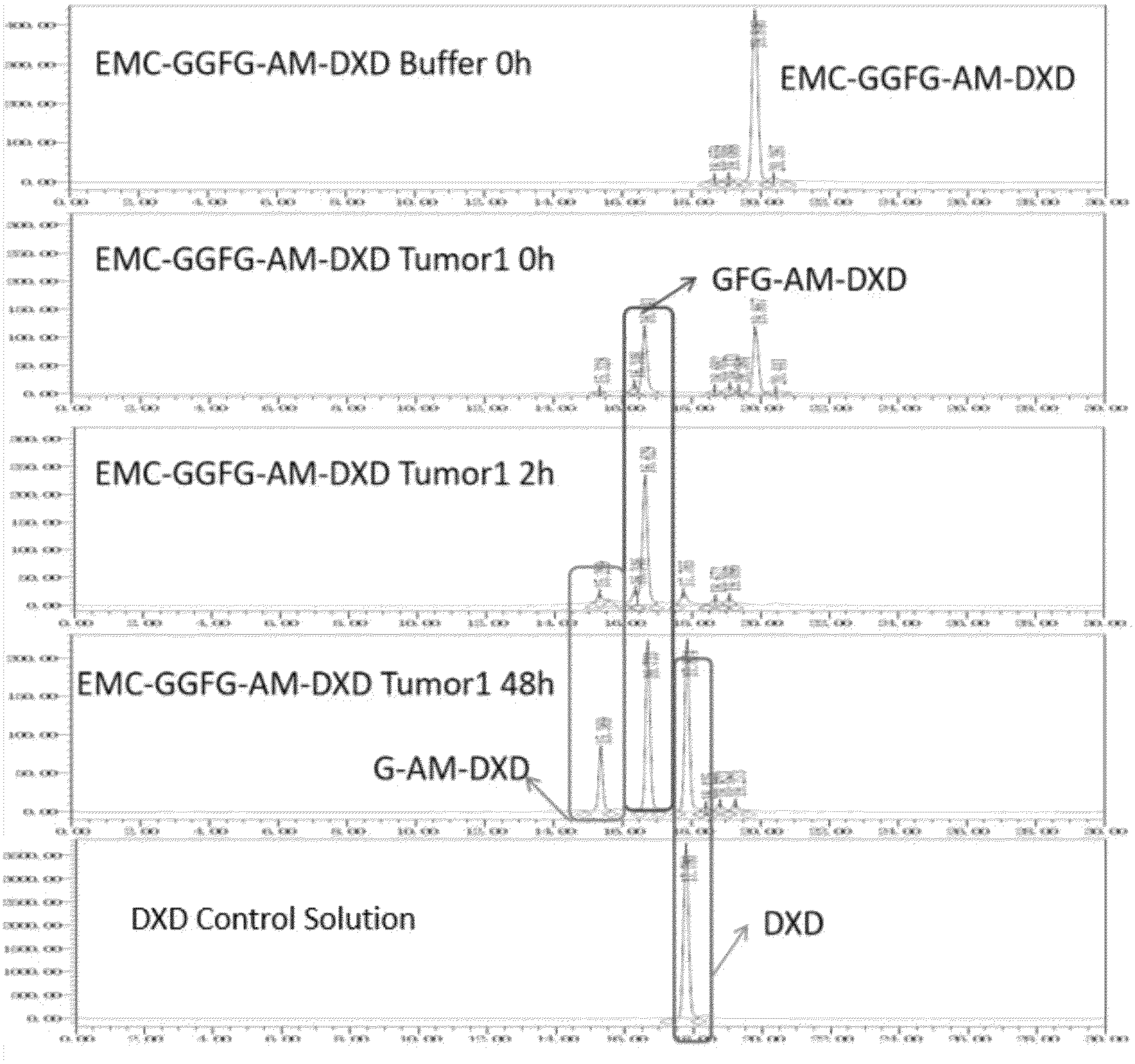
EMC-GGFG-AM-DXD Buffer 0h
EMC-GGFG-AM-DXD
EMC-GGFG-AM-DXD Tumor1 0h
GFG-AM-DXD
EMC-GGFG-AM-DXD Tumor1 2h
EMC-GGFG-AM-DXD Tumor1 48h
G-AM-DXD
DXD Control Solution
DXD

FIG. 12

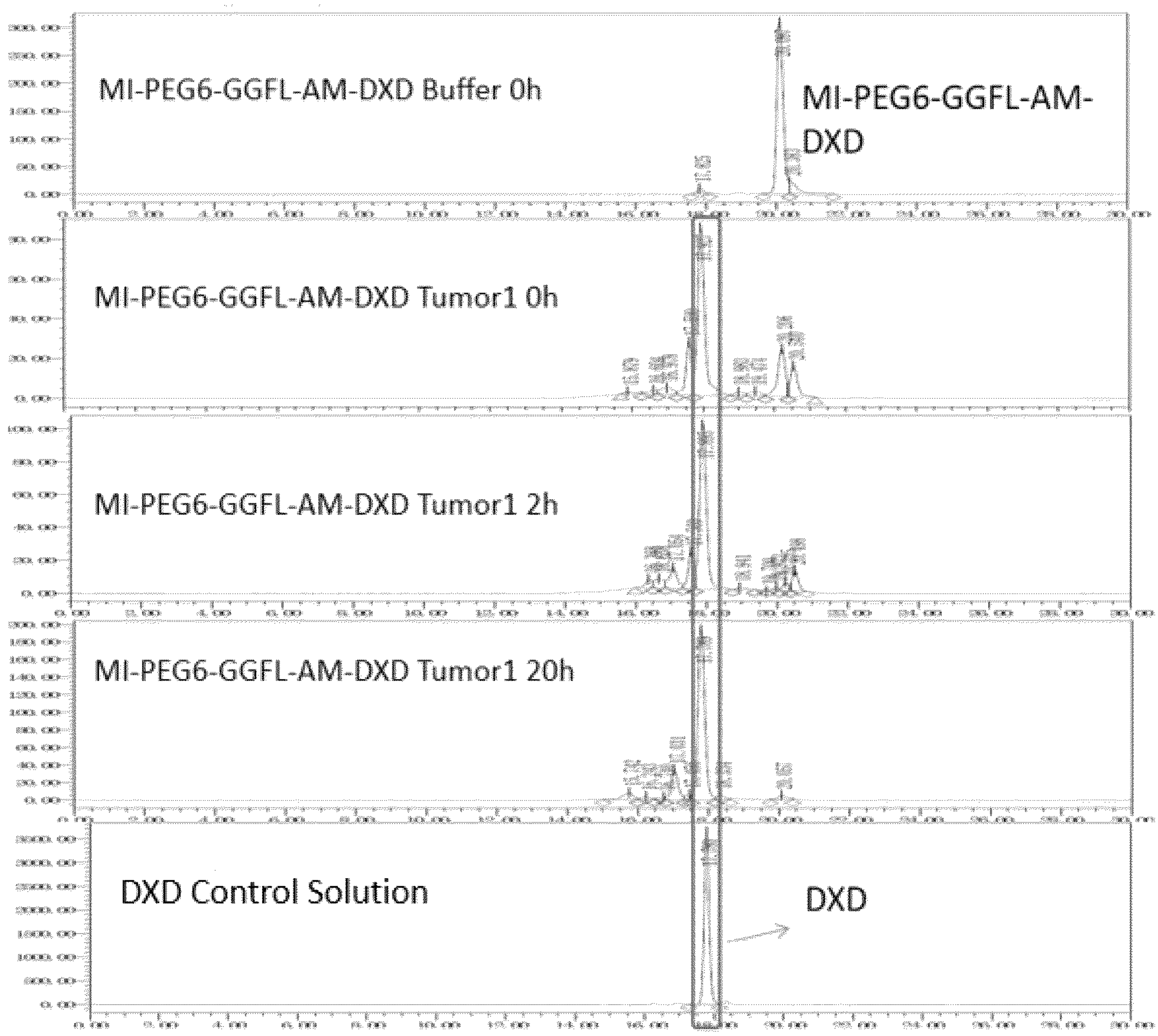
MI-PEG6-GGFL-AM-DXD Buffer 0h
MI-PEG6-GGFL-AM-DXD
MI-PEG6-GGFL-AM-DXD Tumor1 0h
MI-PEG6-GGFL-AM-DXD Tumor1 2h
MI-PEG6-GGFL-AM-DXD Tumor1 20h
DXD Control Solution
DXD

FIG. 13

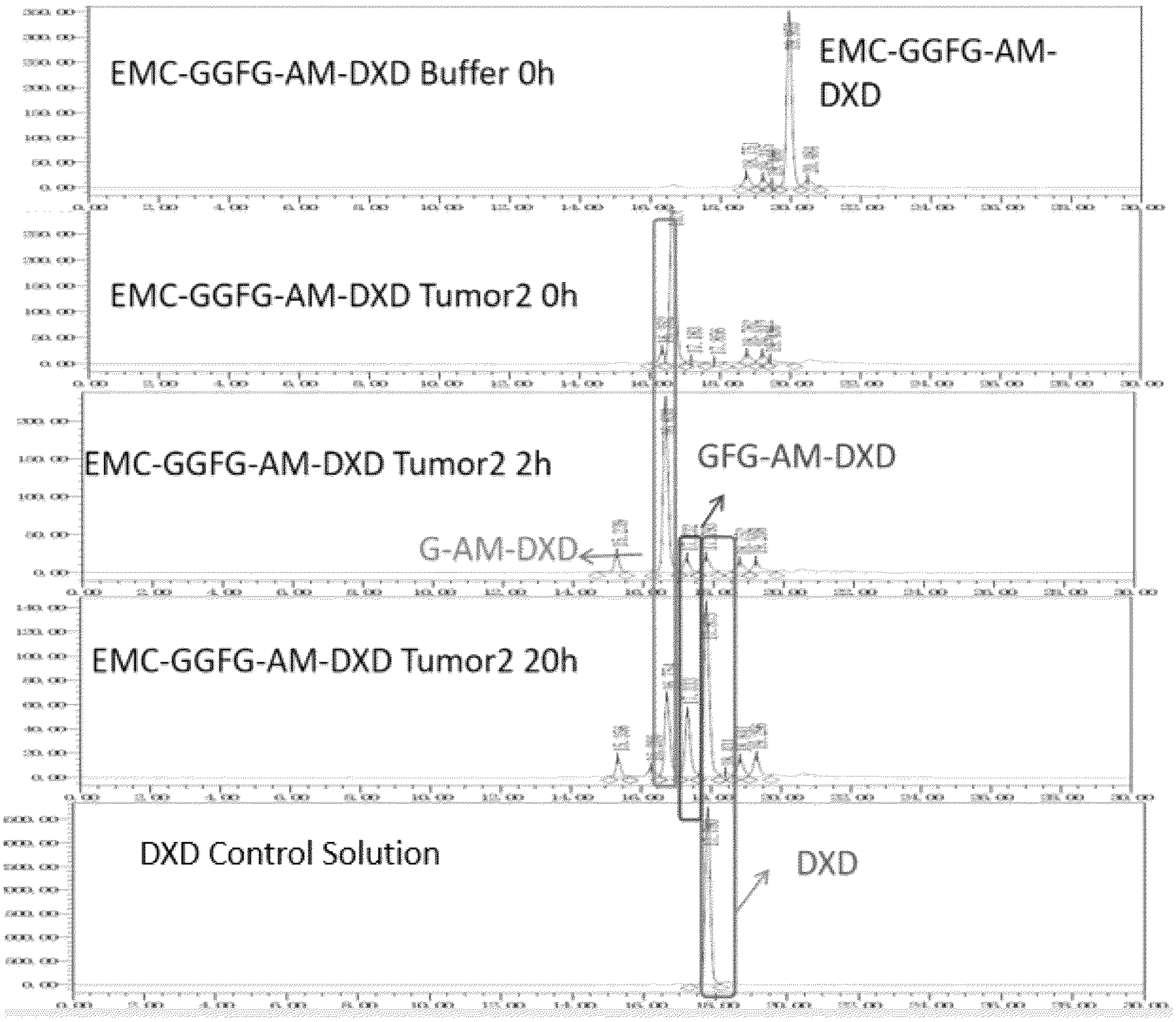
EMC-GGFG-AM-DXD Buffer 0h
EMC-GGFG-AM-DXD
EMC-GGFG-AM-DXD Tumor2 0h
EMC-GGFG-AM-DXD Tumor2 2h
GFG-AM-DXD
G-AM-DXD
EMC-GGFG-AM-DXD Tumor2 20h
DXD Control Solution
DXD

FIG. 14

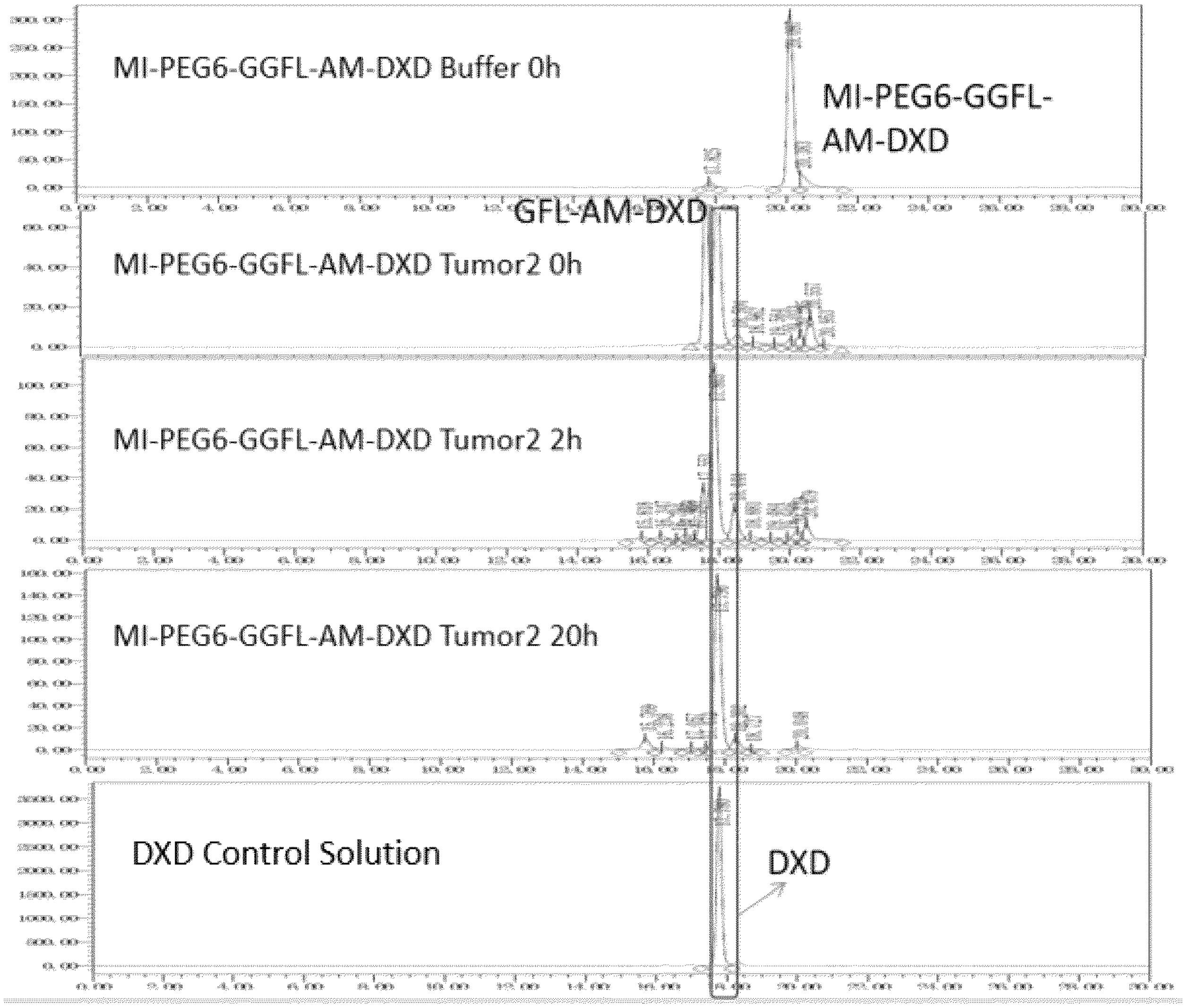
MI-PEG6-GGFL-AM-DXD Buffer 0h
MI-PEG6-GGFL-AM-DXD
GFL-AM-DXD
MI-PEG6-GGFL-AM-DXD Tumor2 0h
MI-PEG6-GGFL-AM-DXD Tumor2 2h
MI-PEG6-GGFL-AM-DXD Tumor2 20h
DXD Control Solution
DXD

FIG. 15

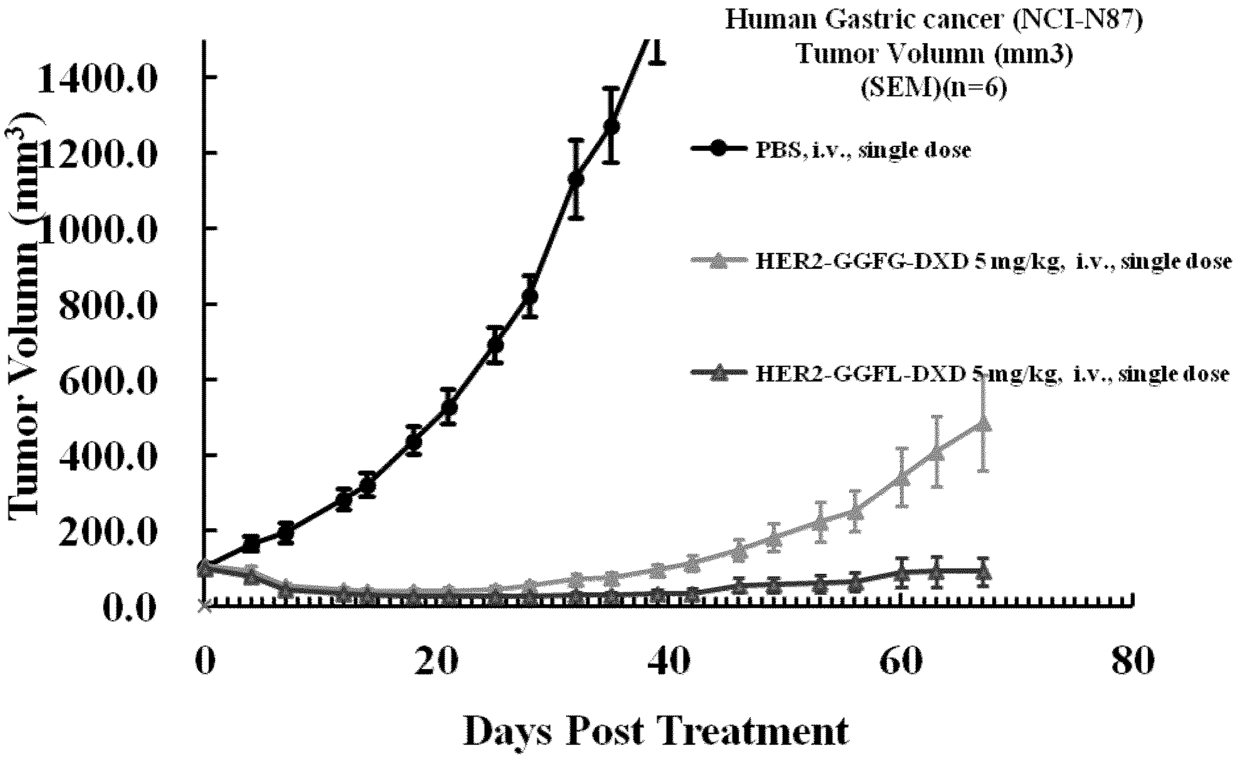
Human Gastric cancer (NCI-N87)
Tumor Volumn (mm3)
(SEM)(n=6)
PBS, i.v., single dose
HER2-GGFG-DXD 5 mg/kg, i.v., single dose
HER2-GGFL-DXD 5 mg/kg, i.v., single dose
Tumor Volumn (mm³)
1400.0
1200.0
1000.0
800.0
600.0
400.0
200.0
0.0
0
20
40
60
80
Days Post Treatment

FIG. 16

## INTERNATIONAL SEARCH REPORT

International application No.
**PCT/CN2024/081715**

**A. CLASSIFICATION OF SUBJECT MATTER**

C07K5/103(2006.01)i; A61K47/64(2017.01)i; A61K47/68(2017.01)i; A61P35/00(2006.01)i; C07K7/02(2006.01)i; C07K7/06(2006.01)i; C07K7/64(2006.01)i; C07K16/32(2006.01)i

According to International Patent Classification (IPC) or to both national classification and IPC

**B. FIELDS SEARCHED**

Minimum documentation searched (classification system followed by classification symbols)

C07K, A61K, A61P

Documentation searched other than minimum documentation to the extent that such documents are included in the fields searched

Electronic data base consulted during the international search (name of data base and, where practicable, search terms used)

CNABS, CNTXT, DWPI, VEN, ENTXTC, ENTXT, CNKI, PUBMED, ISI_Web of Science, Science Direct, STNext: 上海亲合力生物医药科技股份有限公司, 亚飞(上海)生物医药科技有限公司, SHANGHAI AFFINITY BIOMEDICAL TECHNOLOGY, 偶联物, 连接子, Gly-Gly-Phe-Leu, GGFL, 甘氨酸, 苯丙氨酸, 亮氨酸, conjugate, linker, structural formula search

**C. DOCUMENTS CONSIDERED TO BE RELEVANT**

| Category* | Citation of document, with indication, where appropriate, of the relevant passages | Relevant to claim No. |
|---|---|---|
| Y | CN 104755494 A (DAIICHI SANKYO COMPANY, LIMITED) 01 July 2015 (2015-07-01)<br>description, abstract, claims 1-48, and embodiments 1-29 and 43-48 | 1-10 |
| Y | CN 105829346 A (DAIICHI SANKYO COMPANY, LIMITED) 03 August 2016 (2016-08-03)<br>description, abstract, claims 1-27, and embodiments 1-13 | 1-10 |
| Y | CN 111936169 A (SEATTLE GENETICS INC.) 13 November 2020 (2020-11-13)<br>description, abstract, and claims 1-143 | 1-10 |
| Y | CN 114845740 A (INNATE PHARMA S.A.) 02 August 2022 (2022-08-02)<br>abstract, claims 1-36, and description, paragraphs 470-473 | 1-10 |
| Y | WO 2021164765 A1 (YAFEI SHANGHAI BIOLOGY MEDICINE SCIENCE & TECHNOLOGY CO., LTD.) 26 August 2021 (2021-08-26)<br>abstract, and claims 1-20 | 1-10 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **20 June 2024** | **25 June 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No. **PCT/CN2024/081715**

**C. DOCUMENTS CONSIDERED TO BE RELEVANT**

| Category* | Citation of document, with indication, where appropriate, of the relevant passages | Relevant to claim No. |
|---|---|---|
| Y | CN 113274507 A (YAFEI SHANGHAI BIOLOGY MEDICINE SCIENCE & TECHNOLOGY CO., LTD.) 20 August 2021 (2021-08-20)<br>abstract, and claims 1-20 | 1-10 |
| Y | DELMOTTE, F. et al. "Macromolecular prodrugs: antitumor activity of peptidyl daunorubicin linked to highly hydrosoluble polymers"<br>*Recent Adv. Chemother., Proc. Int. Congr. Chemother., 14th (1985),*<br>Vol. Anticancer Sect. 1, 31 December 1985 (1985-12-31),<br>pages 345-346 | 1-10 |
| X | DELMOTTE, F. et al. "Macromolecular prodrugs: antitumor activity of peptidyl daunorubicin linked to highly hydrosoluble polymers"<br>*Recent Adv. Chemother., Proc. Int. Congr. Chemother., 14th (1985),*<br>Vol. Anticancer Sect. 1, 31 December 1985 (1985-12-31),<br>pages 345-346 | 11 |

Form PCT/ISA/210 (second sheet) (July 2022)

**INTERNATIONAL SEARCH REPORT**
**Information on patent family members**

International application No.
**PCT/CN2024/081715**

| Patent document cited in search report | | | Publication date (day/month/year) | Patent family member(s) | | | Publication date (day/month/year) |
|---|---|---|---|---|---|---|---|
| CN | 104755494 | A | 01 July 2015 | US | 2016279259 | A1 | 29 September 2016 |
| | | | | US | 9808537 | B2 | 07 November 2017 |
| | | | | LT | 2907824 | T | 11 June 2018 |
| | | | | JP | 2022008581 | A | 13 January 2022 |
| | | | | JP | 7170812 | B2 | 14 November 2022 |
| | | | | CA | 3021435 | A1 | 17 April 2014 |
| | | | | CA | 3021435 | C | 12 October 2021 |
| | | | | JP | 2023011799 | A | 24 January 2023 |
| | | | | BR | 122021014396 | B1 | 05 July 2022 |
| | | | | JP | 6030267 | B1 | 24 November 2016 |
| | | | | JP | 2017036274 | A | 16 February 2017 |
| | | | | BR | 112015006521 | A2 | 05 September 2017 |
| | | | | BR | 112015006521 | B1 | 03 March 2022 |
| | | | | SI | 2907824 | T1 | 29 June 2018 |
| | | | | NZ | 746440 | A | 29 November 2019 |
| | | | | JP | 5953378 | B2 | 20 July 2016 |
| | | | | JPWO | 2014057687 | A1 | 05 September 2016 |
| | | | | KR | 20220100727 | A | 15 July 2022 |
| | | | | KR | 102456726 | B1 | 20 October 2022 |
| | | | | CY | 1122789 | T1 | 05 May 2021 |
| | | | | SG | 10201804788 | XA | 30 July 2018 |
| | | | | PT | 3342785 | T | 25 February 2020 |
| | | | | PT | 2907824 | T | 07 June 2018 |
| | | | | US | 2020282073 | A1 | 10 September 2020 |
| | | | | US | 11633493 | B2 | 25 April 2023 |
| | | | | CA | 2885800 | A1 | 17 April 2014 |
| | | | | CA | 2885800 | C | 04 December 2018 |
| | | | | WO | 2014057687 | A1 | 17 April 2014 |
| | | | | ES | 2773710 | T3 | 14 July 2020 |
| | | | | US | 2024082413 | A1 | 14 March 2024 |
| | | | | US | 2015297748 | A1 | 22 October 2015 |
| | | | | US | 10195288 | B2 | 05 February 2019 |
| | | | | IL | 302494 | A | 01 June 2023 |
| | | | | AU | 2020200548 | A1 | 13 February 2020 |
| | | | | AU | 2020200548 | B2 | 25 August 2022 |
| | | | | MX | 2020010964 | A | 09 November 2020 |
| | | | | KR | 20150067149 | A | 17 June 2015 |
| | | | | KR | 101841818 | B1 | 26 March 2018 |
| | | | | HRP | 20180870 | T1 | 13 July 2018 |
| | | | | KR | 20180030734 | A | 23 March 2018 |
| | | | | KR | 101901558 | B1 | 21 September 2018 |
| | | | | TR | 201809636 | T4 | 23 July 2018 |
| | | | | TW | 202233186 | A | 01 September 2022 |
| | | | | SG | 11201502887 | WA | 28 May 2015 |
| | | | | KR | 20180105271 | A | 27 September 2018 |
| | | | | KR | 102052319 | B1 | 05 December 2019 |
| | | | | CY | 1120363 | T1 | 10 July 2019 |
| | | | | KR | 20190135559 | A | 06 December 2019 |
| | | | | KR | 102087017 | B1 | 10 March 2020 |
| | | | | TW | 201811746 | A | 01 April 2018 |

Form PCT/ISA/210 (patent family annex) (July 2022)

**INTERNATIONAL SEARCH REPORT**
**Information on patent family members**

International application No.
**PCT/CN2024/081715**

| Patent document cited in search report | Publication date (day/month/year) | Patent family member(s) | | | Publication date (day/month/year) |
|---|---|---|---|---|---|
| | | TWI | 650312 | B | 11 February 2019 |
| | | MY | 170251 | A | 13 July 2019 |
| | | PL | 3342785 | T3 | 07 September 2020 |
| | | DK | 3342785 | T3 | 23 March 2020 |
| | | EP | 3632471 | A1 | 08 April 2020 |
| | | TW | 201420117 | A | 01 June 2014 |
| | | TWI | 615152 | B | 21 February 2018 |
| | | RS | 57278 | B1 | 31 August 2018 |
| | | KR | 20220029776 | A | 08 March 2022 |
| | | KR | 102417310 | B1 | 05 July 2022 |
| | | TW | 202033499 | A | 16 September 2020 |
| | | TWI | 757740 | B | 11 March 2022 |
| | | ZA | 201501825 | B | 25 May 2022 |
| | | KR | 20210132240 | A | 03 November 2021 |
| | | KR | 102369419 | B1 | 02 March 2022 |
| | | DK | 2907824 | T3 | 23 July 2018 |
| | | PH | 12018501433 | A1 | 27 February 2019 |
| | | PL | 2907824 | T3 | 31 August 2018 |
| | | IL | 238143 | A0 | 31 May 2015 |
| | | IL | 238143 | B | 30 January 2020 |
| | | HK | 1256631 | A1 | 27 September 2019 |
| | | KR | 20210038728 | A | 07 April 2021 |
| | | KR | 102320907 | B1 | 02 November 2021 |
| | | JP | 2016196484 | A | 24 November 2016 |
| | | JP | 6186045 | B2 | 23 August 2017 |
| | | KR | 20200026323 | A | 10 March 2020 |
| | | KR | 102237639 | B1 | 07 April 2021 |
| | | SI | 3342785 | T1 | 28 February 2020 |
| | | NZ | 705394 | A | 26 October 2018 |
| | | KR | 20230142808 | A | 11 October 2023 |
| | | RU | 2015113767 | A | 10 December 2016 |
| | | RU | 2664465 | C2 | 17 August 2018 |
| | | EP | 2907824 | A1 | 19 August 2015 |
| | | EP | 2907824 | A4 | 08 June 2016 |
| | | EP | 2907824 | B1 | 11 April 2018 |
| | | RS | 60000 | B1 | 30 April 2020 |
| | | AU | 2013328111 | A1 | 12 March 2015 |
| | | AU | 2013328111 | B2 | 02 November 2017 |
| | | IL | 271760 | A | 27 February 2020 |
| | | IL | 271760 | B | 01 August 2022 |
| | | TW | 201920098 | A | 01 June 2019 |
| | | TWI | 696611 | B | 21 June 2020 |
| | | ES | 2671644 | T3 | 07 June 2018 |
| | | MX | 2015003903 | A | 17 July 2015 |
| | | MX | 364484 | B | 29 April 2019 |
| | | JP | 2020180124 | A | 05 November 2020 |
| | | JP | 6952835 | B2 | 27 October 2021 |
| | | NZ | 746439 | A | 29 November 2019 |
| | | HK | 1210477 | A1 | 22 April 2016 |
| | | EP | 3342785 | A1 | 04 July 2018 |

Form PCT/ISA/210 (patent family annex) (July 2022)

**INTERNATIONAL SEARCH REPORT**
**Information on patent family members**

International application No.
**PCT/CN2024/081715**

| Patent document cited in search report | Publication date (day/month/year) | Patent family member(s) | Publication date (day/month/year) |
|---|---|---|---|
| | | EP 3342785 B1 | 25 December 2019 |
| | | NZ 740948 A | 29 November 2019 |
| | | BR 122021014365 B1 | 05 July 2022 |
| | | JP 2018188455 A | 29 November 2018 |
| | | JP 6715888 B2 | 01 July 2020 |
| | | KR 20220146669 A | 01 November 2022 |
| | | KR 102498405 B1 | 09 February 2023 |
| | | KR 20230086800 A | 15 June 2023 |
| | | KR 102584005 B1 | 27 September 2023 |
| | | HUE 039000 T2 | 28 December 2018 |
| | | MX 2019004502 A | 21 August 2019 |
| | | US 2019008981 A1 | 10 January 2019 |
| | | US 10973924 B2 | 13 April 2021 |
| | | PH 12015500667 A1 | 18 May 2015 |
| | | ZA 201900820 B | 31 August 2022 |
| | | LT 3342785 T | 10 February 2020 |
| | | JP 2018008982 A | 18 January 2018 |
| | | JP 6371452 B2 | 08 August 2018 |
| | | RU 2018128384 A | 02 October 2018 |
| | | RU 2018128384 A3 | 20 December 2021 |
| | | HRP 20200353 T1 | 12 June 2020 |
| | | AU 2022203560 A1 | 16 June 2022 |
| | | HUE 048748 T2 | 28 August 2020 |
| | | KR 20230024433 A | 20 February 2023 |
| | | KR 102540419 B1 | 05 June 2023 |
| | | IL 294622 A | 01 September 2022 |
| | | IL 294622 B1 | 01 June 2023 |
| | | IL 294622 B2 | 01 October 2023 |
| | | AU 2018200308 A1 | 01 February 2018 |
| | | AU 2018200308 B2 | 07 November 2019 |
| CN 105829346 A | 03 August 2016 | BR 112016013482 A2 | 03 October 2017 |
| | | BR 112016013482 B1 | 19 April 2022 |
| | | JP 2022180504 A | 06 December 2022 |
| | | JP 7467557 B2 | 15 April 2024 |
| | | KR 20220025176 A | 03 March 2022 |
| | | KR 102417312 B1 | 05 July 2022 |
| | | IL 278891 A | 31 January 2021 |
| | | IL 278891 B1 | 01 February 2024 |
| | | KR 20210088745 A | 14 July 2021 |
| | | KR 102314913 B1 | 19 October 2021 |
| | | KR 20230162159 A | 28 November 2023 |
| | | AU 2022203818 A1 | 23 June 2022 |
| | | PH 12016500904 A1 | 11 July 2016 |
| | | AU 2020200596 A1 | 20 February 2020 |
| | | AU 2020200596 B2 | 03 March 2022 |
| | | TW 201834692 A | 01 October 2018 |
| | | TWI 661839 B | 11 June 2019 |
| | | HRP 20190431 T1 | 19 April 2019 |
| | | RU 2016123597 A | 02 March 2018 |
| | | RU 2016123597 A3 | 12 October 2018 |

Form PCT/ISA/210 (patent family annex) (July 2022)

**INTERNATIONAL SEARCH REPORT**
**Information on patent family members**

International application No.
**PCT/CN2024/081715**

| Patent document cited in search report | Publication date (day/month/year) | Patent family member(s) | | | Publication date (day/month/year) |
|---|---|---|---|---|---|
| | | RU | 2683780 | C2 | 02 April 2019 |
| | | EP | 3466976 | A1 | 10 April 2019 |
| | | EP | 3466976 | B1 | 08 September 2021 |
| | | SG | 10201800210 | TA | 27 February 2018 |
| | | LTPA | 2021515 | I1 | 10 August 2021 |
| | | LTC | 3101032 | I2 | 25 October 2022 |
| | | IL | 310627 | A | 01 April 2024 |
| | | JP | 2019112403 | A | 11 July 2019 |
| | | JP | 6665325 | B2 | 13 March 2020 |
| | | NL | 301117 | I1 | 19 July 2021 |
| | | NL | 301117 | I2 | 29 July 2021 |
| | | CY | 1121573 | T1 | 29 May 2020 |
| | | ES | 2895254 | T3 | 18 February 2022 |
| | | EP | 3101032 | A1 | 07 December 2016 |
| | | EP | 3101032 | A4 | 06 September 2017 |
| | | EP | 3101032 | B1 | 16 January 2019 |
| | | US | 2024026030 | A1 | 25 January 2024 |
| | | LT | 3101032 | T | 25 March 2019 |
| | | TW | 202237113 | A | 01 October 2022 |
| | | TWI | 796243 | B | 11 March 2023 |
| | | US | 2020385486 | A1 | 10 December 2020 |
| | | US | 11584800 | B2 | 21 February 2023 |
| | | TW | 202322817 | A | 16 June 2023 |
| | | TW | 202237191 | A | 01 October 2022 |
| | | TWI | 796245 | B | 11 March 2023 |
| | | MX | 2016006498 | A | 04 August 2016 |
| | | HUE | 057464 | T2 | 28 May 2022 |
| | | KR | 20210127803 | A | 22 October 2021 |
| | | KR | 102362920 | B1 | 14 February 2022 |
| | | JP | 2020097617 | A | 25 June 2020 |
| | | JP | 6914380 | B2 | 04 August 2021 |
| | | JP | 2021169493 | A | 28 October 2021 |
| | | JP | 7146031 | B2 | 03 October 2022 |
| | | EP | 4212552 | A1 | 19 July 2023 |
| | | RS | 58415 | B1 | 30 April 2019 |
| | | FR | 2C1030 | I1 | 27 August 2021 |
| | | FR | 2C1030 | I2 | 08 April 2022 |
| | | SI | 3101032 | T1 | 28 February 2019 |
| | | EP | 3973995 | A1 | 30 March 2022 |
| | | RU | 2019107788 | A | 17 May 2019 |
| | | HUE | 044389 | T2 | 28 October 2019 |
| | | RS | 62618 | B1 | 31 December 2021 |
| | | WO | 2015115091 | A1 | 06 August 2015 |
| | | LT | 3466976 | T | 10 November 2021 |
| | | TW | 202120088 | A | 01 June 2021 |
| | | TWI | 789700 | B | 11 January 2023 |
| | | US | 2016333112 | A1 | 17 November 2016 |
| | | US | 10155821 | B2 | 18 December 2018 |
| | | TW | 201934145 | A | 01 September 2019 |
| | | TWI | 722422 | B | 21 March 2021 |

Form PCT/ISA/210 (patent family annex) (July 2022)

**INTERNATIONAL SEARCH REPORT**
**Information on patent family members**

International application No.
**PCT/CN2024/081715**

| Patent document cited in search report | Publication date (day/month/year) | Patent family member(s) | | | Publication date (day/month/year) |
|---|---|---|---|---|---|
| | | KR | 20210040181 | A | 12 April 2021 |
| | | KR | 102275925 | B1 | 12 July 2021 |
| | | PT | 3466976 | T | 27 October 2021 |
| | | MX | 2020010682 | A | 06 November 2020 |
| | | ES | 2727351 | T3 | 15 October 2019 |
| | | JP | 6105183 | B1 | 29 March 2017 |
| | | JP | 2017105789 | A | 15 June 2017 |
| | | KR | 20220154251 | A | 21 November 2022 |
| | | KR | 102510128 | B1 | 14 March 2023 |
| | | SI | 3466976 | T1 | 31 December 2021 |
| | | JP | 2017095457 | A | 01 June 2017 |
| | | JP | 6466895 | B2 | 06 February 2019 |
| | | NO | 2021027 | I1 | 06 July 2021 |
| | | KR | 20180122038 | A | 09 November 2018 |
| | | KR | 102190548 | B1 | 14 December 2020 |
| | | PT | 3101032 | T | 03 April 2019 |
| | | KR | 20230113822 | A | 01 August 2023 |
| | | KR | 102606930 | B1 | 29 November 2023 |
| | | HUS | 2100025 | I1 | 28 July 2021 |
| | | TW | 201613649 | A | 16 April 2016 |
| | | TWI | 627967 | B | 01 July 2018 |
| | | HRP | 20211848 | T1 | 04 March 2022 |
| | | ZA | 201602802 | B | 27 September 2017 |
| | | KR | 20200140932 | A | 16 December 2020 |
| | | KR | 102238848 | B1 | 09 April 2021 |
| | | KR | 20230042126 | A | 27 March 2023 |
| | | KR | 102557062 | B1 | 18 July 2023 |
| | | KR | 20220100994 | A | 18 July 2022 |
| | | KR | 102465042 | B1 | 09 November 2022 |
| | | NZ | 719202 | A | 25 February 2022 |
| | | IL | 266790 | A | 31 July 2019 |
| | | CY | 1124774 | T1 | 25 November 2022 |
| | | JP | 6046301 | B1 | 14 December 2016 |
| | | JP | 2017036285 | A | 16 February 2017 |
| | | IL | 245252 | A0 | 30 June 2016 |
| | | IL | 245252 | B | 30 June 2019 |
| | | JP | 5998289 | B2 | 28 September 2016 |
| | | JPWO | 2015115091 | A1 | 23 March 2017 |
| | | TW | 202237114 | A | 01 October 2022 |
| | | TWI | 796244 | B | 11 March 2023 |
| | | DK | 3101032 | T3 | 29 April 2019 |
| | | CA | 2928794 | A1 | 06 August 2015 |
| | | CA | 2928794 | C | 13 August 2019 |
| | | PL | 3101032 | T3 | 31 July 2019 |
| | | TW | 202237115 | A | 01 October 2022 |
| | | TWI | 796246 | B | 11 March 2023 |
| | | KR | 20160113099 | A | 28 September 2016 |
| | | KR | 101916553 | B1 | 07 November 2018 |
| | | US | 2019077880 | A1 | 14 March 2019 |
| | | US | 11795236 | B2 | 24 October 2023 |

Form PCT/ISA/210 (patent family annex) (July 2022)

**INTERNATIONAL SEARCH REPORT**
**Information on patent family members**

International application No.
**PCT/CN2024/081715**

| Patent document cited in search report | | | Publication date (day/month/year) | Patent family member(s) | | | Publication date (day/month/year) |
|---|---|---|---|---|---|---|---|
| | | | | DK | 3466976 | T3 | 13 December 2021 |
| | | | | SG | 11201603960 | XA | 28 July 2016 |
| | | | | AU | 2015212265 | B2 | 02 January 2020 |
| | | | | PL | 3466976 | T3 | 20 December 2021 |
| CN | 111936169 | A | 13 November 2020 | SG | 11202009527 | PA | 29 October 2020 |
| | | | | BR | 112020020466 | A2 | 12 January 2021 |
| | | | | TW | 202010498 | A | 16 March 2020 |
| | | | | CA | 3094313 | A1 | 10 October 2019 |
| | | | | JP | 2024042054 | A | 27 March 2024 |
| | | | | US | 2022193069 | A1 | 23 June 2022 |
| | | | | MX | 2020010458 | A | 29 January 2021 |
| | | | | IL | 310391 | A | 01 March 2024 |
| | | | | AU | 2019247434 | A1 | 08 October 2020 |
| | | | | AR | 114473 | A1 | 09 September 2020 |
| | | | | MA | 52669 | A | 17 February 2021 |
| | | | | IL | 277748 | A | 30 November 2020 |
| | | | | IL | 277748 | B1 | 01 March 2024 |
| | | | | EA | 202092410 | A1 | 09 February 2021 |
| | | | | KR | 20210006362 | A | 18 January 2021 |
| | | | | EP | 3773736 | A1 | 17 February 2021 |
| | | | | EP | 3773736 | A4 | 05 January 2022 |
| | | | | JP | 2021521111 | A | 26 August 2021 |
| | | | | JP | 7430643 | B2 | 13 February 2024 |
| | | | | US | 2019343828 | A1 | 14 November 2019 |
| | | | | WO | 2019195665 | A1 | 10 October 2019 |
| CN | 114845740 | A | 02 August 2022 | EP | 4096717 | A1 | 07 December 2022 |
| | | | | US | 2023099149 | A1 | 30 March 2023 |
| | | | | WO | 2021151984 | A1 | 05 August 2021 |
| | | | | CA | 3160557 | A1 | 05 August 2021 |
| | | | | AU | 2021213421 | A1 | 23 June 2022 |
| | | | | JP | 2023512036 | A | 23 March 2023 |
| WO | 2021164765 | A1 | 26 August 2021 | JP | 2023515034 | A | 12 April 2023 |
| | | | | AU | 2021222203 | A1 | 08 September 2022 |
| | | | | EP | 4108675 | A1 | 28 December 2022 |
| | | | | EP | 4108675 | A4 | 29 May 2024 |
| | | | | KR | 20220143908 | A | 25 October 2022 |
| | | | | US | 2023414771 | A1 | 28 December 2023 |
| | | | | CA | 3167564 | A1 | 26 August 2021 |
| CN | 113274507 | A | 20 August 2021 | None | | | |

Form PCT/ISA/210 (patent family annex) (July 2022)